# EUROPEAN PATENT SPECIFICATION

(11) **EP 0 767 836 B1**
(45) Date of publication and mention of the grant of the patent: **04.09.2002**
(21) Application number: 95923857.7
(22) Date of filing: 15.06.1995
(51) Int. Cl.: C12N 15/53, C12N 9/02, C12N 1/15, A61K 7/13, A61K 7/06, D21C 5/00, C12N 15/80

(54) **PURIFIED POLYPORUS LACCASES AND NUCLEIC ACIDS ENCODING SAME**
GEREINIGTE POLYPORUS LACCASEN UND DAFÜR KODIERENDE NUKLEINSÄUREN
LACCASES DE POLYPORE PURIFIEES ET ACIDES NUCLEIQUES CODANT CELLES-CI

(30) Priority: 24.06.1994 US 265534; 15.05.1995 US 441147
(43) Date of publication of application: 16.04.1997
(73) Proprietor: Novozymes A/S, 2880 Bagsvaerd (DK); Novozymes Biotech, Inc., Davis, CA 95616 (US)
(72) Inventor: YAVER, Debbie Sue, Davis, CA 95616 (US); XU, Feng, Woodland, CA 95776 (US); DALBOGE, Henrik, 2830 Virum (DK); SCHNEIDER, Palle, 2750 Ballerup (DK); AASLYNG, Dorrit Anita, 3500 Vaerlose (DK)
(86) International application number: US9507536
(87) International publication number: WO96000290

(56) References cited:
- WO-A-95/01426
- DE-C- 3 634 761
- DE-C- 4 033 246
- GEN. TECH. REP. NC (NORTH CENT. FOR EXP. STN.), vol. 175, 1994 pages 115-118, YAVER D.S. ET AL. 'The molecular cloning and expression of laccase genes from the white-rot basidiomycete Polyporus pinsitu'
- APPLIED AND ENVIRONMENTAL MICROBIOLOGY, vol. 48, no. 4, 1984 pages 849-854, BOLLAG J.-M. ET AL. 'Comparative studies of extracellular fungal laccases'
- LES COLLOQUES DE L'INRA, vol. 40, 1987 PARIS, pages 223-229, TROJANOWSKI A. ET AL. 'Solubilization and polymerization of lignin by several wood-inhabiting fungi'
- MICROBIOS LETT., vol. 29, no. 113, 1985 pages 37-43, ILAN CHET ET AL. 'Decolourization of the dye Poly B-411 and its correlation with lignin degradation by fungi'

### SEQUENCE LISTING

### (1) GENERAL INFORMATION:

(i) APPLICANT:
   (A) NAME: Novo Nordisk Biotech, Inc.
   (B) STREET: 1445 Drew Avenue
   (C) CITY: Davis, California
   (D) COUNTRY: United States of America
   (E) POSTAL CODE (ZIP): 95616-4880
   (F) TELEPHONE: (916) 757-8100
   (G) TELEFAX: (916) 758-0317
(i) APPLICANT:
   (A) NAME: Novo Nordisk A/S
   (B) STREET: Novo Alle
   (C) CITY: Bagsværd
   (D) COUNTRY: Denmark
   (E) POSTAL CODE (ZIP): DK-2880
   (F) TELEPHONE: +45 4444 8888
   (G) TELEFAX: +45 4449 3256
   (F) TELEX: 37304
(ii) TITLE OF INVENTION: PURIFIED POLYPORUS LACCASES AND NUCLEIC ACIDS ENCODING SAME
(iii) NUMBER OF SEQUENCES: 10
(iv) CORRESPONDENCE ADDRESS:
   (A) ADDRESSEE: Novo Nordisk of North America, Inc.
   (B) STREET: 405 Lexington Avenue, Suite 6400
   (C) CITY and STATE: New York, New York
   (D) COUNTRY: U.S.A.
   (E) ZIP: 10174-6401
(v) COMPUTER READABLE FORM:
   (A) MEDIUM TYPE: Floppy disk
   (B) COMPUTER: IBM PC compatible
   (C) OPERATING SYSTEM: PC-DOS/MS-DOS
   (D) SOFTWARE: PatentIn Release #1.0, Version #1.25 (EPO)
(vi) CURRENT APPLICATION DATA:
   (A) APPLICATION NUMBER: to be assigned
   (B) FILING DATE: 15-June-1995
(vii) PRIOR APPLICATION DATA:
   (A) APPLICATION NUMBER: 08/265,534
   (B) FILING DATE: 24-June-1994
(viii) ATTORNEY/AGENT INFORMATION:
   (A) NAME: Lowney, Karen A.
   (B) REGISTRATION NUMBER: 31,274
   (C) REFERENCE/DOCKET NUMBER: 4185.204-WO
(ix) TELECOMMUNICATION INFORMATION:
   (A) TELEPHONE: 212 867 0123
   (B) TELEFAX: 212 878 9655

### (2) INFORMATION FOR SEQ ID NO: 1:

(i) SEQUENCE CHARACTERISTICS:
   (A) LENGTH: 2418 base pairs
   (B) TYPE: nucleic acid
   (C) STRANDEDNESS: double
   (D) TOPOLOGY: linear
(ii) MOLECULE TYPE: DNA (genomic)
(vi) ORIGINAL SOURCE:
   (A) ORGANISM: Polyporus pinsitus
(ix) FEATURE:
   (A) NAME/KEY: intron
   (B) LOCATION: 414..464
(ix) FEATURE:
   (A) NAME/KEY: intron
   (B) LOCATION: 534..589
(ix) FEATURE:
   (A) NAME/KEY: intron
   (B) LOCATION: 710..764
(ix) FEATURE:
   (A) NAME/KEY: intron
   (B) LOCATION: 879..934
(ix) FEATURE:
   (A) NAME/KEY: intron
   (B) LOCATION: 1001..1050
(ix) FEATURE:
   (A) NAME/KEY: intron
   (B) LOCATION: 1147..1197
(ix) FEATURE:
   (A) NAME/KEY: intron
   (B) LOCATION: 1354..1410
(ix) FEATURE:
   (A) NAME/KEY: intron
   (B) LOCATION: 1609..1662
(ix) FEATURE:
   (A) NAME/KEY: CDS
   (B) LOCATION: join (413..465, 533..590, 709..765, 878..935, 1000..1051, 1146..1198, 1353..1411, 1608..1663)
(xi) SEQUENCE DESCRIPTION: SEQ ID NO: 1:

### (2) INFORMATION FOR SEQ ID NO: 2:

(i) SEQUENCE CHARACTERISTICS:
   (A) LENGTH: 520 amino acids
   (B) TYPE: amino acid
   (C) STRANDEDNESS: single
   (D) TOPOLOGY: linear
(ii) MOLECULE TYPE: protein
(vi) ORIGINAL SOURCE:
   (A) ORGANISM: Polyporus pinsitus
(xi) SEQUENCE DESCRIPTION: SEQ ID NO: 2:
   Met Ser Arg Phe His Ser Leu Leu Ala Phe Val Val Ala Ser Leu Thr
   1               5                   10                  15
   Ala Val Ala His Ala Gly Ile Gly Pro Val Ala Asp Leu Thr Ile Thr
               20                  25                  30
   Asn Ala Ala Val Ser Pro Asp Gly Phe Ser Arg Gln Ala Val Val Val
           35                  40                  45
   Asn Gly Gly Thr Pro Gly Pro Leu Ile Thr Gly Asn Met Gly Asp Arg
       50                  55                  60
   Phe Gln Leu Asn Val Ile Asp Asn Leu Thr Asn His Thr Met Val Lys
   65                  70                  75                  80
   Ser Thr Ser Ile His Trp His Gly Phe Phe Gln Lys Gly Thr Asn Trp
                   85                  90                  95
   Ala Asp Gly Pro Ala Phe Ile Asn Gln Cys Pro Ile Ser Ser Gly His
               100                 105                 110
   Ser Phe Leu Tyr Asp Phe Gln Val Pro Asp Gln Ala Gly Thr Phe Trp
           115                 120                 125
   Tyr His Ser His Leu Ser Thr Gln Tyr Cys Asp Gly Leu Arg Gly Pro
       130                 135                 140
   Phe Val Val Tyr Asp Pro Asn Asp Pro Ala Ala Asp Leu Tyr Asp Val
   145                 150                 155                 160
   Asp Asn Asp Asp Thr Val Ile Thr Leu Val Asp Trp Tyr His Val Ala
                   165                 170                 175
   Ala Lys Leu Gly Pro Ala Phe Pro Leu Gly Ala Asp Ala Thr Leu Ile
           180                 185                 190
   Asn Gly Lys Gly Arg Ser Pro Ser Thr Thr Thr Ala Asp Leu Ser Val
           195                 200                 205
   Ile Ser Val Thr Pro Gly Lys Arg Tyr Arg Phe Arg Leu Val Ser Leu
       210                 215                 220
   Ser Cys Asp Pro Asn Tyr Thr Phe Ser Ile Asp Gly His Asn Met Thr
   225                 230                 235                 240
   Ile Ile Glu Thr Asp Ser Ile Asn Thr Ala Pro Leu Val Val Asp Ser
                   245                 250                 255
   Ile Gln Ile Phe Ala Ala Gln Arg Tyr Ser Phe Val Leu Glu Ala Asn
               260                 265                 270
   Gln Ala Val Asp Asn Tyr Trp Ile Arg Ala Asn Pro Asn Phe Gly Asn
           275                 280                 285
   Val Gly Phe Thr Gly Gly Ile Asn Ser Ala Ile Leu Arg Tyr Asp Gly
       290                 295                 300
   Ala Ala Ala Val Glu Pro Thr Thr Thr Gln Thr Thr Ser Thr Ala Pro
   305                 310                 315                 320
   Leu Asn Glu Val Asn Leu His Pro Leu Val Thr Thr Ala Val Pro Gly
                   325                 330                 335
   Ser Pro Val Ala Gly Gly Val Asp Leu Ala Ile Asn Met Ala Phe Asn
               340                 345                 350
   Phe Asn Gly Thr Asn Phe Phe Ile Asn Gly Thr Ser Phe Thr Pro Pro
           355                 360                 365
   Thr Val Pro Val Leu Leu Gln Ile Ile Ser Gly Ala Gln Asn Ala Gln
       370                 375                 380
   Asp Leu Leu Pro Ser Gly Ser Val Tyr Ser Leu Pro Ser Asn Ala Asp
   385                 390                 395                 400
   Ile Glu Ile Ser Phe Pro Ala Thr Ala Ala Ala Pro Gly Ala Pro His
                   405                 410                 415
   Pro Phe His Leu His Gly His Ala Phe Ala Val Val Arg Ser Ala Gly
               420                 425                 430
   Ser Thr Val Tyr Asn Tyr Asp Asn Pro Ile Phe Arg Asp Val Val Ser
           435                 440                 445
   Thr Gly Thr Pro Ala Ala Gly Asp Asn Val Thr Ile Arg Phe Arg Thr
       450                 455                 460
   Asp Asn Pro Gly Pro Trp Phe Leu His Cys His Ile Asp Phe His Leu
   465                 470                 475                 480
   Glu Ala Gly Phe Ala Val Val Phe Ala Glu Asp Ile Pro Asp Val Ala
                   485                 490                 495
   Ser Ala Asn Pro Val Pro Gln Ala Trp Ser Asp Leu Cys Pro Thr Tyr
               500                 505                 510
   Asp Ala Leu Asp Pro Ser Asp Gln
           515                 520

### (2) INFORMATION FOR SEQ ID NO:3:

(i) SEQUENCE CHARACTERISTICS:
   (A) LENGTH: 2880 base pairs
   (B) TYPE: nucleic acid
   (C) STRANDEDNESS: single
   (D) TOPOLOGY: linear
(ix) FEATURE:
   (A) NAME/KEY: intron
   (B) LOCATION: 544..592
(ix) FEATURE:
   (A) NAME/KEY: intron
   (B) LOCATION: 837..899
(ix) FEATURE:
   (A) NAME/KEY: intron
   (B) LOCATION: 1014..1066
(ix) FEATURE:
   (A) NAME/KEY: intron
   (B) LOCATION: 1133..1187
(ix) FEATURE:
   (A) NAME/KEY: intron
   (B) LOCATION: 1284..1342
(ix) FEATURE:
   (A) NAME/KEY: intron
   (B) LOCATION: 1752..1815
(ix) FEATURE:
   (A) NAME/KEY: intron
   (B) LOCATION: 1873..1928
(ix) FEATURE:
   (A) NAME/KEY: intron
   (B) LOCATION: 2136..2195
(ix) FEATURE:
   (A) NAME/KEY: CDS
   (B) LOCATION: join(364..543, 593..661, 716..835, 900..1013, 1067..1132, 1188..1283, 1343..1498, 1554..1751, 1816..1872, 1929..2135, 2196..2489)
(ix) FEATURE:
   (A) NAME/KEY: intron
   (B) LOCATION: 662..715
(ix) FEATURE:
   (A) NAME/KEY: intron
   (B) LOCATION: 1499..1553
(xi) SEQUENCE DESCRIPTION: SEQ ID NO:3:
   GCGGCGCACA AACCGTGGGA GCCAACACAC TCCCGTCCAC TCTCACACTG GCCAGATTCG       60
   CGCGACCGCC GCCTTTCAGG CCCAAACAGA TCTGGCAGGT TTCGATGGCG CACGCCGCCG      120
   TGCCTGCCGG ATTCAATTGT GCGCCAGTCG GGCATCCGGA TGGCTCTACC AGCGCGGTTG      180
   ACTGGAAGAG AACACCGAGG TCATGCATTC TGGCCAAGTG CGGCCAAAGG ACCGCTCGCT      240
   GGTGCGGATA CTTAAAGGGC GGCGCGGGGA GGCCTGTCTA CCAAGCTCAA GCTCGCCTTG      300
   GGTTCCCAGT CTCCGCCACC CTCCTCTTCC CCCACACAGT CGCTCCATAG CACCGTCGGC      360
   GCC ATG GGT CTG CAG CGA TTC AGC TTC TTC GTC ACC CTC GCG CTC GTC       408
       Met Gly Leu Gln Arg Phe Ser Phe Phe Val Thr Leu Ala Leu Val
        1               5                  10                  15
   GCT CGC TCT CTT GCA GCC ATC GGG CCG GTG GCG AGC CTC GTC GTC GCG       456
   Ala Arg Ser Leu Ala Ala Ile Gly Pro Val Ala Ser Leu Val Val Ala
                    20                  25                  30
   AAC GCC CCC GTC TCG CCC GAC GGC TTC CTT CGG GAT GCC ATC GTG GTC       504
   Asn Ala Pro Val Ser Pro Asp Gly Phe Leu Arg Asp Ala Ile Val Val
                    35                  40                  45
   AAC GGC GTG GTC CCT TCC CCG CTC ATC ACC GGG AAG AAG GTCGGCGTGT          553
   Asn Gly Val Val Pro Ser Pro Leu Ile Thr Gly Lys Lys
           50                  55                  60
   TCGTCGTCGT CCTACTCCTT TGCTGACAGC GATCTACAG GGA GAC CGC TTC CAG          607
                                              Gly Asp Arg Phe Gln
                                                              65
   CTC AAC GTC GTC GAC ACC TTG ACC AAC CAC AGC ATG CTC AAG TCC ACT         655
   Leu Asn Val Val Asp Thr Leu Thr Asn His Ser Met Leu Lys Ser Thr
                   70                  75                  80
   AGT ATC GTAAGTGTGA CGATCCGAAT GTGACATCAA TCGGGGCTAA TTAACCGCGC          711
   Ser Ile
   ACAG CAC TGG CAC GGC TTC TTC CAG GCA GGC ACC AAC TGG GCA GAA GGA        760
        His Trp His Gly Phe Phe Gln Ala Gly Thr Asn Trp Ala Glu Gly
            85                  90                  95
   CCC GCG TTC GTC AAC CAG TGC CCT ATT GCT TCC GGG CAT TCA TTC CTG         808
   Pro Ala Phe Val Asn Gln Cys Pro Ile Ala Ser Gly His Ser Phe Leu
       100                 105                 110
   TAC GAC TTC CAT GTG CCC GAC CAG GCA GTAAGCAGGA TTTTCTGGGG               855
   Tyr Asp Phe His Val Pro Asp Gln Ala
   115                 120
   TCCCCGTGTG ATGCAATGTT CTCATGCTCC GACGTGATCG ACAG GGG ACG TTC TGG        911
                                                    Gly Thr Phe Trp
                                                        125
   TAC CAC AGT CAT CTG TCT ACG CAG TAC TGT GAC GGG CTG CGG GGG CCG         959
   Tyr His Ser His Leu Ser Thr Gln Tyr Cys Asp Gly Leu Arg Gly Pro
           130                 135                 140
   TTC GTC GTG TAC GAC CCC AAG GAC CCG CAC GCC AGC CGT TAC GAT GTT        1007
   Phe Val Val Tyr Asp Pro Lys Asp Pro His Ala Ser Arg Tyr Asp Val
       145                 150                 155
   GAC AAT GTACGTGCGC CACGGAGTAT ATCACACAGC ATGCGTTGAC GTCGGGCCAA         1063
   Asp Asn
   160
   CAG GAG AGC ACG GTC ATC ACG TTG ACC GAC TGG TAC CAC ACC GCT GCC        1111
       Glu Ser Thr Val Ile Thr Leu Thr Asp Trp Tyr His Thr Ala Ala
                   165                 170                 175
   CGG CTC GGT CCC AAG TTC CCA GTAAGCTCGC AATGGCTTAG TGTTCACAGG           1162
   Arg Leu Gly Pro Lys Phe Pro
               180
   TTCTTTGCTT ATGTTGCTTC GATAG CTC GGC GCG GAC GCC ACG CTC ATC AAC        1214
                               Leu Gly Ala Asp Ala Thr Leu Ile Asn
                                   185                 190
   GGT CTG GGG CGG TCG GCC TCG ACT CCC ACC GCT GCG CTT GCC GTG ATC        1262
   Gly Leu Gly Arg Ser Ala Ser Thr Pro Thr Ala Ala Leu Ala Val Ile
           195                 200                 205
   AAC GTC CAG CAC GGA AAG CGC GTGAGCATTC TCTTGTATGC CATTTCAATG           1313
   Asn Val Gln His Gly Lys Arg
       210                 215
   CTTTGTGCTG ACCTATCGGA ACCGCGCAG TAC CGC TTC CGT CTC GTT TCG ATC        1366
                                   Tyr Arg Phe Arg Leu Val Ser Ile
                                                   220
   TCG TGT GAC CCG AAC TAC ACG TTC AGC ATC GAC GGG CAC AAC CTG ACC     1414
   Ser Cys Asp Pro Asn Tyr Thr Phe Ser Ile Asp Gly His Asn Leu Thr
       225                 230                 235
   GTC ATC GAG GTC GAC GGC ATC AAT AGC CAG CCT CTC CTT GTC GAC TCT     1462
   Val Ile Glu Val Asp Gly Ile Asn Ser Gln Pro Leu Leu Val Asp Ser
   240                 245                 250                 255
   ATC CAG ATC TTC GCC GCA CAG CGC TAC TCC TTC GTG GTAAGTCCTG          1508
   Ile Gln Ile Phe Ala Ala Gln Arg Tyr Ser Phe Val
                   260                 265
   GCTTGTCGAT GCTCCAAAGT GGCCTCACTC ATATACTTTC GTTAG TTG AAT GCG       1562
                                                     Leu Asn Ala
                                                             270
   AAT CAA ACG GTG GGC AAC TAC TGG GTT CGT GCG AAC CCG AAC TTC GGA     1610
   Asn Gln Thr Val Gly Asn Tyr Trp Val Arg Ala Asn Pro Asn Phe Gly
                   275                 280                 285
   ACG GTT GGG TTC GCC GGG GGG ATC AAC TCC GCC ATC TTG CGC TAC CAG     1658
   Thr Val Gly Phe Ala Gly Gly Ile Asn Ser Ala Ile Leu Arg Tyr Gln
               290                 295                 300
   GGC GCA CCG GTC GCC GAG CCT ACC ACG ACC CAG ACG CCG TCG GTG ATC     1706
   Gly Ala Pro Val Ala Glu Pro Thr Thr Thr Gln Thr Pro Ser Val Ile
           305                 310                 315
   CCG CTC ATC GAG ACG AAC TTG CAC CCG CTC GCG CGC ATG CCA GTG         1751
   Pro Leu Ile Glu Thr Asn Leu His Pro Leu Ala Arg Met Pro Val
       320                 325                 330
   GTATGTCTCT TTTTCTGATC ATCTGAGTTG CCCGTTGTTG ACCGCATTAT GTGTTACTAT   1811
   CTAG CCT GGC AGC CCG ACA CCC GGG GGC GTC GAC AAG GCG CTC AAC CTC    1860
        Pro Gly Ser Pro Thr Pro Gly Gly Val Asp Lys Ala Leu Asn Leu
            335                 340                 345
   GCG TTT AAC TTC GTAAGTATCT CTACTACTTA GGCTGGAGGC TCGTCGCTGA         1912
   Ala Phe Asn Phe
       350
   TCATACGGTG CTTCAG AAC GGC ACC AAC TTC TTC ATC AAC AAC GCG ACT       1961
                     Asn Gly Thr Asn Phe Phe Ile Asn Asn Ala Thr
                             355                 360
   TTC ACG CCG CCG ACC GTC CCG GTA CTC CTC CAG ATT CTG AGC GGT GCG     2009
   Phe Thr Pro Pro Thr Val Pro Val Leu Leu Gln Ile Leu Ser Gly Ala
       365                 370                 375
   CAG ACC GCA CAA GAC CTG CTC CCC GCA GGC TCT GTC TAC CCG CTC CCG     2057
   Gln Thr Ala Gln Asp Leu Leu Pro Ala Gly Ser Val Tyr Pro Leu Pro
   380                 385                 390                 395
   GCC CAC TCC ACC ATC GAG ATC ACG CTG CCC GCG ACC GCC TTG GCC CCG     2105
   Ala His Ser Thr Ile Glu Ile Thr Leu Pro Ala Thr Ala Leu Ala Pro
                   400                 405                 410
   GGT GCA CCG CAC CCC TTC CAC CTG CAC GGT GTATGTTCCC CTGCCTTCCC       2155
   Gly Ala Pro His Pro Phe His Leu His Gly
               415                 420
   TTCTTATCCC CGAACCAGTG CTCACGTCCG TCCCATCTAG CAC GCC TTC GCG GTC     2210
                                               His Ala Phe Ala Val
                                                               425
   GTT CGC AGC GCG GGG AGC ACC ACG TAT AAC TAC AAC GAC CCG ATC TTC     2258
   Val Arg Ser Ala Gly Ser Thr Thr Tyr Asn Tyr Asn Asp Pro Ile Phe
               430                 435                 440
   CGC GAC GTC GTG AGC ACG GGC ACG CCC GCC GCG GGC GAC AAC GTC ACG      2306
   Arg Asp Val Val Ser Thr Gly Thr Pro Ala Ala Gly Asp Asn Val Thr
           445                 450                 455
   ATC CGC TTC CAG ACG GAC AAC CCC GGG CCG TGG TTC CTC CAC TGC CAC      2354
   Ile Arg Phe Gln Thr Asp Asn Pro Gly Pro Trp Phe Leu His Cys His
       460                 465                 470
   ATC GAC TTC CAC CTC GAC GCA GGC TTC GCG ATC GTG TTC GCA GAG GAC      2402
   Ile Asp Phe His Leu Asp Ala Gly Phe Ala Ile Val Phe Ala Glu Asp
   475                 480                 485                 490
   GTT GCG GAC GTG AAG GCG GCG AAC CCG GTT CCG AAG GCG TGG TCG GAC      2450
   Val Ala Asp Val Lys Ala Ala Asn Pro Val Pro Lys Ala Trp Ser Asp
                   495                 500                 505
   CTG TGC CCG ATC TAC GAC GGG CTG AGC GAG GCT AAC CAG TGAGCGGAGG       2499
   Leu Cys Pro Ile Tyr Asp Gly Leu Ser Glu Ala Asn Gln
               510                 515
   GCGTGGTGTT GAGCGTAAAG CTCGGGCGTC GACCTGGGGG GTTGAAGGTG TTCTGATTGA    2559
   AATGGTCTTT GGGTTTATTT GTTGTTATTC TAACTCGGTT CTCTACGCAA GGACCGAGGA    2619
   TTGTATAGGA TGAAGTAACT TCCCTAATGT ATTATGATAT CAATTGACGG AGGCATGGAC    2679
   TGCGAAGTGT GTACAATGTG GTAGTGGTCT AGGCCTTGGA GACAAGCTGT GGATTTTTCT    2739
   TGGGGGATGA AGAGGCGTGA AGGCTGAGAG CTATGCTATG CCTAGTGACG TGGTTATAGT    2799
   AAATGTCCAT TACATTGACC AAGAACGACA AGAACCATAA GCTTGCTGAG GATAGATGGG    2859
   GGCGCGTCCG CGAACGACTT G                                              2880

### (2) INFORMATION FOR SEQ ID NO:4:

(i) SEQUENCE CHARACTERISTICS:
   (A) LENGTH: 519 amino acids
   (B) TYPE: amino acid
   (D) TOPOLOGY: linear
(ii) MOLECULE TYPE: protein
(xi) SEQUENCE DESCRIPTION: SEQ ID NO:4:
   Met Gly Leu Gln Arg Phe Ser Phe Phe Val Thr Leu Ala Leu Val Ala
    1               5                  10                  15
   Arg Ser Leu Ala Ala Ile Gly Pro Val Ala Ser Leu Val Val Ala Asn
               20                  25                  30
   Ala Pro Val Ser Pro Asp Gly Phe Leu Arg Asp Ala Ile Val Val Asn
           35                  40                  45
   Gly Val Val Pro Ser Pro Leu Ile Thr Gly Lys Lys Gly Asp Arg Phe
       50                  55                  60
   Gln Leu Asn Val Val Asp Thr Leu Thr Asn His Ser Met Leu Lys Ser
   65                  70                  75                  80
   Thr Ser Ile His Trp His Gly Phe Phe Gln Ala Gly Thr Asn Trp Ala
                   85                  90                  95
   Glu Gly Pro Ala Phe Val Asn Gln Cys Pro Ile Ala Ser Gly His Ser
               100                 105                 110
   Phe Leu Tyr Asp Phe His Val Pro Asp Gln Ala Gly Thr Phe Trp Tyr
           115                 120                 125
   His Ser His Leu Ser Thr Gln Tyr Cys Asp Gly Leu Arg Gly Pro Phe
       130                 135                 140
   Val Val Tyr Asp Pro Lys Asp Pro His Ala Ser Arg Tyr Asp Val Asp
   145                 150                 155                 160
   Asn Glu Ser Thr Val Ile Thr Leu Thr Asp Trp Tyr His Thr Ala Ala
                   165                 170                 175
   Arg Leu Gly Pro Lys Phe Pro Leu Gly Ala Asp Ala Thr Leu Ile Asn
               180                 185                 190
   Gly Leu Gly Arg Ser Ala Ser Thr Pro Thr Ala Ala Leu Ala Val Ile
           195                 200                 205
   Asn Val Gln His Gly Lys Arg Tyr Arg Phe Arg Leu Val Ser Ile Ser
       210                 215                 220
   Cys Asp Pro Asn Tyr Thr Phe Ser Ile Asp Gly His Asn Leu Thr Val
   225                 230                 235                 240
   Ile Glu Val Asp Gly Ile Asn Ser Gln Pro Leu Leu Val Asp Ser Ile
                   245                 250                 255
   Gln Ile Phe Ala Ala Gln Arg Tyr Ser Phe Val Leu Asn Ala Asn Gln
               260                 265                 270
   Thr Val Gly Asn Tyr Trp Val Arg Ala Asn Pro Asn Phe Gly Thr Val
           275                 280                 285
   Gly Phe Ala Gly Gly Ile Asn Ser Ala Ile Leu Arg Tyr Gln Gly Ala
       290                 295                 300
   Pro Val Ala Glu Pro Thr Thr Thr Gln Thr Pro Ser Val Ile Pro Leu
   305                 310                 315                 320
   Ile Glu Thr Asn Leu His Pro Leu Ala Arg Met Pro Val Pro Gly Ser
                   325                 330                 335
   Pro Thr Pro Gly Gly Val Asp Lys Ala Leu Asn Leu Ala Phe Asn Phe
               340                 345                 350
   Asn Gly Thr Asn Phe Phe Ile Asn Asn Ala Thr Phe Thr Pro Pro Thr
           355                 360                 365
   Val Pro Val Leu Leu Gln Ile Leu Ser Gly Ala Gln Thr Ala Gln Asp
       370                 375                 380
   Leu Leu Pro Ala Gly Ser Val Tyr Pro Leu Pro Ala His Ser Thr Ile
   385                 390                 395                 400
   Glu Ile Thr Leu Pro Ala Thr Ala Leu Ala Pro Gly Ala Pro His Pro
                   405                 410                 415
   Phe His Leu His Gly His Ala Phe Ala Val Val Arg Ser Ala Gly Ser
               420                 425                 430
   Thr Thr Tyr Asn Tyr Asn Asp Pro Ile Phe Arg Asp Val Val Ser Thr
           435                 440                 445
   Gly Thr Pro Ala Ala Gly Asp Asn Val Thr Ile Arg Phe Gln Thr Asp
       450                 455                 460
   Asn Pro Gly Pro Trp Phe Leu His Cys His Ile Asp Phe His Leu Asp
   465                 470                 475                 480
   Ala Gly Phe Ala Ile Val Phe Ala Glu Asp Val Ala Asp Val Lys Ala
                   485                 490                 495
   Ala Asn Pro Val Pro Lys Ala Trp Ser Asp Leu Cys Pro Ile Tyr Asp
               500                 505                 510
   Gly Leu Ser Glu Ala Asn Gln
           515

### (2) INFORMATION FOR SEQ ID NO: 5:

(i) SEQUENCE CHARACTERISTICS:
   (A) LENGTH: 3102 base pairs
   (B) TYPE: nucleic acid
   (C) STRANDEDNESS: double
   (D) TOPOLOGY: linear
(ii) MOLECULE TYPE: DNA (genomic)
(vi) ORIGINAL SOURCE:
   (A) ORGANISM: Polyporus pinsitus
(ix) FEATURE:
   (A) NAME/KEY: intron
   (B) LOCATION: 666..720
(ix) FEATURE:
   (A) NAME/KEY: intron
   (B) LOCATION: 790..845
(ix) FEATURE:
   (A) NAME/KEY: intron
   (B) LOCATION: 1125..1182
(ix) FEATURE:
   (A) NAME/KEY: intron
   (B) LOCATION: 1390..1450
(ix) FEATURE:
   (A) NAME/KEY: intron
   (B) LOCATION: 1607..1661
(ix) FEATURE:
   (A) NAME/KEY: intron
   (B) LOCATION: 1863..1918
(ix) FEATURE:
   (A) NAME/KEY: intron
   (B) LOCATION: 1976..2025
(ix) FEATURE:
   (A) NAME/KEY: intron
   (B) LOCATION: 2227..2285
(ix) FEATURE:
   (A) NAME/KEY: intron
   (B) LOCATION: 2403..2458
(ix) FEATURE:
   (A) NAME/KEY: intron
   (B) LOCATION: 2576..2627
(ix) FEATURE:
   (A) NAME/KEY: CDS
   (B) LOCATION: join (665..721, 789..846, 1124..1183, 1389..1451, 1606..1662, 1862..1919, 1975..2026, 2226..2286, 2402..2459, 2575..2628).
(xi) SEQUENCE DESCRIPTION: SEQ ID NO: 5:

### (2) INFORMATION FOR SEQ ID NO: 6

(i) SEQUENCE CHARACTERISTICS:
   (A) LENGTH: 512 amino acids
   (B) TYPE: amino acid
   (C) STRANDEDNESS: single
   (D) TOPOLOGY: linear
(ii) MOLECULE TYPE: protein
(vi) ORIGINAL SOURCE:
   (A) ORGANISM: Polyporus pinsitus
(xi) SEQUENCE DESCRIPTION: SEQ ID NO: 6:
   Met Ser Phe Ser Ser Leu Arg Arg Ala Leu Val Phe Leu Gly Ala Cys
   1               5                   10                  15
   Ser Ser Ala Leu Ala Ser Ile Gly Pro Val Thr Glu Leu Asp Ile Val
               20                  25                  30
   Asn Lys Val Ile Ala Pro Asp Gly Val Ala Arg Asp Thr Val Leu Ala
           35                  40                  45
   Gly Gly Thr Phe Pro Gly Pro Leu Ile Thr Gly Lys Lys Gly Asp Asn
       50                  55                  60
   Phe Arg Ile Asn Val Val Asp Lys Leu Val Asn Gln Thr Met Leu Thr
   65                  70                  75                  80
   Ser Thr Thr Ile His Trp His Gly Met Phe Gln His Thr Thr Asn Trp
                   85                  90                  95
   Ala Asp Gly Pro Ala Phe Val Thr Gln Cys Pro Ile Thr Thr Gly Asp
               100                 105                 110
   Asp Phe Leu Tyr Asn Phe Arg Val Pro Asp Gln Thr Gly Thr Tyr Trp
           115                 120                 125
   Tyr His Ser His Leu Ala Leu Gln Tyr Cys Asp Gly Leu Arg Gly Pro
       130                 135                 140
   Leu Val Ile Tyr Asp Pro His Asp Pro Gln Ala Tyr Leu Tyr Asp Val
   145                 150                 155                 160
   Asp Asp Glu Ser Thr Val Ile Thr Leu Ala Asp Trp Tyr His Thr Pro
                   165                 170                 175
   Ala Pro Leu Leu Pro Pro Ala Ala Thr Leu Ile Asn Gly Leu Gly Arg
               180                 185                 190
   Trp Pro Gly Asn Pro Thr Ala Asp Leu Ala Val Ile Glu Val Gln His
           195                 200                 205
   Gly Lys Arg Tyr Arg Phe Arg Leu Val Ser Thr Ser Cys Asp Pro Asn
       210                 215                 220
   Tyr Asn Phe Thr Ile Asp Gly His Thr Met Thr Ile Ile Glu Ala Asp
   225                 230                 235                 240
   Gly Gln Asn Thr Gln Pro His Gln Val Asp Gly Leu Gln Ile Phe Ala
                   245                 250                 255
   Ala Gln Arg Tyr Ser Phe Val Leu Asn Ala Asn Gln Ala Val Asn Asn
               260                 265                 270
   Tyr Trp Ile Arg Ala Asn Pro Asn Arg Ala Asn Thr Thr Gly Phe Ala
           275                 280                 285
   Asn Gly Ile Asn Ser Ala Ile Leu Arg Tyr Lys Gly Ala Pro Ile Lys
       290                 295                 300
   Glu Pro Thr Thr Asn Gln Thr Thr Ile Arg Asn Phe Leu Trp Glu Thr
   305                 310                 315                 320
   Asp Leu His Pro Leu Thr Asp Pro Arg Ala Pro Gly Leu Pro Phe Lys
                   325                 330                 335
   Gly Gly Val Asp His Ala Leu Asn Leu Asn Leu Thr Phe Asn Gly Ser
               340                 345                 350
   Glu Phe Phe Ile Asn Asp Ala Pro Phe Val Pro Pro Thr Val Pro Val
           355                 360                 365
   Leu Leu Gln Ile Leu Asn Gly Thr Leu Asp Ala Asn Asp Leu Leu Pro
       370                 375                 380
   Pro Gly Ser Val Tyr Asn Leu Pro Pro Asp Ser Thr Ile Glu Leu Ser
   385                 390                 395                 400
   Ile Pro Gly Gly Val Thr Gly Gly Pro His Pro Phe His Leu His Gly
                   405                 410                 415
   His Ala Phe Ser Val Val Arg Ser Ala Gly Ser Thr Glu Tyr Asn Tyr
               420                 425                 430
   Ala Asn Pro Val Lys Arg Asp Thr Val Ser Ile Gly Leu Ala Gly Asp
           435                 440                 445
   Asn Val Thr Val Arg Phe Val Thr Asp Asn Pro Gly Pro Trp Phe Leu
       450                 455                 460
   His Cys His Ile Asp Phe His Leu Gln Ala Gly Leu Ala Ile Val Phe
   465                 470                 475                 480
   Ala Glu Asp Ala Gln Asp Thr Lys Leu Val Asn Pro Val Pro Glu Asp
                   485                 490                 495
   Trp Asn Lys Leu Cys Pro Thr Phe Asp Lys Ala Met Asn Ile Thr Val
               500                 505                 510

### (2) INFORMATION FOR SEQ ID NO:7:

(i) SEQUENCE CHARACTERISTICS:
   (A) LENGTH: 2860 base pairs
   (B) TYPE: nucleic acid
   (C) STRANDEDNESS: single
   (D) TOPOLOGY: linear
(ix) FEATURE:
   (A) NAME/KEY: intron
   (B) LOCATION: 851..905
(ix) FEATURE:
   (A) NAME/KEY: intron
   (B) LOCATION: 1266..1320
(ix) FEATURE:
   (A) NAME/KEY: intron
   (B) LOCATION: 1351..1376
(ix) FEATURE:
   (A) NAME/KEY: intron
   (B) LOCATION: 1416..1468
(ix) FEATURE:
   (A) NAME/KEY: intron
   (B) LOCATION: 1625..1683
(ix) FEATURE:
   (A) NAME/KEY: intron
   (B) LOCATION: 1882..1934
(ix) FEATURE:
   (A) NAME/KEY: intron
   (B) LOCATION: 2202..2252
(ix) FEATURE:
   (A) NAME/KEY: intron
   (B) LOCATION: 2370..2425
(ix) FEATURE:
   (A) NAME/KEY: intron
   (B) LOCATION: 2543..2599
(ix) FEATURE:
   (A) NAME/KEY: CDS
   (B) LOCATION: join(540..725, 782..850, 906..1025, 1086..1265, 1321..1350, 1377..1415, 1469..1624, 1684..1881, 1935..2201, 2253..2369, 2426..2542, 2600..2653)
(xi) SEQUENCE DESCRIPTION: SEQ ID NO:7:
   GGGGGGCGCG TCAATGGTCC GTTTGCGAAC ACATATGCAG GATAAACAGT GCGAAATATC      60
   AATGTGGCGG CGACACAACC TCGCCGGCCG ACACTCGACG CTGTTGATCA TGATCATGTC     120
   TTGTGAGCAT TCTATACGCA GCCTTGGAAA TCTCAGGCGA ATTTGTCTGA ATTGCGCTGG     180
   GAGGCTGGCA GCGCAGATCG GTGTGTCGGT GCAGTAGCCG ACGCAGCACC TGGCGGAAGC     240
   CGACATCTCG GGTACGACTT GATCTCCGCC AGATCACTGC GGTTCCGCCA TCGGCCGCGG     300
   GGCCCATTCT GTGTGTGCGC TGTAGCACTC TGCATTCAGG CTCAACGTAT CCATGCTAGA     360
   GGACCGTCCA GCTGTTGGCG CACGATTCGC GCAGAAAGCT GTACAGGCAG ATATAAGGAT     420
   GTCCGTCCGT CAGAGACTCG TCACTCACAA GCCTCTTTTC CTCTTCGCCT TTCCAGCCTC     480
   TTCCAACGCC TGCCATCGTC CTCTTAGTTC GCTCGTCCAT TCTTTCTGCG TAGTTAATC      539
   ATG GGC AGG TTC TCA TCT CTC TGC GCG CTC ACC GCC GTC ATC CAC TCT       587
   Met Gly Arg Phe Ser Ser Leu Cys Ala Leu Thr Ala Val Ile His Ser
    1               5                  10                  15
   TTT GGT CGT GTC TCC GCC GCT ATC GGG CCT GTG ACC GAC CTC ACC ATC       635
   Phe Gly Arg Val Ser Ala Ala Ile Gly Pro Val Thr Asp Leu Thr Ile
               20                  25                  30
   TCC AAT GGG GAC GTT TCT CCC GAC GGC TTC ACT CGT GCC GCA GTG CTT       683
   Ser Asn Gly Asp Val Ser Pro Asp Gly Phe Thr Arg Ala Ala Val Leu
           35                  40                  45
   GCA AAC GGC GTC TTC CCG GGT CCT CTT ATC ACG GGA AAC AAG               725
   Ala Asn Gly Val Phe Pro Gly Pro Leu Ile Thr Gly Asn Lys
       50                  55                  60
   GTACGTGGCA TGCGTTCAGT CTACACCCTA CAAGCCTTCT AACTCTTTTA CCACAG         781
   GGC GAC AAC TTC CAG ATC AAT GTT ATC GAC AAC CTC TCT AAC GAG ACG       829
   Gly Asp Asn Phe Gln Ile Asn Val Ile Asp Asn Leu Ser Asn Glu Thr
           65                  70                  75
   ATG TTG AAG TCG ACC TCC ATC GTATGTGCTT CTACTGCTTC TTAGTCTTGG          880
   Met Leu Lys Ser Thr Ser Ile
       80                  85
   CAATGGCTCA AGGTCTCCTC CGCAG CAT TGG CAC GGC TTC TTC CAG AAG GGT      932
                               His Trp His Gly Phe Phe Gln Lys Gly
                                               90
   ACT AAC TGG GCT GAT GGA GCT GCC TTC GTC AAC CAG TGC CCT ATC GCG       980
   Thr Asn Trp Ala Asp Gly Ala Ala Phe Val Asn Gln Cys Pro Ile Ala
    95                 100                 105                 110
   ACG GGG AAC TCT TTC CTT TAC GAC TTC ACC GCG ACG GAC CAA GCA          1025
   Thr Gly Asn Ser Phe Leu Tyr Asp Phe Thr Ala Thr Asp Gln Ala
                   115                 120                 125
   GTCAGTGCCT GTGGCGCTTA TGTTTTCCCG TAATCAGCAG CTAACACTCC GCACCCACAG    1085
   GGC ACC TTC TGG TAC CAC AGT CAC TTG TCT ACG CAG TAC TGC GAT GGT      1133
   Gly Thr Phe Trp Tyr His Ser His Leu Ser Thr Gln Tyr Cys Asp Gly
                   130                 135                 140
   TTG CGG GGC CCG ATG GTC GTA TAC GAC CCG AGT GAC CCG CAT GCG GAC      1181
   Leu Arg Gly Pro Met Val Val Tyr Asp Pro Ser Asp Pro His Ala Asp
               145                 150                 155
   CTT TAC GAC GTC GAC GAC GAG ACC ACG ATC ATC ACG CTC TCT GAT TGG      1229
   Leu Tyr Asp Val Asp Asp Glu Thr Thr Ile Ile Thr Leu Ser Asp Trp
           160                 165                 170
   TAT CAC ACC GCT GCT TCG CTC GGT GCT GCC TTC CCG GTAAGTTTAC           1275
   Tyr His Thr Ala Ala Ser Leu Gly Ala Ala Phe Pro
       175                 180                 185
   CCCAGCGCAC GGAGTTAAGA CCGGATCTAA CTGTAATACG TTCAG ATT GGC TCG        1329
                                                     Ile Gly Ser
   GAC TCT ACC CTG ATT AAC GGC GTTGGCCGCT TCGCGGGTGG TGACAG ACT GAC     1382
   Asp Ser Thr Leu Ile Asn Gly                              Thr Asp
       190                 195
   CTT GCG GTT ATC ACT GTC GAG CAG GGC AAG CGC GTTAGTGATA CCCTCTACAG    1435
   Leu Ala Val Ile Thr Val Glu Gln Gly Lys Arg
           200                 205
   TTGACACTGT GCCATTGCTG ACAGTACTCT CAG TAC CGT ATG CGT CTT CTC TCG     1489
                                        Tyr Arg Met Arg Leu Leu Ser
                                            210                 215
   CTG TCT TGC GAC CCC AAC TAT GTC TTC TCC ATT GAC GGC CAC AAC ATG      1537
   Leu Ser Cys Asp Pro Asn Tyr Val Phe Ser Ile Asp Gly His Asn Met
                   220                 225                 230
   ACC ATC ATC GAG GCC GAC GCC GTC AAC CAC GAG CCC CTC ACG GTT GAC      1585
   Thr Ile Ile Glu Ala Asp Ala Val Asn His Glu Pro Leu Thr Val Asp
               235                 240                 245
   TCC ATC CAG ATC TAC GCC GGC CAA CGT TAC TCC TTC GTC GTACGTATTC       1634
   Ser Ile Gln Ile Tyr Ala Gly Gln Arg Tyr Ser Phe Val
           250                 255                 260
   CGAACAGCCA TGATCACGCC AAGCCCGATG CTAACGCGCC TACCCTCAG CTT ACC        1689
                                                         Leu Thr
   GCT GAC CAG GAC ATC GAC AAC TAC TTC ATC CGT GCC CTG CCC AGC GCC      1737
   Ala Asp Gln Asp Ile Asp Asn Tyr Phe Ile Arg Ala Leu Pro Ser Ala
           265                 270                 275
   GGT ACC ACC TCG TTC GAC GGC GGC ATC AAC TCG GCT ATC CTG CGC TAC      1785
   Gly Thr Thr Ser Phe Asp Gly Gly Ile Asn Ser Ala Ile Leu Arg Tyr
       280                 285                 290
   TCT GGT GCC TCC GAG GTT GAC CCG ACG ACC ACG GAG ACC ACG AGC GTC      1833
   Ser Gly Ala Ser Glu Val Asp Pro Thr Thr Thr Glu Thr Thr Ser Val
   295                 300                 305                 310
   CTC CCC CTC GAC GAG GCG AAC CTC GTG CCC CTT GAC AGC CCC GCT GCT      1881
   Leu Pro Leu Asp Glu Ala Asn Leu Val Pro Leu Asp Ser Pro Ala Ala
                   315                 320                 325
   GTACGTCGTA TTCTGCGCTT GCAAGGATCG CACATACTAA CATGCTCTTG TAG CCC      1937
                                                              Pro
   GGT GAC CCC AAC ATT GGC GGT GTC GAC TAC GCG CTG AAC TTG GAC TTC      1985
   Gly Asp Pro Asn Ile Gly Gly Val Asp Tyr Ala Leu Asn Leu Asp Phe
           330                 335                 340
   AAC TTC GAT GGC ACC AAC TTC TTC ATC AAC GAC GTC TCC TTC GTG TCC      2033
   Asn Phe Asp Gly Thr Asn Phe Phe Ile Asn Asp Val Ser Phe Val Ser
       345                 350                 355
   CCC ACG GTC CCT GTC CTC CTC CAG ATT CTT AGC GGC ACC ACC TCC GCG      2081
   Pro Thr Val Pro Val Leu Leu Gln Ile Leu Ser Gly Thr Thr Ser Ala
   360                 365                 370                 375
   GCC GAC CTT CTC CCC AGC GGT AGT CTC TTC GCG GTC CCG TCC AAC TCG      2129
   Ala Asp Leu Leu Pro Ser Gly Ser Leu Phe Ala Val Pro Ser Asn Ser
                   380                 385                 390
   ACG ATC GAG ATC TCG TTC CCC ATC ACC GCG ACG AAC GCT CCC GGC GCG      2177
   Thr Ile Glu Ile Ser Phe Pro Ile Thr Ala Thr Asn Ala Pro Gly Ala
               395                 400                 405
   CCG CAT CCC TTC CAC TTG CAC GGT GTACGTGTCC CATCTCATAT GCTACGGAGC     2231
   Pro His Pro Phe His Leu His Gly
           410                 415
   TCCACGCTGA CCGCCCTATA G CAC ACC TTC TCT ATC GTT CGT ACC GCC GGC      2282
                           His Thr Phe Ser Ile Val Arg Thr Ala Gly
                                           420                 425
   AGC ACG GAT ACG AAC TTC GTC AAC CCC GTC CGC CGC GAC GTC GTG AAC      2330
   Ser Thr Asp Thr Asn Phe Val Asn Pro Val Arg Arg Asp Val Val Asn
                   430                 435                 440
   ACC GGT ACC GTC GGC GAC AAC GTC ACC ATC CGC TTC ACG GTACGCAGCA       2379
   Thr Gly Thr Val Gly Asp Asn Val Thr Ile Arg Phe Thr
               445                 450
   CTCTCCTAAC ATTCCCACTG CGCGATCACT GACTCCTCGC CCACAG ACT GAC AAC      2434
                                                      Thr Asp Asn
                                                          455
   CCC GGC CCC TGG TTC CTC CAC TGC CAC ATC GAC TTC CAC TTG GAG GCC      2482
   Pro Gly Pro Trp Phe Leu His Cys His Ile Asp Phe His Leu Glu Ala
           460                 465                 470
   GGT TTC GCC ATC GTC TTC AGC GAG GAC ACC GCC GAC GTC TCG AAC ACG      2530
   Gly Phe Ala Ile Val Phe Ser Glu Asp Thr Ala Asp Val Ser Asn Thr
       475                 480                 485
   ACC ACG CCC TCG GTACGTTGTG CTCCCGTGCC CATCTCCGCG CGCCTGACTA          2582
   Thr Thr Pro Ser
   490
   ACGAGCACCC CTTACAG ACT GCT TGG GAA GAT CTG TGC CCC ACG TAC AAC      2632
                      Thr Ala Trp Glu Asp Leu Cys Pro Thr Tyr Asn
                              495                 500
   GCT CTT GAC TCA TCC GAC CTC TAATCGGTTC AAAGGGTCGC TCGCTACCTT         2683
   Ala Leu Asp Ser Ser Asp Leu
   505                 510
   AGTAGGTAGA CTTATGCACC GGACATTATC TACAATGGAC TTTAATTTGG GTTAACGGCC 2743
   GTTATACATA CGCGCACGTA GTATAAAGGT TCTCTGGATT GGTCGGACCT ACAGACTGCA 2803
   ATTTTCGTGA CCTATCAACT GTATATTGAA GCACGACAGT GAATGGAAAT AGAGACA 2860

### (2) INFORMATION FOR SEQ ID NO:8:

(i) SEQUENCE CHARACTERISTICS:
   (A) LENGTH: 511 amino acids
   (B) TYPE: amino acid
   (D) TOPOLOGY: linear
(ii) MOLECULE TYPE: protein
(xi) SEQUENCE DESCRIPTION: SEQ ID NO:8:
   Met Gly Arg Phe Ser Ser Leu Cys Ala Leu Thr Ala Val Ile His Ser
    1               5                  10                  15
   Phe Gly Arg Val Ser Ala Ala Ile Gly Pro Val Thr Asp Leu Thr Ile
               20                  25                  30
   Ser Asn Gly Asp Val Ser Pro Asp Gly Phe Thr Arg Ala Ala Val Leu
           35                  40                  45
   Ala Asn Gly Val Phe Pro Gly Pro Leu Ile Thr Gly Asn Lys Gly Asp
       50                  55                  60
   Asn Phe Gln Ile Asn Val Ile Asp Asn Leu Ser Asn Glu Thr Met Leu
   65                  70                  75                  80
   Lys Ser Thr Ser Ile His Trp His Gly Phe Phe Gln Lys Gly Thr Asn
                   85                  90                  95
   Trp Ala Asp Gly Ala Ala Phe Val Asn Gln Cys Pro Ile Ala Thr Gly
               100                 105                 110
   Asn Ser Phe Leu Tyr Asp Phe Thr Ala Thr Asp Gln Ala Gly Thr Phe
           115                 120                 125
   Trp Tyr His Ser His Leu Ser Thr Gln Tyr Cys Asp Gly Leu Arg Gly
       130                 135                 140
   Pro Met Val Val Tyr Asp Pro Ser Asp Pro His Ala Asp Leu Tyr Asp
   145                 150                 155                 160
   Val Asp Asp Glu Thr Thr Ile Ile Thr Leu Ser Asp Trp Tyr His Thr
                   165                 170                 175
   Ala Ala Ser Leu Gly Ala Ala Phe Pro Ile Gly Ser Asp Ser Thr Leu
               180                 185                 190
   Ile Asn Gly Thr Asp Leu Ala Val Ile Thr Val Glu Gln Gly Lys Arg
           195                 200                 205
   Tyr Arg Met Arg Leu Leu Ser Leu Ser Cys Asp Pro Asn Tyr Val Phe
       210                 215                 220
   Ser Ile Asp Gly His Asn Met Thr Ile Ile Glu Ala Asp Ala Val Asn
   225                 230                 235                 240
   His Glu Pro Leu Thr Val Asp Ser Ile Gln Ile Tyr Ala Gly Gln Arg
                   245                 250                 255
   Tyr Ser Phe Val Leu Thr Ala Asp Gln Asp Ile Asp Asn Tyr Phe Ile
               260                 265                 270
   Arg Ala Leu Pro Ser Ala Gly Thr Thr Ser Phe Asp Gly Gly Ile Asn
           275                 280                 285
   Ser Ala Ile Leu Arg Tyr Ser Gly Ala Ser Glu Val Asp Pro Thr Thr
       290                 295                 300
   Thr Glu Thr Thr Ser Val Leu Pro Leu Asp Glu Ala Asn Leu Val Pro
   305                 310                 315                 320
   Leu Asp Ser Pro Ala Ala Pro Gly Asp Pro Asn Ile Gly Gly Val Asp
                   325                 330                 335
   Tyr Ala Leu Asn Leu Asp Phe Asn Phe Asp Gly Thr Asn Phe Phe Ile
               340                 345                 350
   Asn Asp Val Ser Phe Val Ser Pro Thr Val Pro Val Leu Leu Gln Ile
           355                 360                 365
   Leu Ser Gly Thr Thr Ser Ala Ala Asp Leu Leu Pro Ser Gly Ser Leu
       370                 375                 380
   Phe Ala Val Pro Ser Asn Ser Thr Ile Glu Ile Ser Phe Pro Ile Thr
   385                 390                 395                 400
   Ala Thr Asn Ala Pro Gly Ala Pro His Pro Phe His Leu His Gly His
                   405                 410                 415
   Thr Phe Ser Ile Val Arg Thr Ala Gly Ser Thr Asp Thr Asn Phe Val
               420                 425                 430
   Asn Pro Val Arg Arg Asp Val Val Asn Thr Gly Thr Val Gly Asp Asn
           435                 440                 445
   Val Thr Ile Arg Phe Thr Thr Asp Asn Pro Gly Pro Trp Phe Leu His
       450                 455                 460
   Cys His Ile Asp Phe His Leu Glu Ala Gly Phe Ala Ile Val Phe Ser
   465                 470                 475                 480
   Glu Asp Thr Ala Asp Val Ser Asn Thr Thr Thr Pro Ser Thr Ala Trp
                   485                 490                 495
   Glu Asp Leu Cys Pro Thr Tyr Asn Ala Leu Asp Ser Ser Asp Leu
               500                 505                 510

### (2) INFORMATION FOR SEQ ID NO: 9:

(i) SEQUENCE CHARACTERISTICS:
   (A) LENGTH: 2925 base pairs
   (B) TYPE: nucleic acid
   (C) STRANDEDNESS: double
   (D) TOPOLOGY: linear
(ii) MOLECULE TYPE: DNA (genomic)
(vi) ORIGINAL SOURCE:
   (A) ORGANISM: Polyporus pinsitus
(ix) FEATURE:
   (A) NAME/KEY: intron
   (B) LOCATION: 734..808
(ix) FEATURE:
   (A) NAME/KEY: intron
   (B) LOCATION: 878..932
(ix) FEATURE:
   (A) NAME/KEY: intron
   (B) LOCATION: 1051..1104
(ix) FEATURE:
   (A) NAME/KEY: intron
   (B) LOCATION: 1219..1270
(ix) FEATURE:
   (A) NAME/KEY: intron
   (B) LOCATION: 1336..1397
(ix) FEATURE:
   (A) NAME/KEY: intron
   (B) LOCATION: 1713..7744
(ix) FEATURE:
   (A) NAME/KEY: intron
   (B) LOCATION: 2030..2085
(ix) FEATURE:
   (A) NAME/KEY: intron
   (B) LOCATION: 2308..2375
(ix) FEATURE:
   (A) NAME/KEY: intron
   (B) LOCATION: 2492..2569
(ix) FEATURE:
   (A) NAME/KEY: CDS
   (B) LOCATION: join (733..809, 877..933, 1050..1105, 1218..1271, 1335..1398, 1712..1775, 2029..2086, 2307..2376, 2492..2570). 2542..2600).
(xi) SEQUENCE DESCRIPTION: SEQ ID NO: 9:
   CTCATAACTC TTCGCTTCTA GCATGGGGGC TGCGCACACC TGACAGACCC TTCGGGAGGC        60
   GAACTCGAAT GCAGCGTACT CTATCNCACC TCCAGGAAAG GTAGGGATGG ACNCCGTGCA       120
   CCAACAACTG TCTCTCCACC AGCAACCATC CCTTGGATAT GTCTCCACAC ACCCGGTGTC       180
   TACAAGCGGG GATCTGTGCT GGTGAAGTGC TGTCTCCGGA GCGGCGGCGG CGAGCGACCA       240
   GAACCCGAAC CAGTGCTAGT GCCCGACACC CGCGAGACAA TTGTGCAGGG TGAGTTATAT       300
   TCTTCGTGAG ACGGCGCTGC GCGTCGGCAC TGAAAGCGTC GCAGTTAGGT GATGCAGCGG       360
   TCCGCGCTAT TTTTGACGTC TGGCAGCTAT CCTAAGCCGC GCCTCCATAC ACCCCAGGCG       420
   CTCTCGTTTG CTATAGGTAT AAATCCCTCA GCTTCAGAGC GTCGATCCTC ATCCCACACG       480
   ACACCCGTTT CAGTCTTCTC GTAGCGCATT CCCTAGCCGC CCAGCCTCCG CTTTCGTTTT       540
   CAAC ATG GGC AAG TAT CAC TCT TTT GTG AAC GTC GTC GCC CTT AGT CTT        589
        Met Gly Lys Tyr His Ser Phe Val Asn Val Val Ala Leu Ser Leu
        1               5                   10                  15
   TCT TTG AGC GGT CGT GTG TTC GGC GCC ATT GGG CCC GTC ACC GAC TTG         637
   Ser Leu Ser Gly Arg Val Phe Gly Ala Ile Gly Pro Val Thr Asp Leu
                   20                  25                  30
   ACT ATC TCT AAC GCC GAT GTT ACG CCT GAC GGC ATT ACT CGT GCT GCT         685
   Thr Ile Ser Asn Ala Asp Val Thr Pro Asp Gly Ile Thr Arg Ala Ala
               35                  40                  45
   GTC CTC GCG GGC GGC GTT TTC CCC GGG CCC CTC ATT ACC GGC AAC AAG         733
   Val Leu Ala Gly Gly Val Phe Pro Gly Pro Leu Ile Thr Gly Asn Lys
           50                  55                  60
   GTGAGCCGCG AAACCTTCTA CTAGCGCGCT CGTACGGTGC ACCGTTACTG AAGCCACACT       793
   TTGCGCTGTC AACAG GGG GAT GAA TTC CAG ATC AAT GTC ATC GAC AAC CTG          844
                    Gly Asp Glu Phe Gln Ile Asn Val Ile Asp Asn Leu
                        65                  70                  75
   ACC AAC GAG ACC ATG TTG AAG TCG ACC ACA ATC GTAAGGTGCT TGCTCCCATA         897
   Thr Asn Glu Thr Met Leu Lys Ser Thr Thr Ile
                   80                  85
   ATTAAGCCCG TCGCTGACTC GAAGTTTATC TGTAG CAC TGG CAT GGT ATC TTC            950
                                          His Trp His Gly Ile Phe
                                                      90
   CAG GCC GGC ACC AAC TGG GCA GAC GGC GCG GCC TTC GTG AAC CAG TGC           998
   Gln Ala Gly Thr Asn Trp Ala Asp Gly Ala Ala Phe Val Asn Gln Cys
           95                  100                 105
   CCT ATC GCC ACG GGA AAC TCG TTC TTG TAC GAC TTC ACC GTT CCT GAT          1046
   Pro Ile Ala Thr Gly Asn Ser Phe Leu Tyr Asp Phe Thr Val Pro Asp
       110                 115                 120
   CAA GCC GTACGTTTAT ACACTTCCCT TTCTGCGGCA TACTCTGACG CGCCGCTGGA           1102
   Gln Ala
   125
   TCAG GGC ACC TTC TGG TAC CAC AGC CAC CTG TCC ACC CAG TAC TGT GAC         1151
        Gly Thr Phe Trp Tyr His Ser His Leu Ser Thr Gln Tyr Cys Asp
                    130                 135                 140
   GGC CTG CGC GGT CCT CTT GTG GTC TAC GAC CCC GAC GAT CCC AAC GCG          1199
   Gly Leu Arg Gly Pro Leu Val Val Tyr Asp Pro Asp Asp Pro Asn Ala
               145                 150                 155
   TCT CTT TAC GAC GTC GAT GAC GTAAGCAGGC TACTTGTGGA CTTGTATGGA             1250
   Ser Leu Tyr Asp Val Asp Asp
           160
   TGTATCTCAC GCTCCCCTAC AG GAT ACT ACG GTT ATT ACG CTT GCG GAC TGG         1302
                            Asp Thr Thr Val Ile Thr Leu Ala Asp Trp
                            165                 170
   TAC CAC ACT GCG GCG AAG CTG GGC CCT GCC TTC CCC GTGAGTCTAC               1348
   Tyr His Thr Ala Ala Lys Leu Gly Pro Ala Phe Pro
   175                 180                 185
   TCTTCCTCGT GTGTTAACAT AGGTGACGGC CGCTGATACG AGAGCTACCA G GCG GGT         1405
                                                            Ala Gly
   CCG GAT AGC GTC TTG ATC AAT GGT CTT GGT CGG TTC TCC GGC GAT GGT          1453
   Pro Asp Ser Val Leu Ile Asn Gly Leu Gly Arg Phe Ser Gly Asp Gly
       190                 195                 200
   GGA GGA GCG ACA AAC CTC ACC GTG ATC ACC GTC ACG CAA GGC AAA CGG          1501
   Gly Gly Ala Thr Asn Leu Thr Val Ile Thr Val Thr Gln Gly Lys Arg
   205                 210                 215                 220
   GTGAGTCCGC CCTGAGCTGG CCTCAATAGC GATATTGACG AGTCCATGCC CTCCCAG           1558
   TAC CGC TTC CGC CTT GTG TCG ATC TCG TGC GAC CCC AAC TTC ACG TTC          1606
   Tyr Arg Phe Arg Leu Val Ser Ile Ser Cys Asp Pro Asn Phe Thr Phe
                   225                 230                 235
   TCG ATC GAC GGG CAC AAC ATG ACC ATC ATC GAG GTG GAC GGT GTC AAC          1654
   Ser Ile Asp Gly His Asn Met Thr Ile Ile Glu Val Asp Gly Val Asn
               240                 245                 250
   CAC GAG GCC TTG GAC GTC GAC TCC ATT CAG ATT TTT GCG GGG CAG CGG          1702
   His Glu Ala Leu Asp Val Asp Ser Ile Gln Ile Phe Ala Gly Gln Arg
           255                 260                 265
   TAC TCC TTC ATC GTACGTTCCC TTGCCCTCGT GCTATATCCG CCCGTCTGCT         1754
   Tyr Ser Phe Ile
       270
   CACAGAGGCT TCTATATCGC AG CTC AAC GCC AAC CAG TCC ATC GAC AAC        1803
                            Leu Asn Ala Asn Gln Ser Ile Asp Asn
                                    275                 280
   TAC TGG ATC CGC GCG ATC CCC AAC ACC GGT ACC ACC GAC ACC ACG GGC     1851
   Tyr Trp Ile Arg Ala Ile Pro Asn Thr Gly Thr Thr Asp Thr Thr Gly
               285                 290                 295
   GGC GTG AAC TCT GCT ATT CTT CGC TAC GAC ACC GCA GAA GAT ATC GAG     1899
   Gly Val Asn Ser Ala Ile Leu Arg Tyr Asp Thr Ala Glu Asp Ile Glu
           300                 305                 310
   CCT ACG ACC AAC GCG ACC ACC TCC GTC ATC CCT CTC ACC GAG ACG GAT     1947
   Pro Thr Thr Asn Ala Thr Thr Ser Val Ile Pro Leu Thr Glu Thr Asp
       315                 320                 325
   CTG GTG CCG CTC GAC AAC CCT GCG GCT CCC GGT GAC CCC CAG GTC GGC     1995
   Leu Val Pro Leu Asp Asn Pro Ala Ala Pro Gly Asp Pro Gln Val Gly
   330                 335                 340                 345
   GGT GTT GAC CTG GCT ATG AGT CTC GAC TTC TCC TTC GTGAGTCCCA          2041
   Gly Val Asp Leu Ala Met Ser Leu Asp Phe Ser Phe
                   350                 355
   CAGCACTCCG CGCCATTTCG CTTATTTACG CAGGAGTATT GTTCAG AAC GGT TCC      2096
                                                      Asn Gly Ser
                                                              360
   AAC TTC TTT ATC AAC AAC GAG ACC TTC GTC CCG CCC ACA GTT CCC GTG     2144
   Asn Phe Phe Ile Asn Asn Glu Thr Phe Val Pro Pro Thr Val Pro Val
                   365                 370                 375
   CTC CTG CAG ATT TTG AGT GGT GCG CAG GAC GCG GCG AGC CTG CTC CCC     2192
   Leu Leu Gln Ile Leu Ser Gly Ala Gln Asp Ala Ala Ser Leu Leu Pro
               380                 385                 390
   AAC GGG AGT GTC TAC ACA CTC CCT TCG AAC TCG ACC ATT GAG ATC TCG     2240
   Asn Gly Ser Val Tyr Thr Leu Pro Ser Asn Ser Thr Ile Glu Ile Ser
           395                 400                 405
   TTC CCC ATC ATC ACC ACC GAC GGT GTT CTG AAC GCG CCC GGT GCT CCG     2288
   Phe Pro Ile Ile Thr Thr Asp Gly Val Leu Asn Ala Pro Gly Ala Pro
       410                 415                 420
   CAC CCG TTC CAT CTC CAC GGC GTAAGTCCTT GCTTTCCTCA GTGCCTCGCT        2339
   His Pro Phe His Leu His Gly
   425                 430
   TCCACGACGT CCACTGATCC CACACATCCC ATGTGCAG CAC ACC TTC TCG GTG       2392
                                            His Thr Phe Ser Val
                                                            435
   GTG CGC AGC GCC GGG AGC TCG ACC TTC AAC TAC GCC AAC CCA GTC CGC     2440
   Val Arg Ser Ala Gly Ser Ser Thr Phe Asn Tyr Ala Asn Pro Val Arg
               440                 445                 450
   CGG GAC ACC GTC AGT ACT GGT AAC TCT GGC GAC AAC GTC ACT ATC CGC     2488
   Arg Asp Thr Val Ser Thr Gly Asn Ser Gly Asp Asn Val Thr Ile Arg
           455                 460                 465
   TTC ACG GTACGTCTTC TCCGGAGCCC TCCCACCCGT GTGTCCGCTG AGCGCTGAAC      2544
   Phe Thr
       470
   ACCGCCCACC GTGCTGCTGC TGCGCAG ACC GAC AAC CCA GGC CCG TGG TTC       2595
                                 Thr Asp Asn Pro Gly Pro Trp Phe
                                                 475
   CTC CAC TGC CAC ATC GAC TTC CAC CTG GAG GCC GGC TTC GCC ATC GTC      2643
   Leu His Cys His Ile Asp Phe His Leu Glu Ala Gly Phe Ala Ile Val
       480                 485                 490
   TGG GGG GAG GAC ACT GCG GAC ACC GCG TCC GCG AAT CCC GTT CCT          2688
   Trp Gly Glu Asp Thr Ala Asp Thr Ala Ser Ala Asn Pro Val Pro
   495                 500                 505
   GTACGTCGTG CCTGCTGAGC TCTTTGTGCC CGAACAGGGT GCTGATCGTG CCTTCCTCCG    2748
   TGCAG ACG GCG TGG AGC GAT TTG TGC CCC ACT TAC GAT GCT TTG GAC TCG    2798
   Thr Ala Trp Ser Asp Leu Cys Pro Thr Tyr Asp Ala Leu Asp Ser
   510                 515                 520
   TCC GAC CTC TGATCGACAA GGCATGAAGG CTGAAGCAGC TGCGGTCAAT              2847
   Ser Asp Leu
   525
   TCTCGAACAC ACTTTACTCG AACATTCATT TTTCTTTGGC TCGGGATCGG AACAAATCAT    2907
   GGGGGGGCCG GACCGTCT                                                  2925

### (2) INFORMATION FOR SEQ ID NO: 10

(i) SEQUENCE CHARACTERISTICS:
   (A) LENGTH: 527 amino acids
   (B) TYPE: amino acid
   (C) STRANDEDNESS: single
   (D) TOPOLOGY: linear
(ii) MOLECULE TYPE: protein
(vi) ORIGINAL SOURCE:
   (A) ORGANISM: Polyporus pinsitus
(xi) SEQUENCE DESCRIPTION: SEQ ID NO: 10:

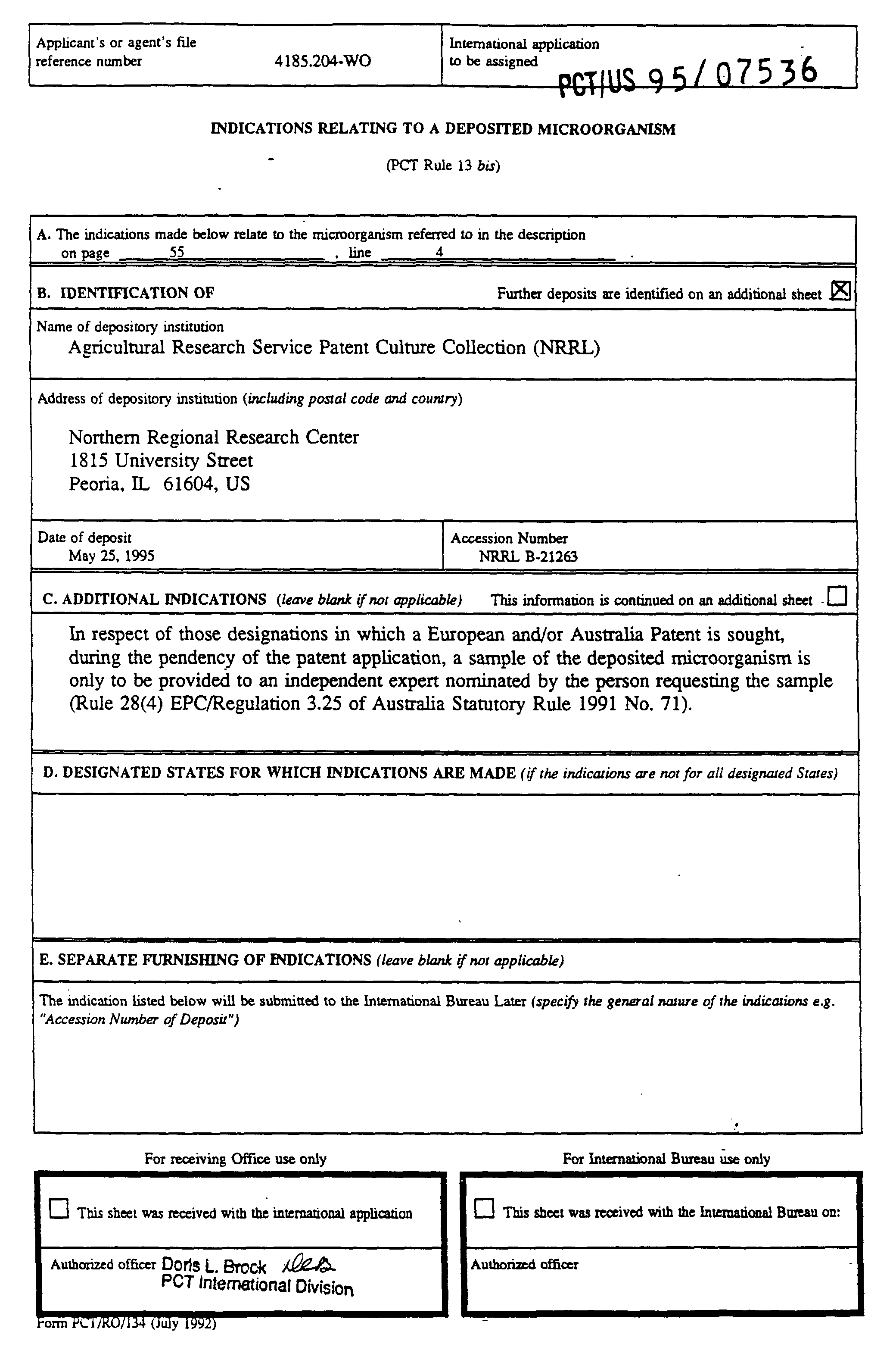

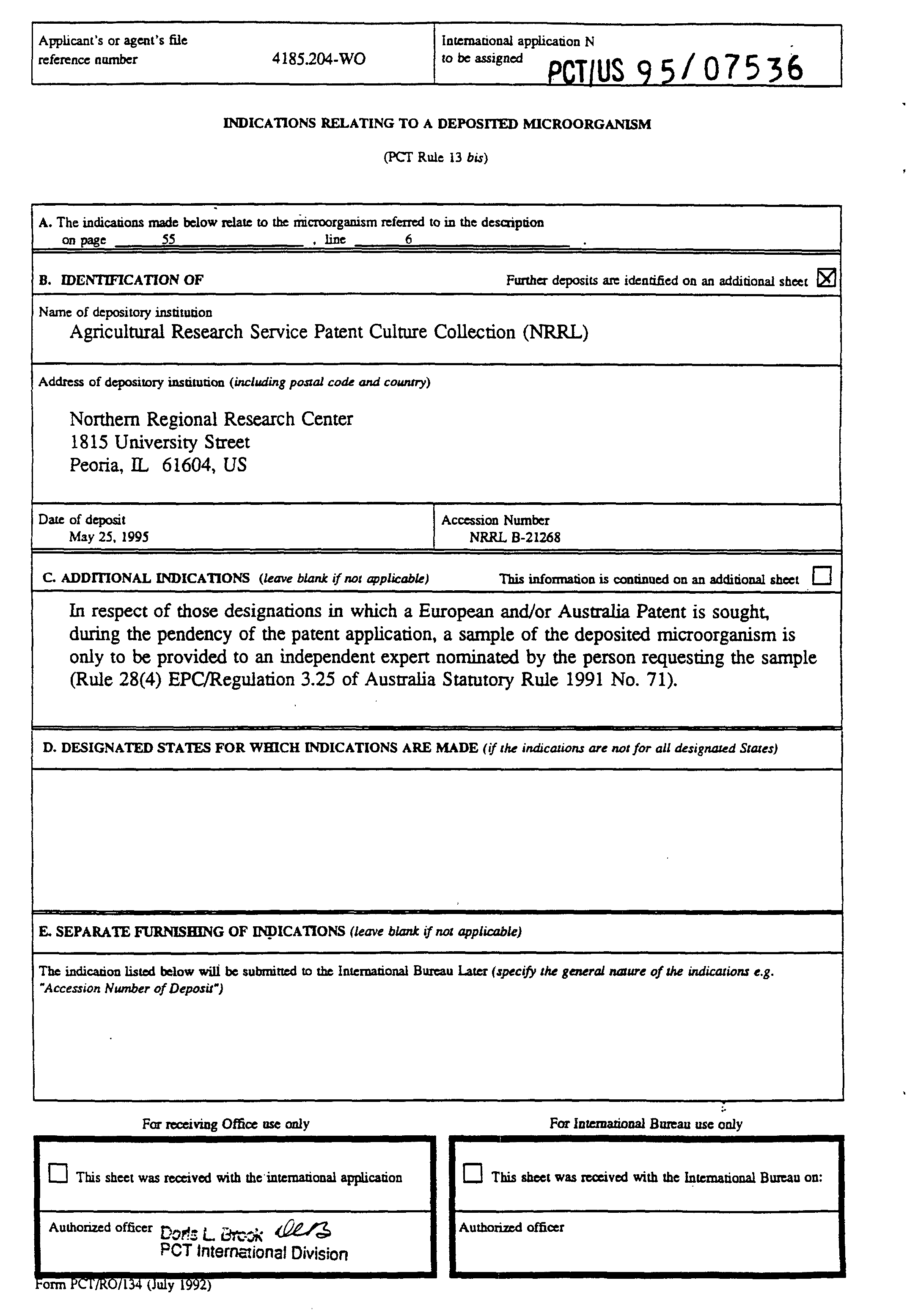

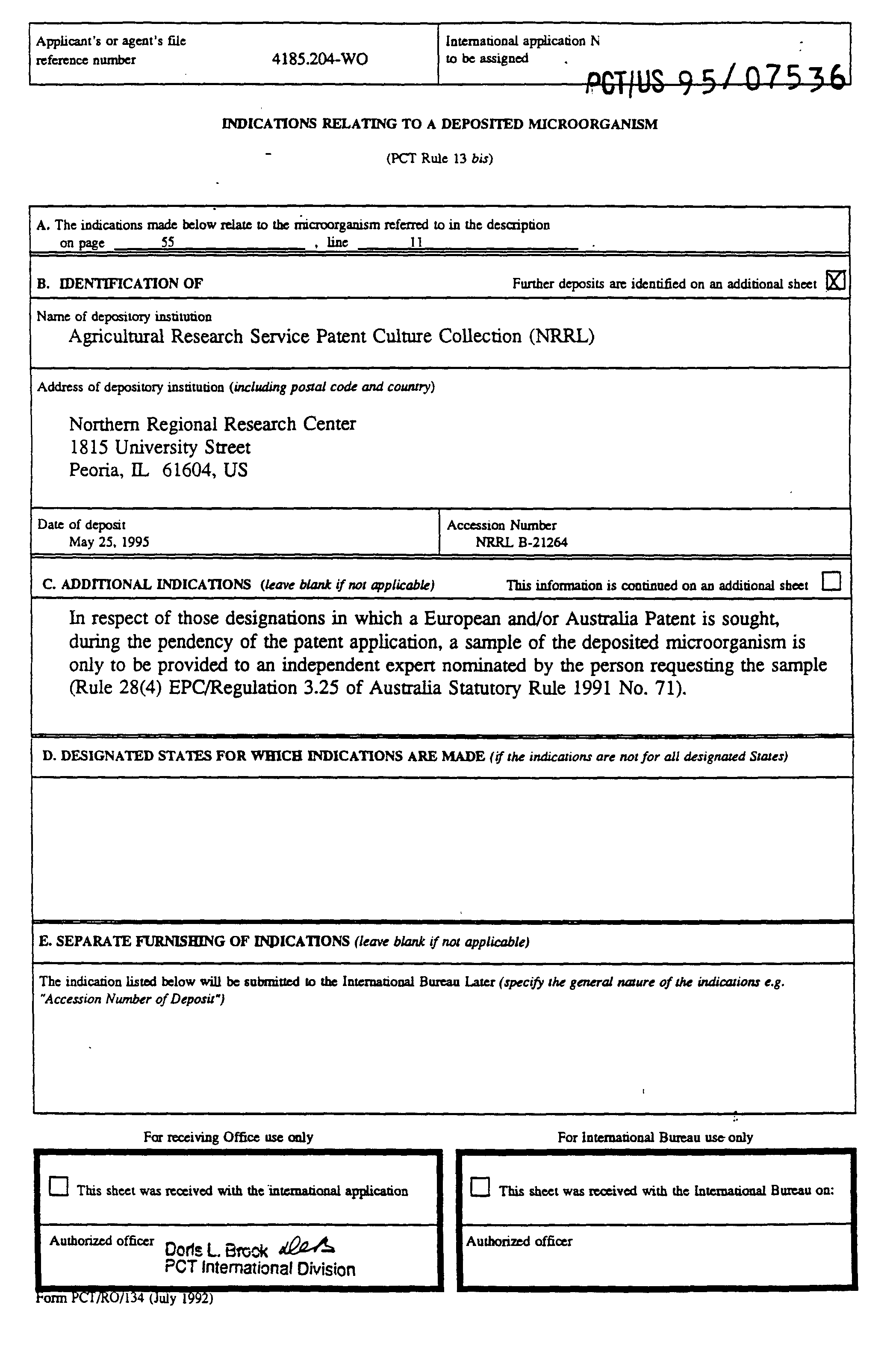

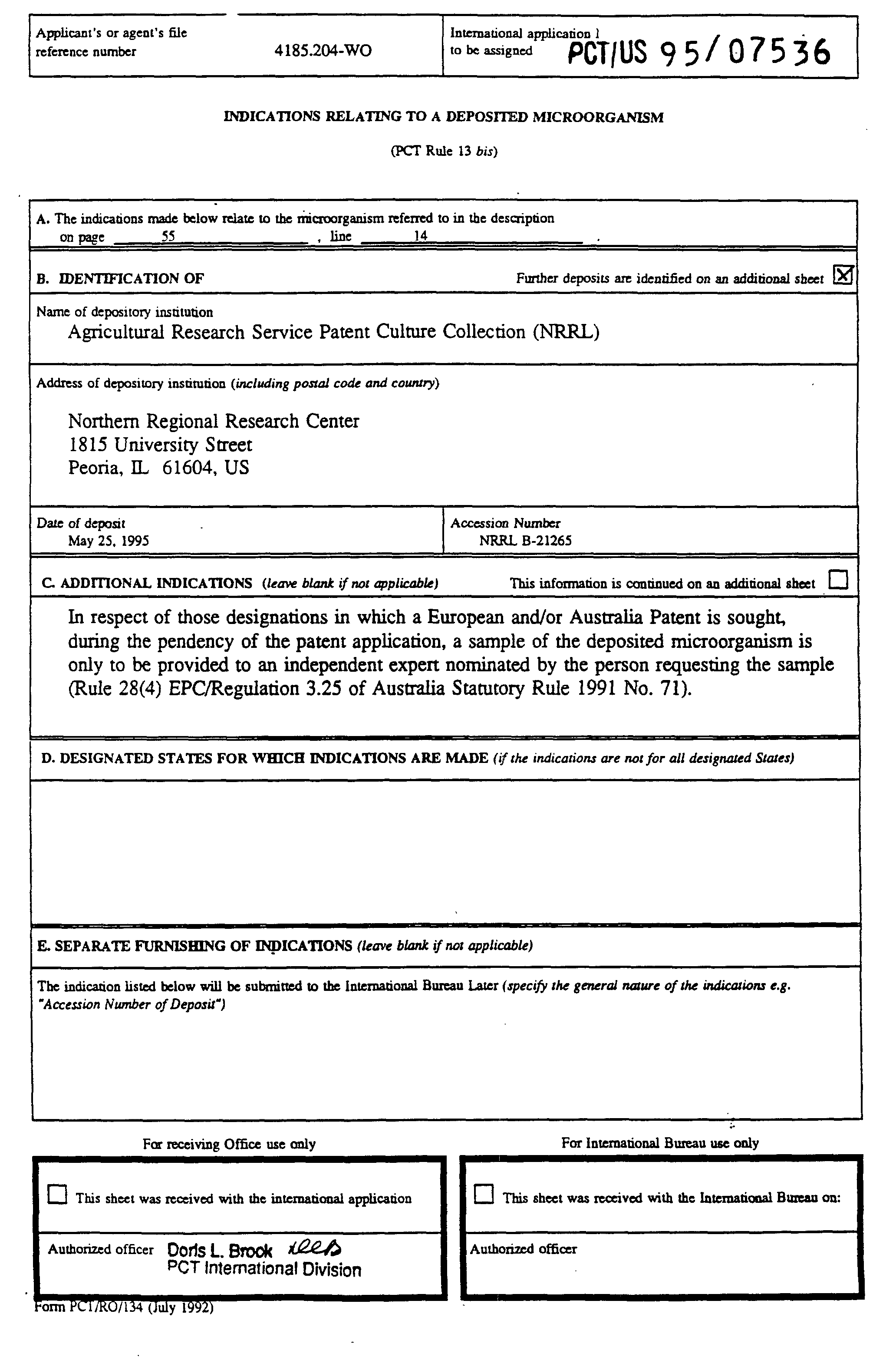

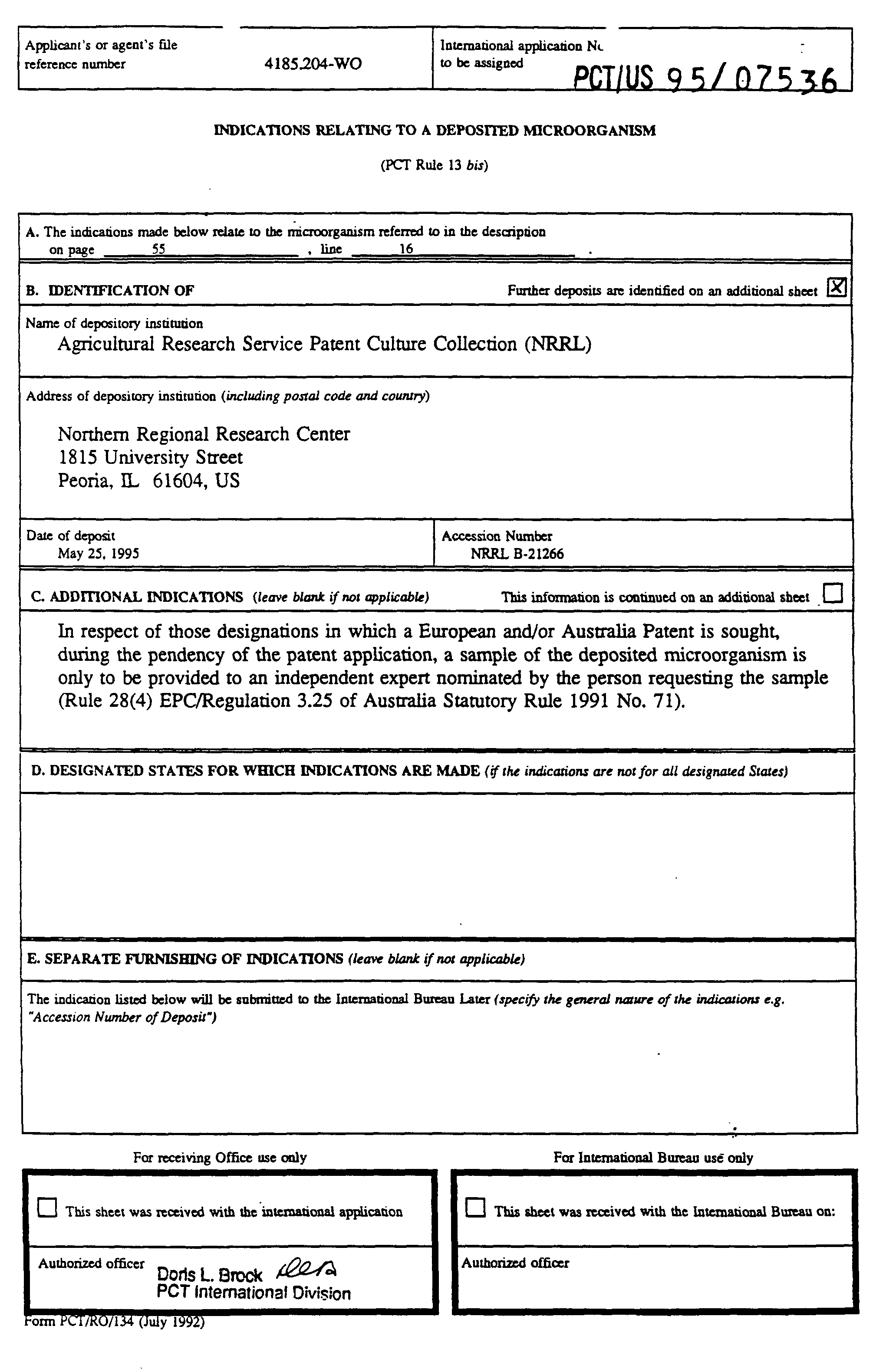

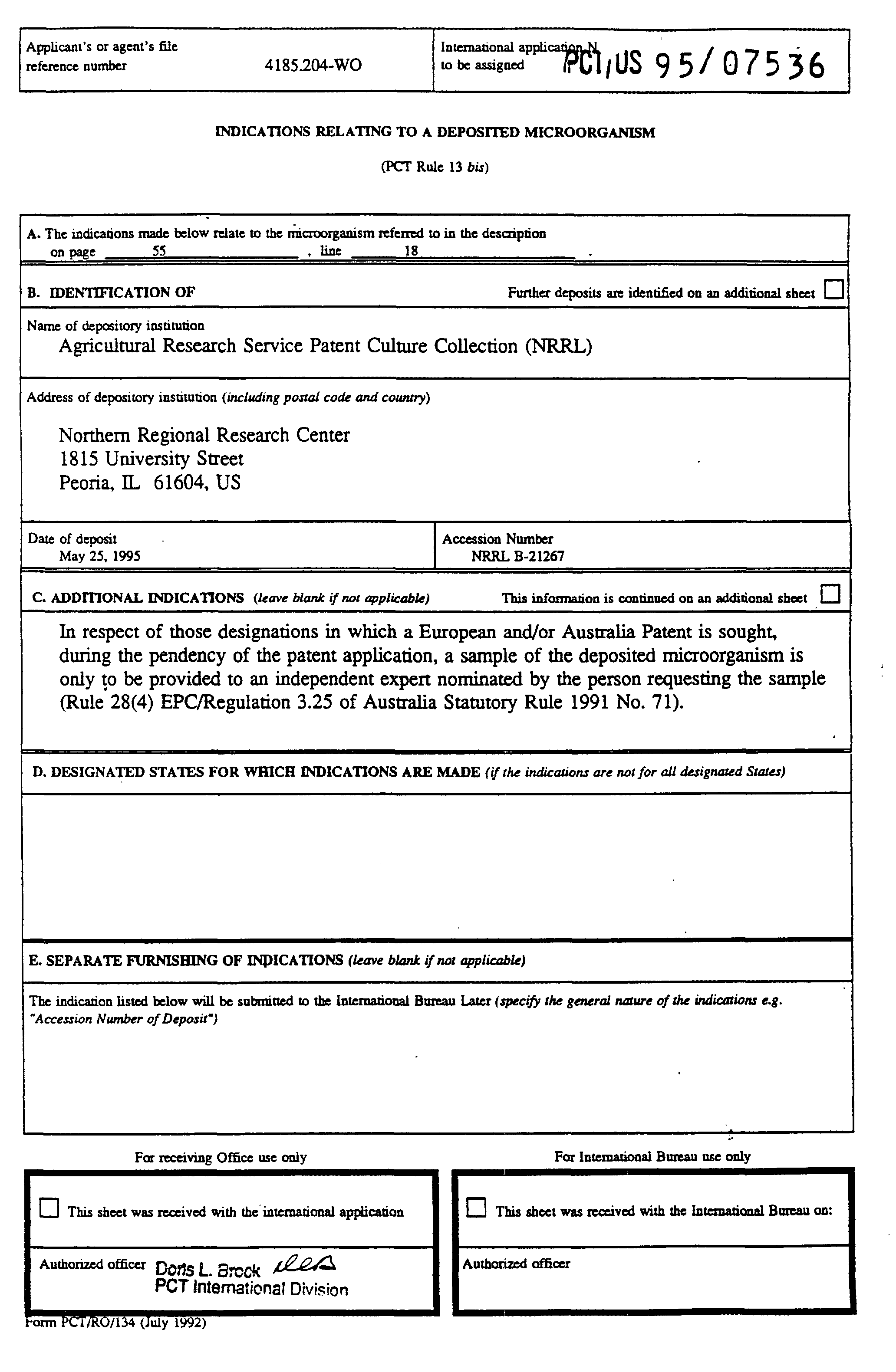

## Description

### Field of the Invention

The present invention relates to isolated nucleic acid fragments encoding a fungal oxidoreductase enzyme and the purified enzymes produced thereby. More particularly, the invention relates to nucleic acid fragments encoding a phenol oxidase, specifically a laccase, of a basidiomycete, *Polyporus.*

### Background of the Invention

Laccases (benzenediol:oxygen oxidoreductases) are multi-copper-containing enzymes that catalyze the oxidation of phenolics. Laccase-mediated oxidations result in the production of aryloxy-radical intermediates from suitable phenolic substrate; the ultimate coupling of the intermediates so produced provides a combination of dimeric, oligomeric, and polymeric reaction products. Such reactions are important in nature in biosynthetic pathways which lead to the formation of melanin, alkaloids, toxins, lignins, and humic acids. Laccases are produced by a wide variety of fungi, including ascomycetes such as *Aspergillus, Neurospora,* and *Podospora,* the deuteromycete *Botrytis*, and basidiomycetes such as *Collybia, Fomes, Lentinus, Pleurotus, Trametes, Polyporus* and perfect forms of *Rhizoctonia.* Laccases exhibit a wide range of substrate specificity, and each different fungal laccase usually differs only quantitatively from others in its ability to oxidize phenolic substrates. Because of the substrate diversity, laccases generally have found many potential industrial applications. Among these are lignin modification, paper strengthening, dye transfer inhibition in detergents, phenol polymerization, juice manufacture, phenol resin production, and waste water treatment.

Although the catalytic capabilities are similar, laccases made by different fungal species do have different temperature and pH optima, and these may also differ depending on the specific substrate. A number of these fungal laccases have been isolated, and the genes for several of these have been cloned. For example, Choi *et al.* (Mol. Plant-Microbe Interactions 5: 119-128, 1992) describe the molecular characterization and cloning of the gene encoding the laccase of the chestnut blight fungus, *Cryphonectria parasitica.* Kojima *et al.* (J. Biol. Chem. 265: 15224-15230, 1990; JP 2-238885) provide a description of two allelic forms of the laccase of the white-rot basidiomycete *Coriolus hirsutus.* Germann and Lerch (Experientia 41: 801,1985; PNAS USA 83: 8854-8858, 1986) have reported the cloning and partial sequencing of the *Neurospora crassa* laccase gene. Saloheimo *et al.* (J. Gen. Microbiol. 137: 1537-1544, 1985; WO 92/01046) have disclosed a structural analysis of the laccase gene from the fungus *Phlebia radiata.*

Attempts to express laccase genes in heterologous fungal systems frequently give very low yields(Kojima et al., *supra;* Saloheimo et al., Bio/Technol. 9: 987-990, 1991). For example, heterologous expression of *Phlebia radiata* laccase in *Trichoderma reesei* gave only 20 mg per liter of active enzyme in lab-scale fermentation(Saloheimo, 1991, *supra*). Although laccases have great commercial potential, the ability to express the enzyme in significant quantities is critical to their commercial utility. Previous attempts to express basidiomycete laccases in recombinant hosts have resulted in very low yields. The present invention now provides novel basidiomycete laccases which are well expressed in *Aspergillus.*

DE-C-4033246 discloses a process for production of *Polyporus pinsitus* laccase from potato juice using 2,5-xylidine as an inducer.

In Appl. Environ. Microbiol. 48: 849-854 (1984), it is disclosed that stimulation of the extracellular laccase formation by 2,5-xylidine was obtained in *Trametes versicolor.*

WO95/01426 discloses the purification of a laccase from *Trametes villosa* culture broth.

### SUMMARY OF THE INVENTION

The present invention relates to a DNA construct containing a nucleic acid sequence selected from SEQ ID NOS. 1, 3, 5, 7 and 9 encoding a *Polyporus* laccase selected from SEQ ID NOS. 2, 4, 6, 8 and 10. The invention also relates to an isolated laccase encoded by a nucleic acid sequence selected from SEQ ID NOS. 4, 6, 8 and 10. Preferably, the laccase is substantially pure. By "substantially pure" is meant a laccase which is essentially (i.e.,≥90%) free of other non-laccase proteins.

In order to facilitate production of the novel laccase, the invention also provides vectors and host cells comprising the claimed nucleic acid sequence, which vectors and host cells are useful in recombinant production of the laccase. The sequence is operably linked to transcription and translation signals capable of directing expression of the laccase protein in the host cell of choice. A preferred host cell is a fungal cell, most preferably of the genus *Aspergillus.* Recombinant production of the laccase of the invention is achieved by culturing a host cell transformed or transfected with the construct of the invention, or progeny thereof, under conditions suitable for expression of the laccase protein, and recovering the laccase protein from the culture.

The laccases of the present invention are useful in a number of industrial processes in which oxidation of phenolics is required. These processes include lignin manipulation, juice manufacture, oxidation of dyes or dye precursors (eg. for hair dying), phenol polymerization and phenol resin production.

### BRIEF DESCRIPTION OF THE FIGURES

Figure 1 shows the DNA sequence and translation of genomic clone 21GEN, containing LCC1 (SEQ ID NO. 1)
Figure 2 shows the DNA sequence and translation of genomic clone 23GEN, containing LCC2 (SEQ ID NO. 3)
Figure 3 shows the DNA sequence and translation of genomic clone 24GEN, containing LCC3 (SEQ ID NO. 5)
Figure 4 shows the DNA sequence and translation of genomic clone 31GEN, containing LCC4 (SEQ ID NO. 7)
Figure 5 shows the DNA sequence and translation of genomic clone 41GEN, containing LCC5 (SEQ ID NO. 9)
Figure 6 shows the structure of vector pMWR1
Figure 7 shows the structure of vector pDSY1
Figure 8 shows the structure of vector pDSY10
Figure 9 shows the pH profile of the laccase produced by pDSY2; (A) syringaldazine oxidation; (B) ABTS oxidation.
Figure 10 illustrates a comparison of the use of laccase vs. H₂O₂, with various dye precursors, in hair dyeing, as a measurement of DL*.
Figure 11 illustrates a comparison of the use of laccase vs. H₂O₂, with various dye precursors, in hair dyeing, as a measurement of Da*.
Figure 12 illustrates a comparison of the use of laccase vs. H₂O₂, with various dye precursors and modifiers, in hair dyeing, as a measurement of DL*.
Figure 13 illustrates a comparison of the wash stability of hair dyed with laccase vs. H₂O₂.
Figure 14 illustrates the light fastness of hair dyed with laccase vs. H₂O₂.

### Detailed Description of the Invention

*Polyporus pinsitus* is a basidiomycete, also referred to as *Trametes villosa. Polyporus* species have previously been identified as laccase producers(Fahraeus and Lindeberg, Physiol. Plant. 6: 150-158, 1953). However, there has been no previous description of a purified laccase from *Polyporus pinsitus.* It has now been determined that *Polyporus* *pinsitus* produces at least two different laccases, and the genes encoding these laccases can be used to produce relatively large yields of the enzyme in convenient host systems such as *Aspergillus.* In addition, three other genes which appear to code for laccases have also been isolated.

Initial screenings of a variety of fungal strains indicate that *Polyporus pinisitus* is a laccase producer. The production of laccase by *P. pinsitus* is induced by 2,5-xylidine. Attempts are first initiated to isolate the laccase from the supernatant of the induced strains. Anion exchange chromatography identifies an approximately 65 kD(on SDS-PAGE) protein which exhibits laccase activity. The enzyme is purified sufficiently to provide several internal peptide sequences, as well as an N-terminal sequence. The initial sequence information indicates the laccase has significant homology to that of *Coriolus hirsutus,* as well as to an unidentified basidiomycete laccase (Coll et al., Appl. Environ. Microbiol. 59: 4129-4135, 1993. Based on the sequence information, PCR primers are designed and PCR carried out on cDNA isolated from *P. pinsitus.* A band of the expected size is obtained by PCR, and the isolated fragment linked to a cellulase signal sequence is shown to express an active laccase in *A. oryzae*, but at low levels. One of the PCR fragments is also used as a probe in screening a *P*. *pinsitus* cDNA library. In this manner, more than 100 positive clones are identified. The positive clones are characterized and the ends of the longest clones sequenced; none of the clones are found to be full-length.

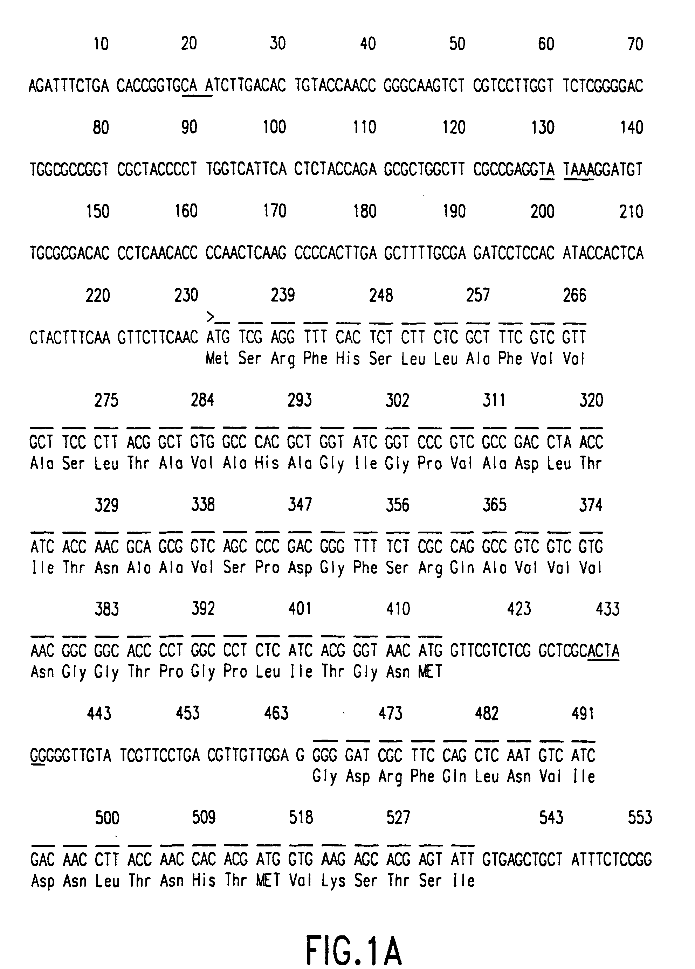

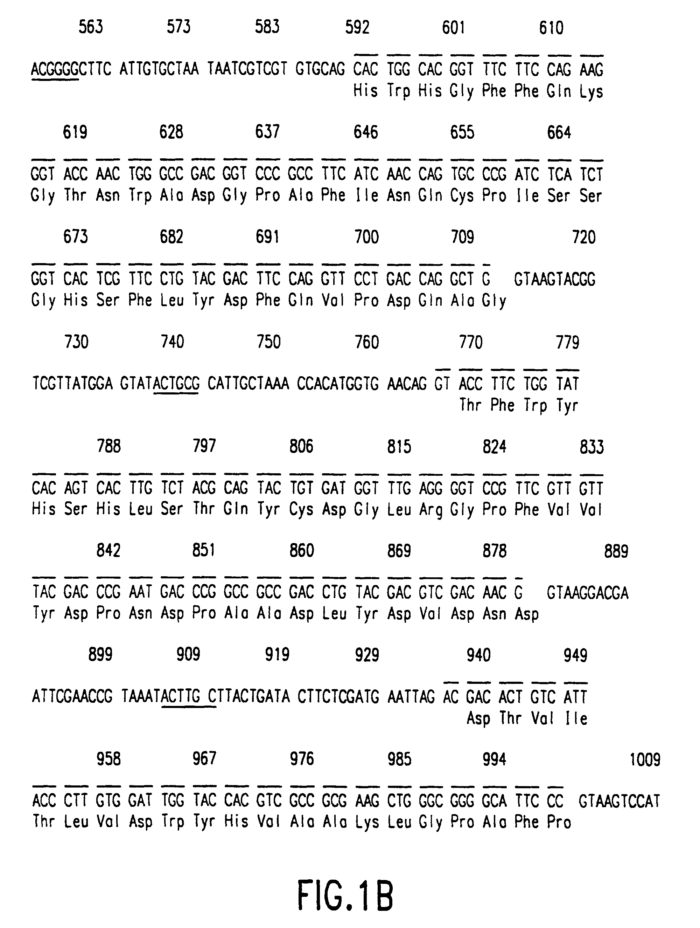

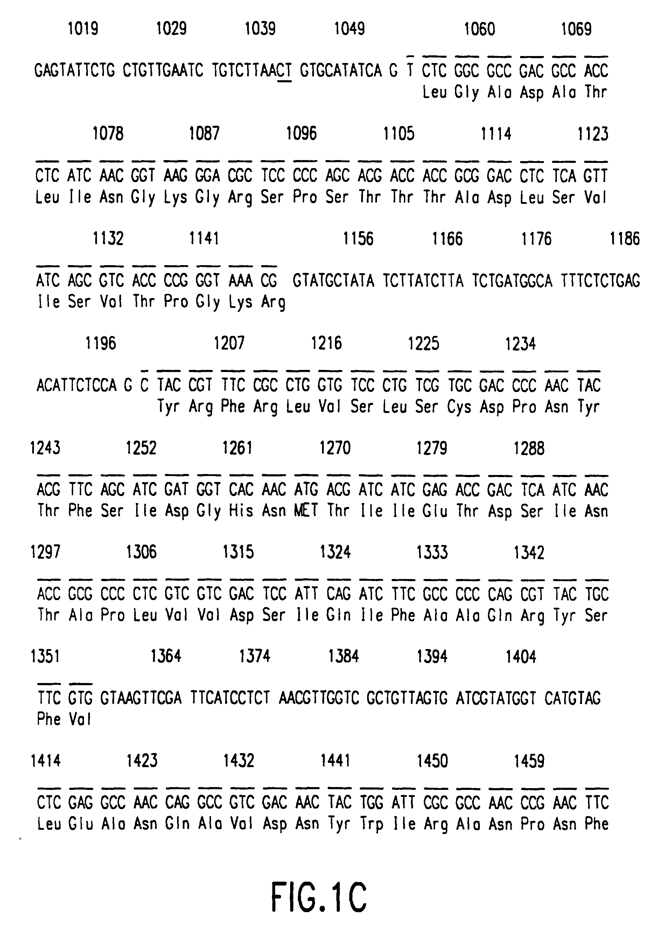

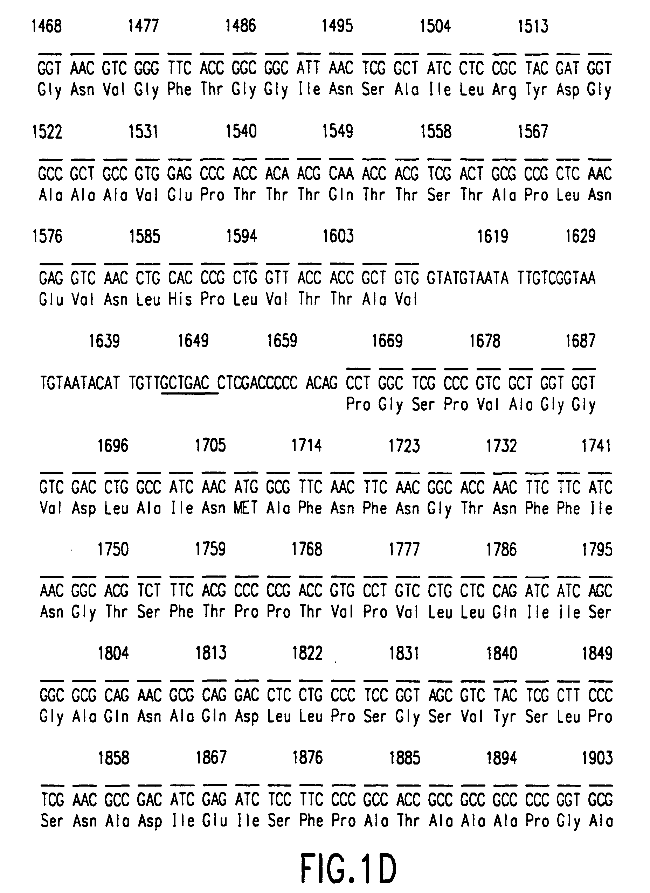

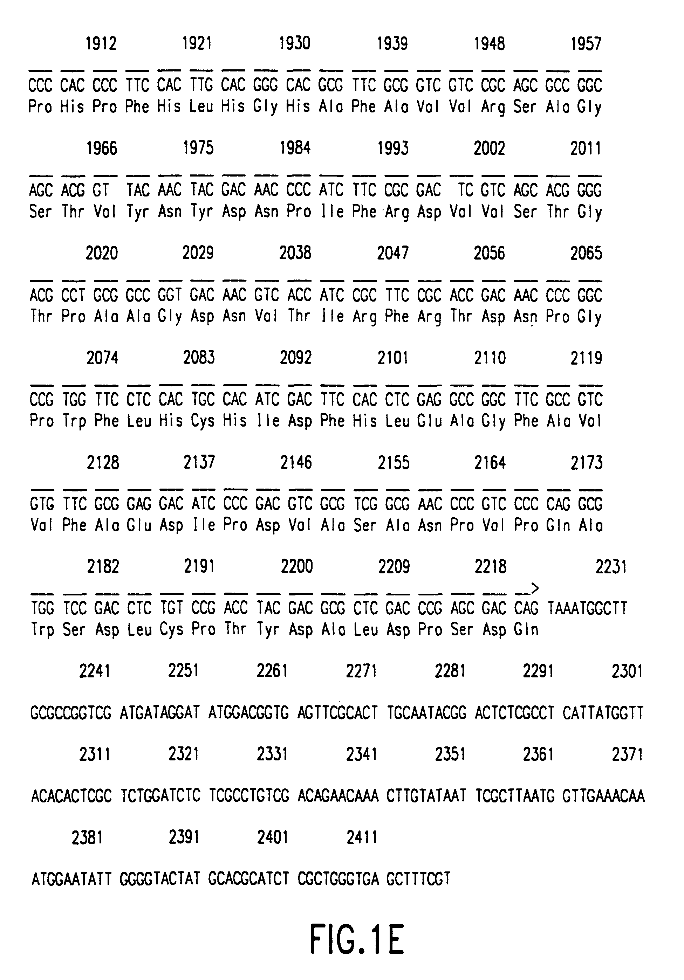

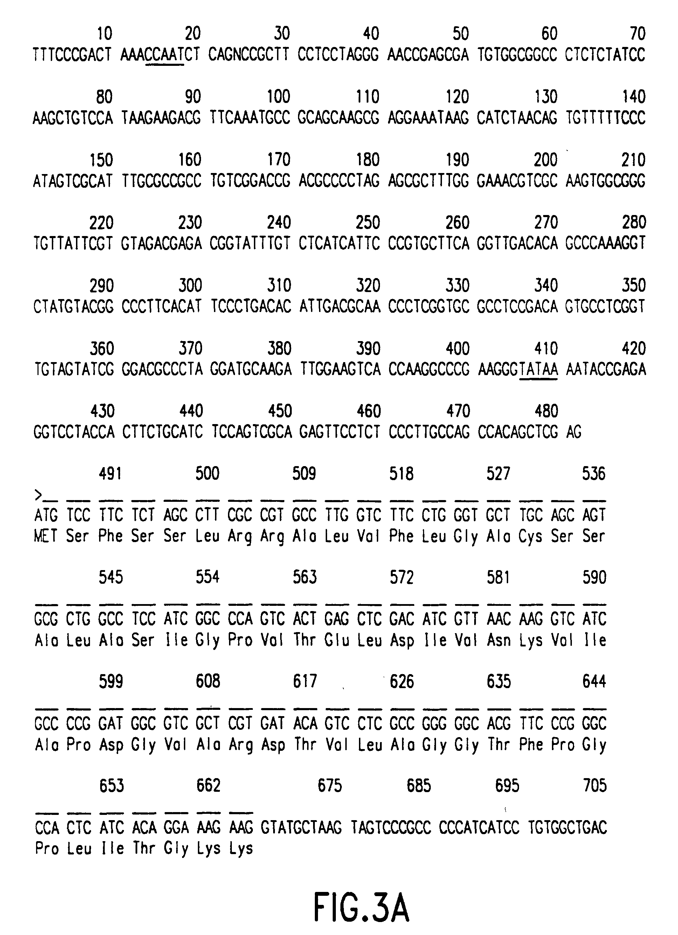

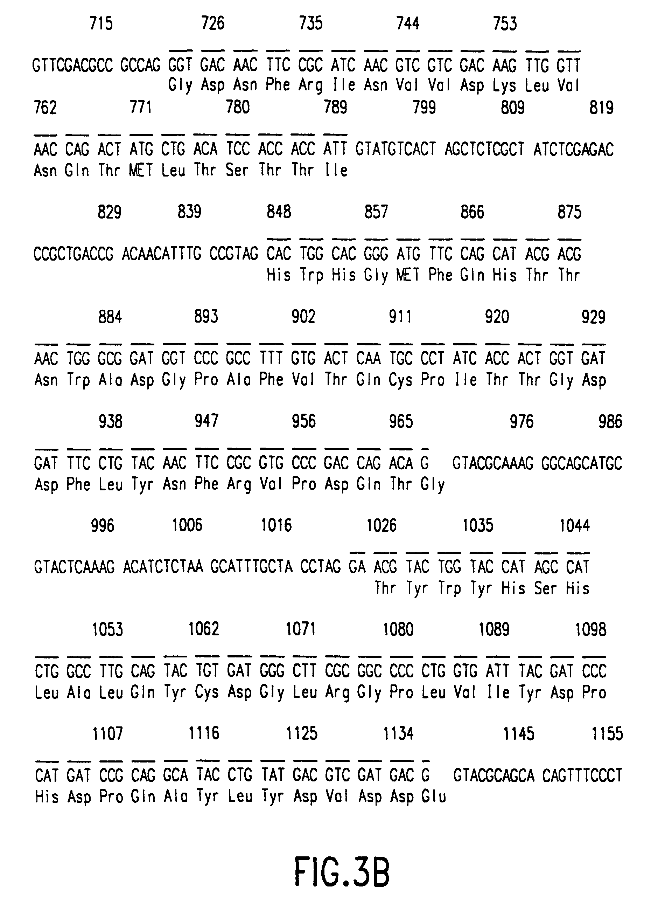

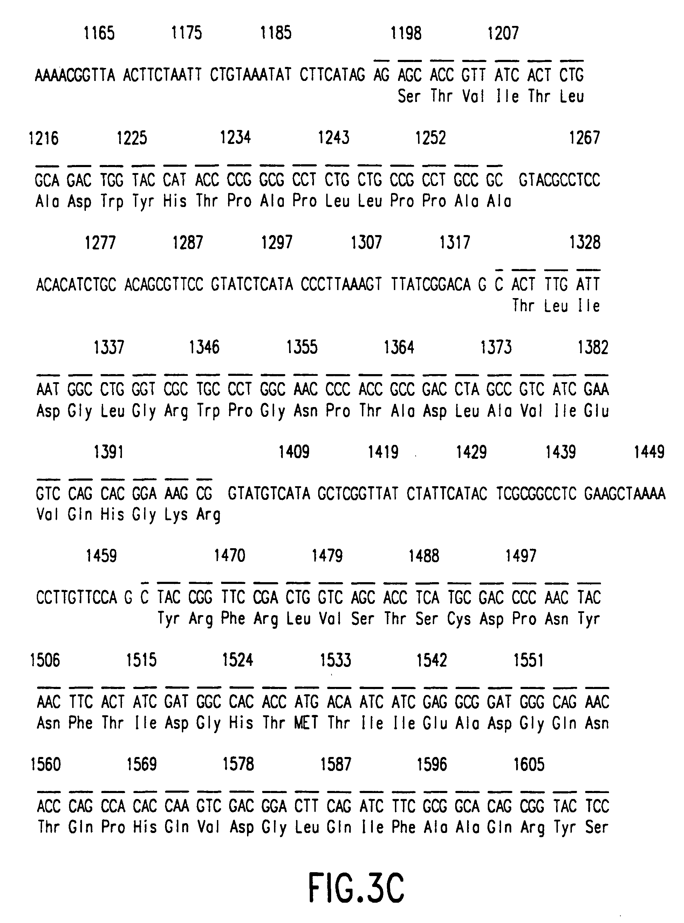

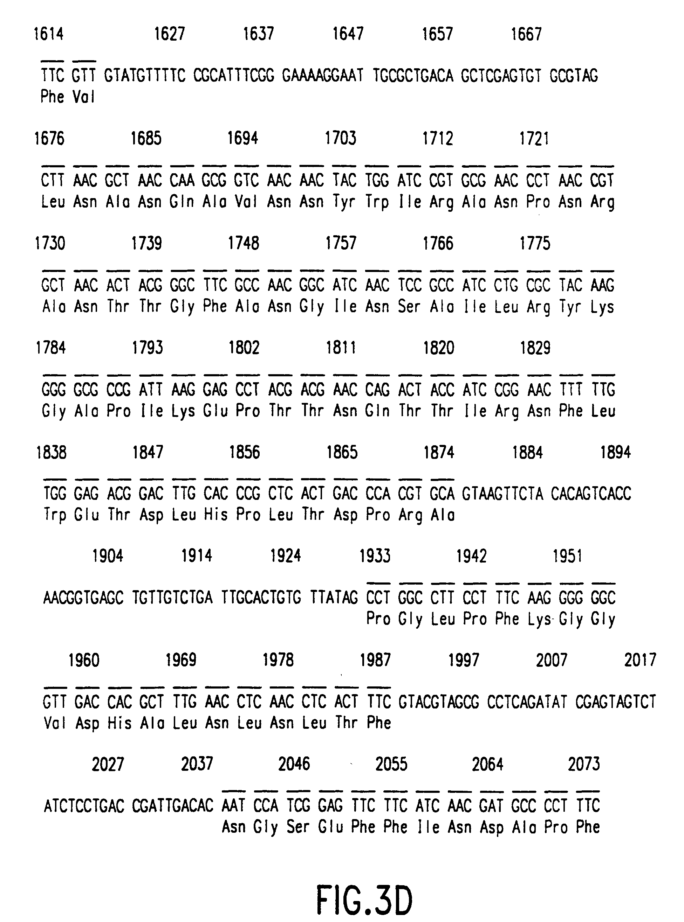

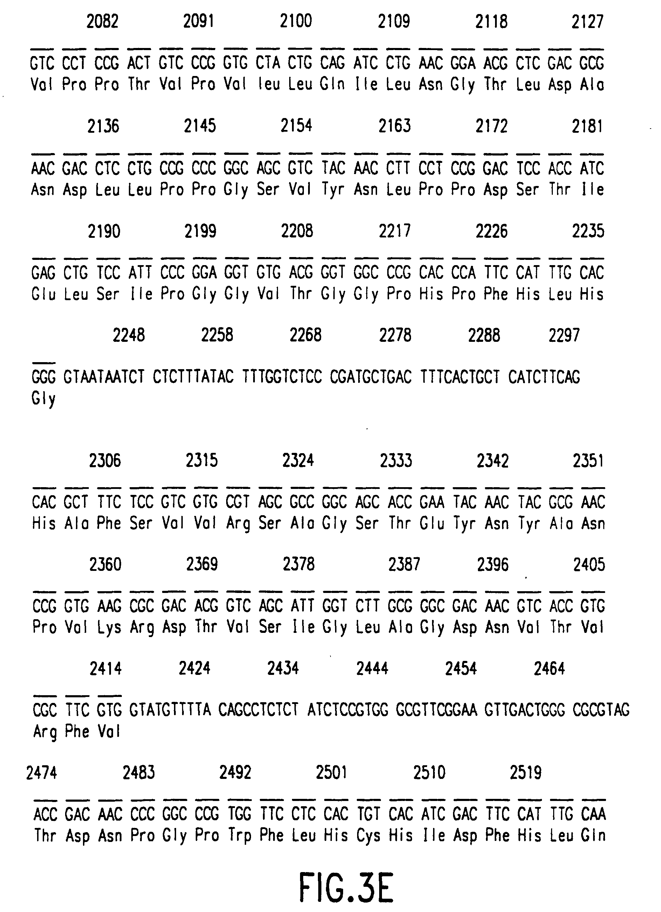

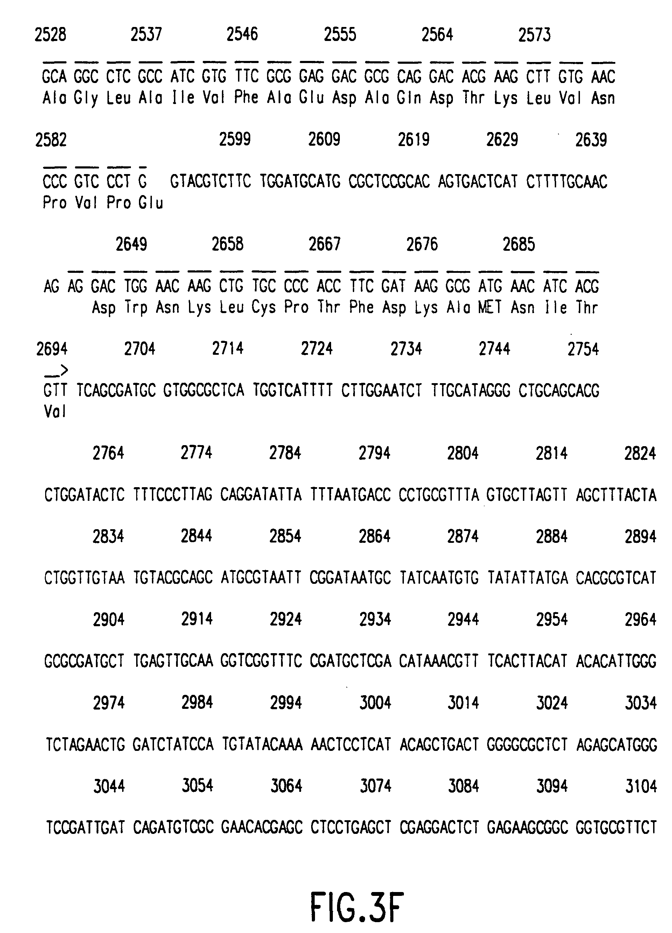

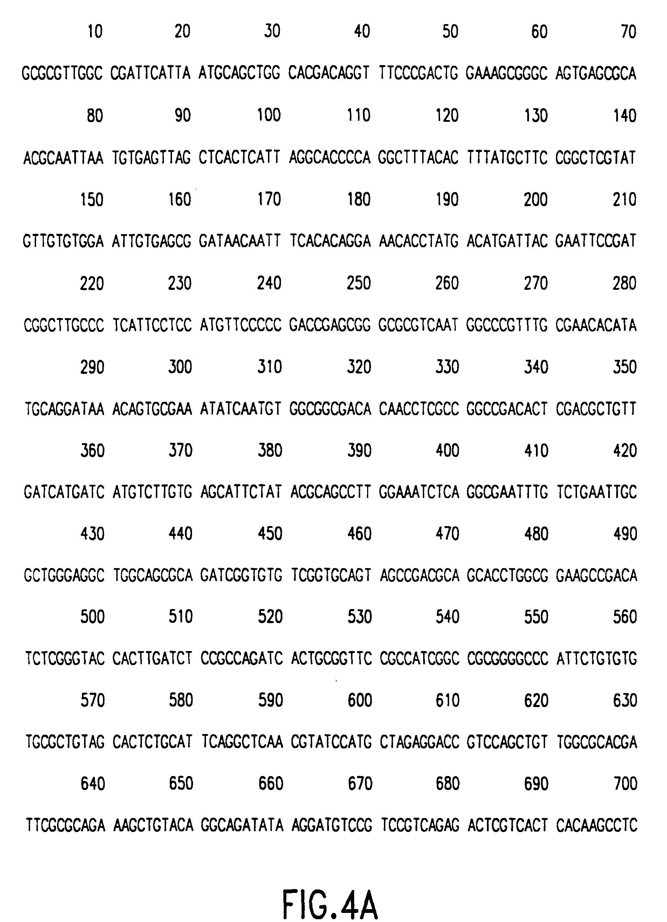

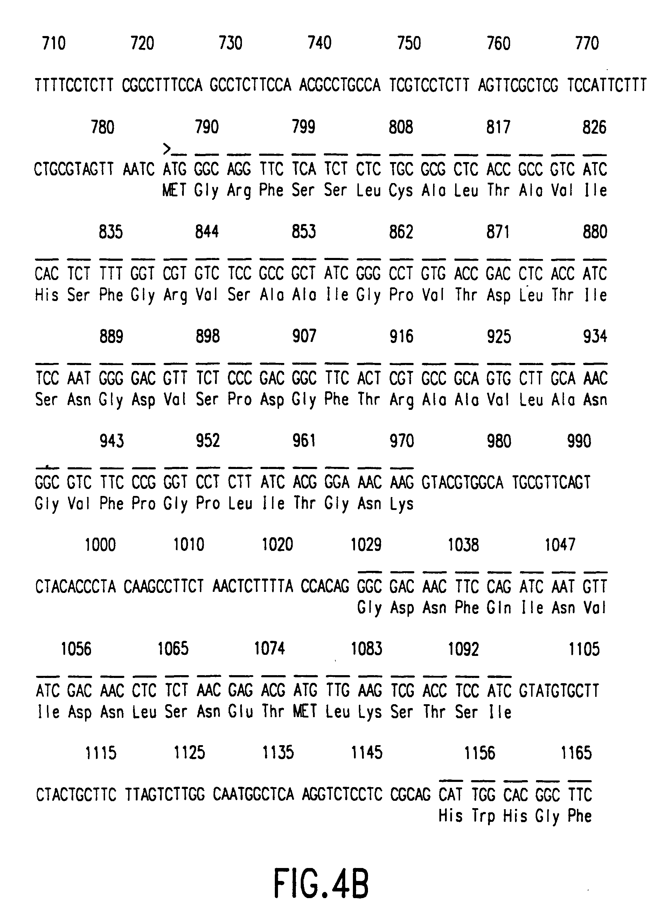

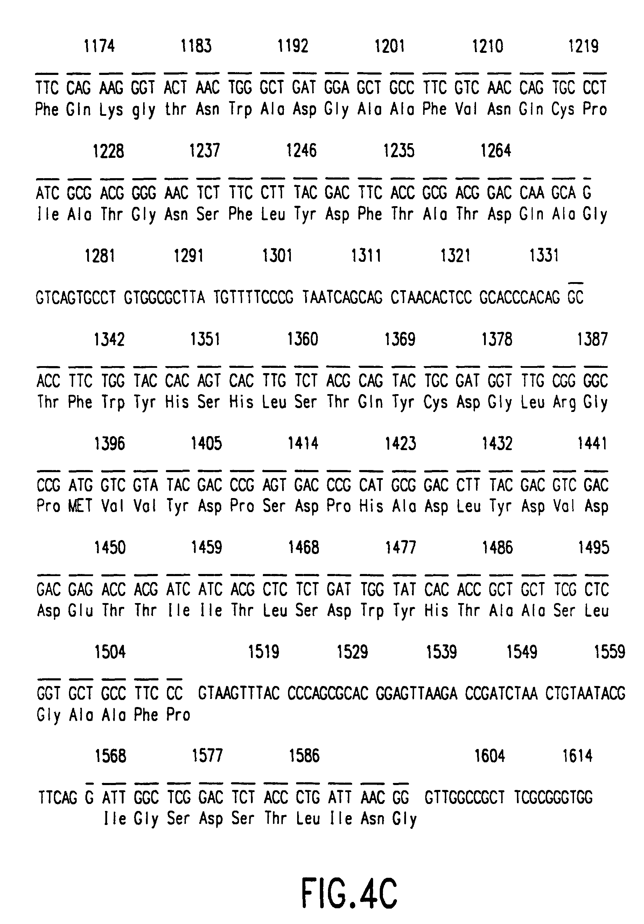

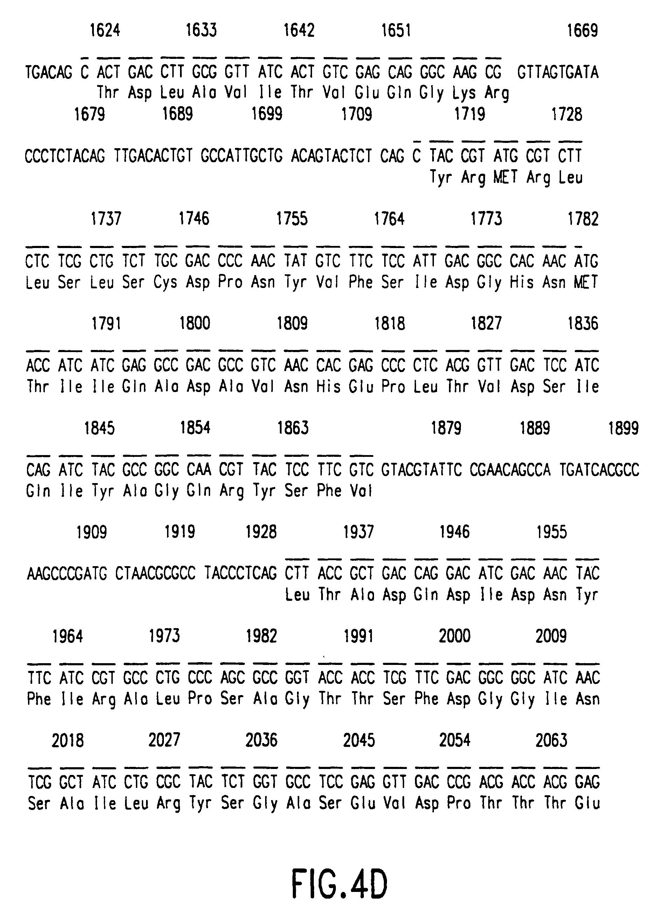

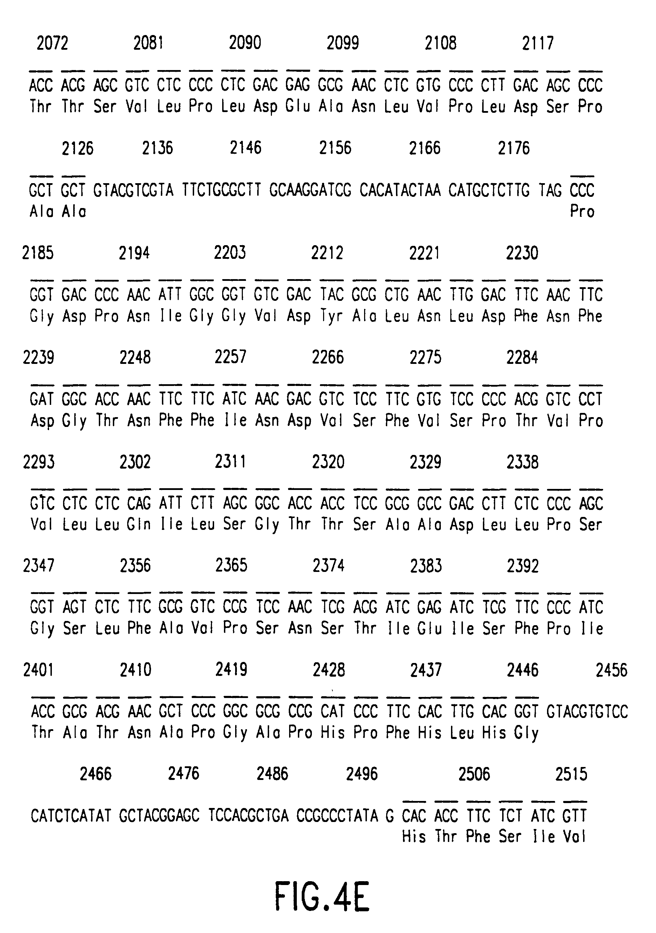

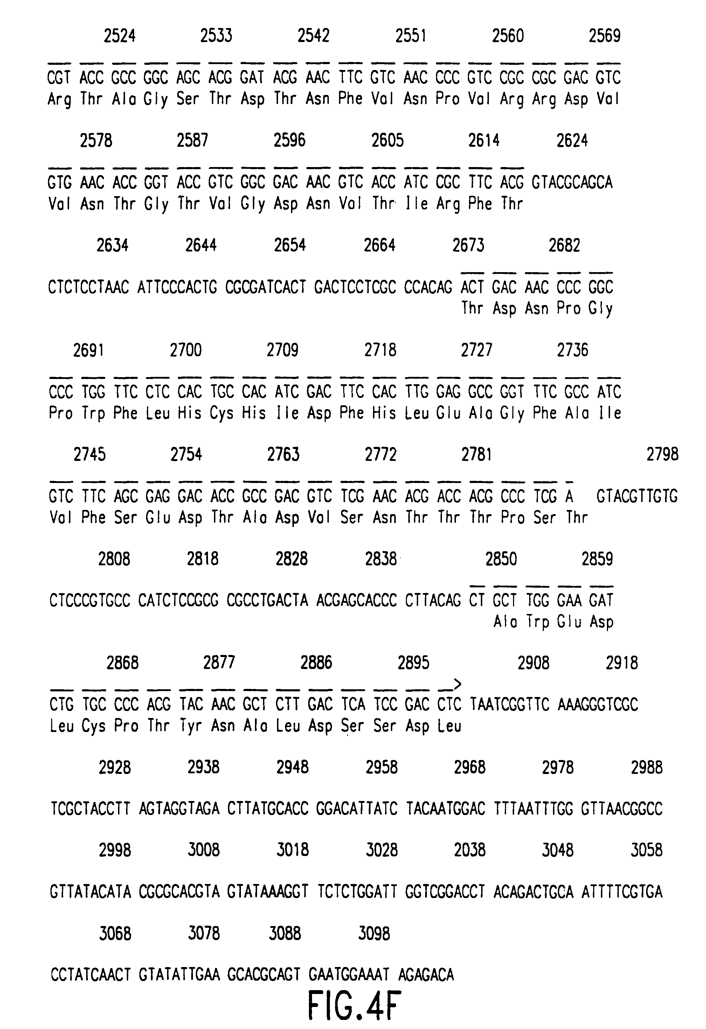

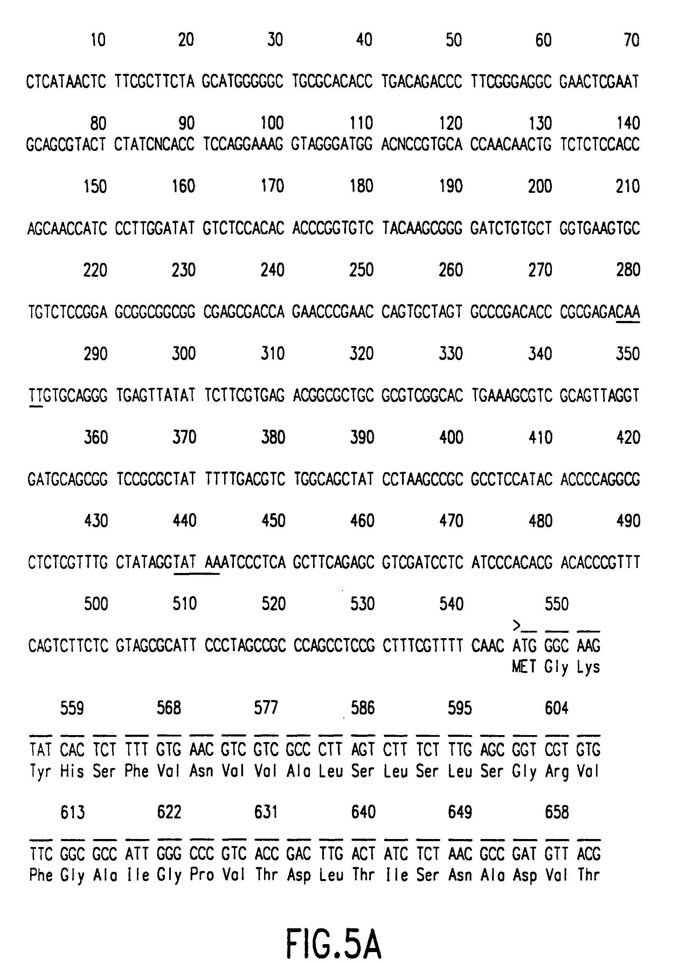

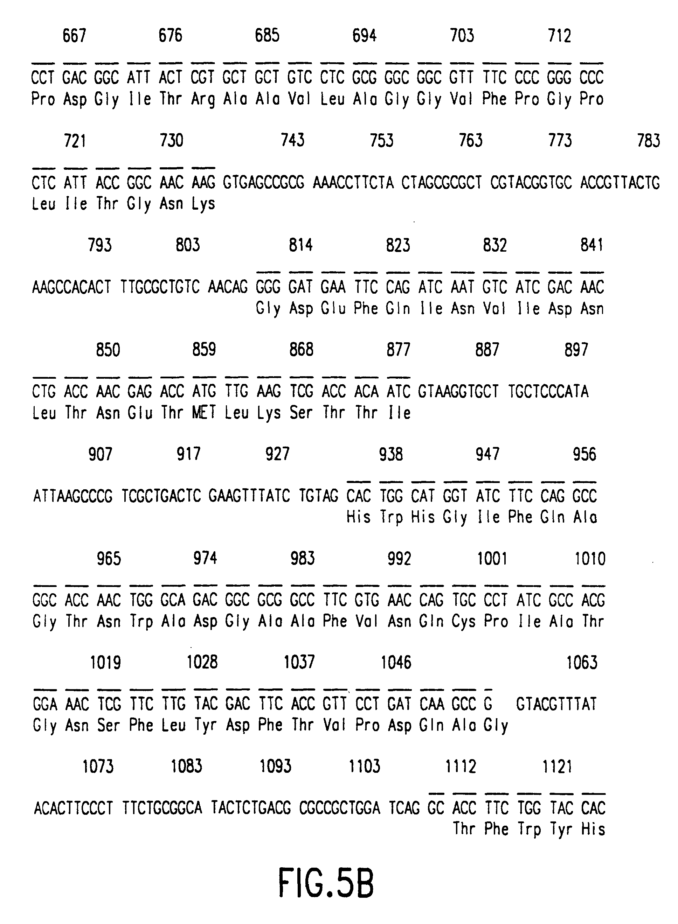

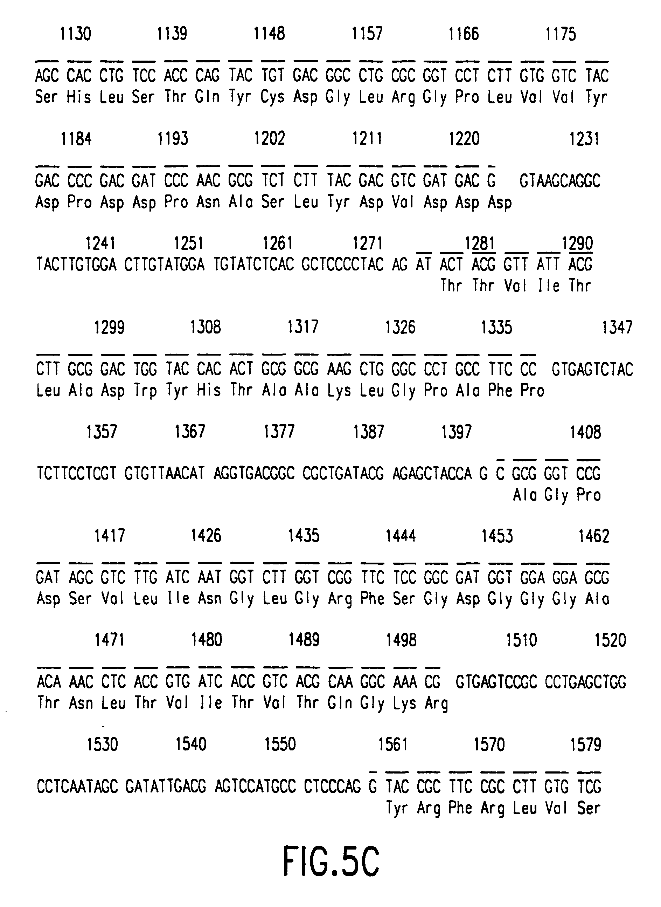

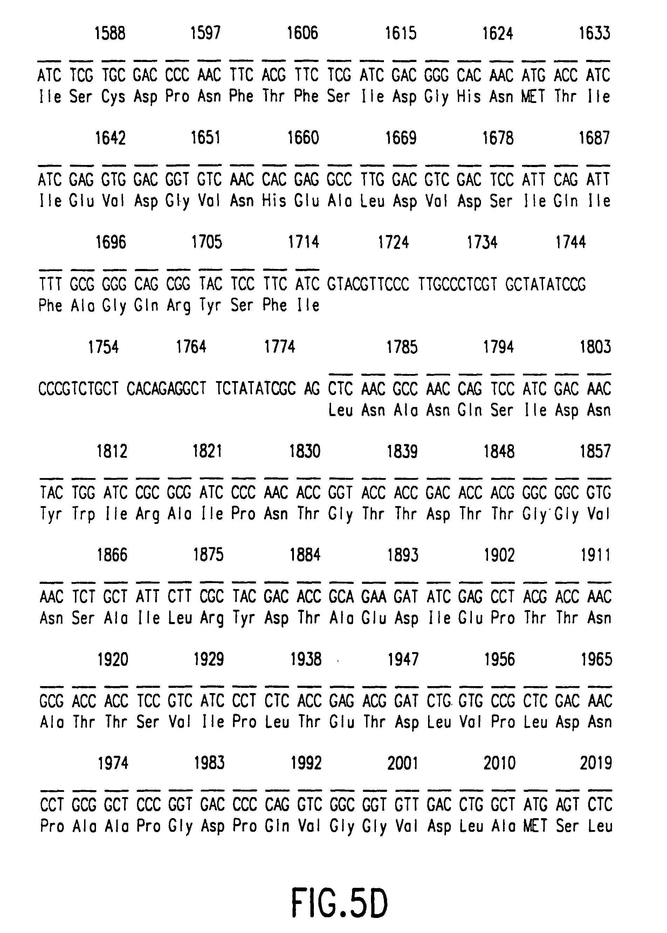

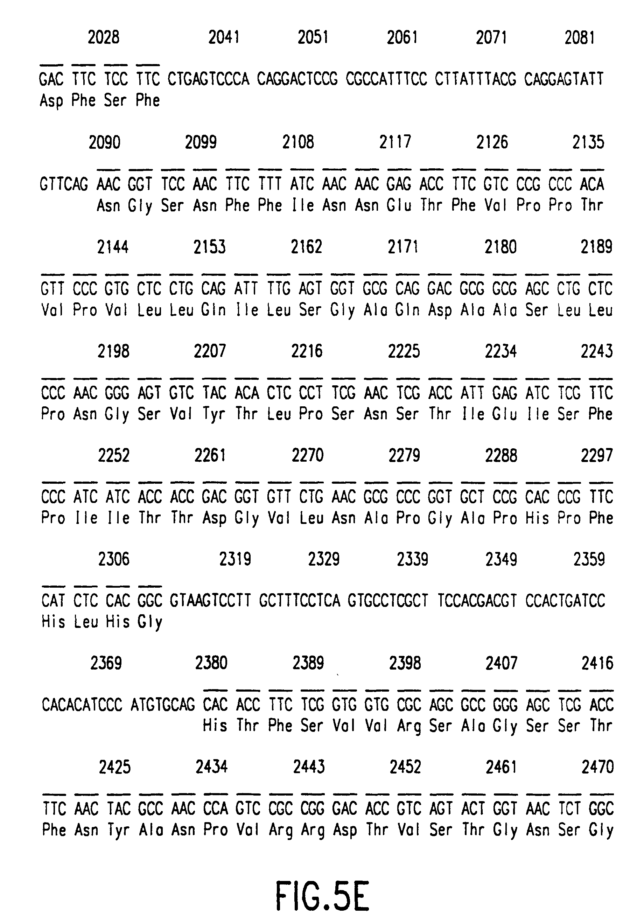

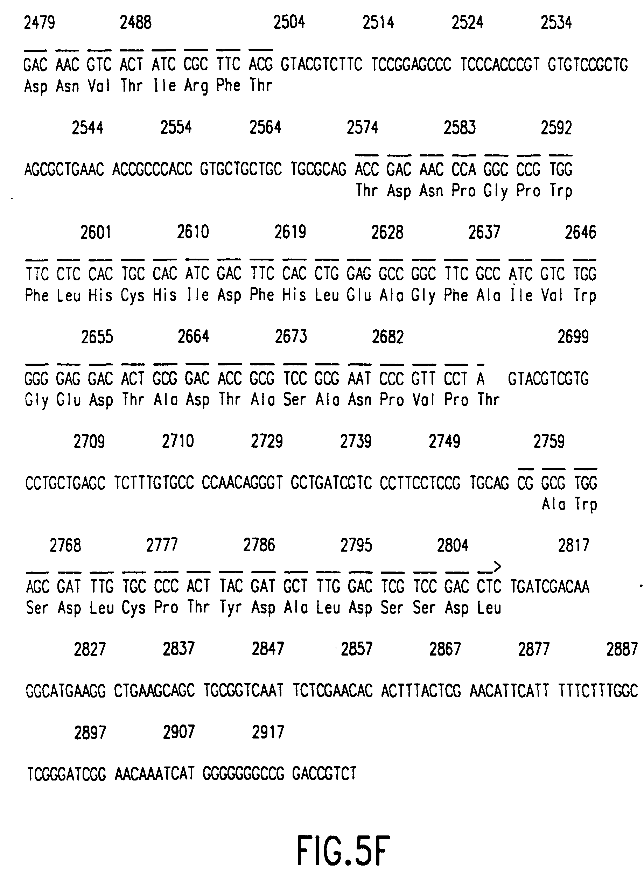

Further attempts to isolate a full length clone are made. A 5-6 kb BamHI size-selected *P. pinsitus* genomic library is probed with the most complete cDNA fragment isolated as described above. Initial screening identifies one clone 24GEN(LCC3) having homology to the cDNA, but which is not the cDNA-encoded laccase and also not full length. Subsequent screening of a 7-8kb BamHI/EcoRi size-selected library indicates the presence of at least two laccases; partial sequencing shows that one, called 21GEN(LCC1), is identical to the original partial cDNA clone isolated, and the second,called 31GEN(LCC4) is a new, previously unidentified laccase. Secondary screenings of an EMBL4 genomic bank with LCC1 as probe identifies a class of clone containing the entire LCC1 insert as well as the 5' and 3' flanking regions. Screening of the EMBL bank with LCC3 identifies two additional clones encoding laccases which had not previously been identified,41GEN(LCC5) and 23GEN(LCC2) and which differed structurally from the other three clones LCC1, LCC3, and LCC4. The nucleic acid and predicted amino acid sequences of each of the laccases is presented in Figures 1-5, and in SEQ ID NOS. 1-10. A comparison of the structural organization of each of the laccases is presented in Table 2. The laccases are generally optimally active at acid pH, between about 4-5.5.

LCC1 is used to create expression vectors, which are in turn used to transform various species of *Aspergillus.* Transformation is successful in all species tested, although expression levels are highest in *Aspergillus niger.* Shake flask cultures are capable of producing 15 or more mg/liter of laccase, and in lab-scale fermentors, yields of over 300mg/liter are observed. This is a significant improvement over laccase levels observed previously with other laccases and other fungal host cells.

According to the invention, a *Polyporus* gene encoding a laccase can be obtained by methods described above, or any alternative methods known in the art, using the information provided herein. The gene can be expressed, in active form, using an expression vector. A useful expression vector contains an element that permits stable integration of the vector into the host cell genome or autonomous replication of the vector in a host cell independent of the genome of the host cell, and preferably one or more phenotypic markers which permit easy selection of transformed host cells. The expression vector may also include control sequences encoding a promoter, ribosome binding site, translation initiation signal, and, optionally, a repressor gene or various activator genes. To permit the secretion of the expressed protein, nucleotides encoding a signal sequence may be inserted prior to the coding sequence of the gene. For expression under the direction of control sequences, a laccase gene to be used according to the invention is operably linked to the control sequences in the proper reading frame. Promoter sequences that can be incorporated into plasmid vectors, and which can direct the transcription of the laccase gene, include but are not limited to the prokaryotic β-lactamase promoter (Villa-Kamaroff, et al., 1978, Proc. Natl. Acad. Sci. U.S.A. 75:3727-3731) and the tac promoter (DeBoer, et al., 1983, Proc. Natl. Acad. Sci. U.S.A. 80:21-25). Further references can also be found in "Useful proteins from recombinant bacteria" in Scientific American, 1980, 242:74-94; and in Sambrook et al., Molecular Cloning, 1989.

The expression vector carrying the DNA construct of the invention may be any vector which may conveniently be subjected to recombinant DNA procedures, and the choice of vector will typically depend on the host cell into which it is to be introduced. Thus, the vector may be an autonomously replicating vector, i.e. a vector which exists as an extrachromosomal entity, the replication of which is independent of chromosomal replication, e.g. a plasmid, or an extrachromosomal element, minichromosome or an artificial chromosome. Alternatively, the vector may be one which, when introduced into a host cell, is integrated into the host cell genome and replicated together with the chromosome(s) into which it has been integrated.

In the vector, the laccase DNA sequence should be operably connected to a suitable promoter sequence. The promoter may be any DNA sequence which shows transcriptional activity in the host cell of choice and may be derived from genes encoding proteins either homologous or heterologous to the host cell. Examples of suitable promoters for directing the transcription of the DNA construct of the invention, especially in a bacterial host, are the promoter of the *lac* operon of *E.coli,* the *Streptomyces coelicolor* agarase gene *dagA* promoters, the promoters of the *Bacillus licheniformis* α-amylase gene (*amy*L), the promoters of the *Bacillus stearothermophilus* maltogenic amylase gene (*amy*M), the promoters of the *Bacillus amyloliquefaciens* α-amylase (*amy*Q), or the promoters of the *Bacillus subtilis* xylA and xylB genes. In a yeast host, a useful promoter is the eno-1 promoter. For transcription in a fungal host, examples of useful promoters are those derived from the gene encoding *A. oryzae* TAKA amylase, *Rhizomucor miehei* aspartic proteinase, *A. niger* neutral α-amylase, *A. niger* acid stable α-amylase, *A. niger or A. awamori* glucoamylase (*glaA*), *Rhizomucor miehei* lipase, *A. oryzae* alkaline protease, *A. oryzae* triose phosphate isomerase or *A. nidulans* acetamidase. Preferred are the TAKA-amylase and *glaA* promoters.

The expression vector of the invention may also comprise a suitable transcription terminator and, in eukaryotes, polyadenylation sequences operably connected to the DNA sequence encoding the laccase of the invention. Termination and polyadenylation sequences may suitably be derived from the same sources as the promoter. The vector may further comprise a DNA sequence enabling the vector to replicate in the host cell in question. Examples of such sequences are the origins of replication of plasmids pUC19, pACYC177, pUB110, pE194, pAMB1 and pIJ702.

The vector may also comprise a selectable marker, e.g. a gene the product of which complements a defect in the host cell, such as the *dal* genes from *B.subtilis* or *B.licheniformis,* or one which confers antibiotic resistance such as ampicillin, kanamycin, chloramphenicol or tetracycline resistance. Examples of *Aspergillus* selection markers include *amdS, pyrG, argB, niaD, sC, trpC* and *hygB,* a marker giving rise to hygromycin resistance. Preferred for use in an *Aspergillus* host cell are the *amdS* and *pyrG* markers of *A. nidulans* or *A. oryzae.* A frequently used mammalian marker is the dihydrofolate reductase (DHFR) gene. Furthermore, selection may be accomplished by co-transformation, e.g. as described in WO 91/17243.

It is generally preferred that the expression gives rise to a product which is extracellular. The laccases of the present invention may thus comprise a preregion permitting secretion of the expressed protein into the culture medium. If desirable, this preregion may be native to the laccase of the invention or substituted with a different preregion or signal sequence, conveniently accomplished by substitution of the DNA sequences encoding the respective preregions. For example, the preregion may be derived from a glucoamylase or an amylase gene from an *Aspergillus* species, an amylase gene from a *Bacillus* species, a lipase or proteinase gene from *Rhizomucor miehei,* the gene for the α-factor from *Saccharomyces cerevisiae* or the calf preprochymosin gene. Particularly preferred, when the host is a fungal cell, is the signal sequence for *A. oryzae* TAKA amylase, *A. niger* neutral amylase, the *Rhizomucor miehei* aspartic proteinase signal, the *Rhizomucor miehei* lipase signal, the maltogenic amylase from *Bacillus* NCIB 11837, *B. stearothermophilus* α-amylase, or *B. licheniformis* subtilisin.

The procedures used to ligate the DNA construct of the invention, the promoter, terminator and other elements, respectively, and to insert them into suitable vectors containing the information necessary for replication, are well known to persons skilled in the art (cf., for instance, Sambrook et al. Molecular Cloning, 1989).

The cell of the invention either comprising a DNA construct or an expression vector of the invention as defined above is advantageously used as a host cell in the recombinant production of a enzyme of the invention. The cell may be transformed with the DNA construct of the invention, conveniently by integrating the DNA construct in the host chromosome. This integration is generally considered to be an advantage as the DNA sequence is more likely to be stably maintained in the cell. Integration of the DNA constructs into the host chromosome may be performed according to conventional methods, e.g. by homologous or heterologous recombination. Alternatively, the cell may be transformed with an expression vector as described above in connection with the different types of host cells.

The host cell may be selected from prokaryotic cells, such as bacterial cells. Examples of suitable bacteria are gram positive bacteria such as *Bacillus subtilis, Bacillus licheniformis, Bacillus lentus, Bacillus brevis, Bacillus stearothermophilus, Bacillus alkalophilus, Bacillus amyloliquefaciens, Bacillus coagulans, Bacillus circulans, Bacillus lautus, Bacillus megaterium, Bacillus thuringiensis,* or *Streptomyces lividans* or *Streptomyces* *murinus*, or gram negative bacteria such as *E.coli.* The transformation of the bacteria may for instance be effected by protoplast transformation or by using competent cells in a manner known *per se.*

The host cell may also be a eukaryote, such as mammalian cells, insect cells, plant cells or preferably fungal cells, including yeast and filamentous fungi. For example, useful mammalian cells include CHO or COS cells. A yeast host cell may be selected from a species of *Saccharomyces* or *Schizosaccharomyces,* e.g. *Saccharomyces cerevisiae.* Useful filamentous fungi may be selected from a species of *Aspergillus,* e.g. *Aspergillus oryzae* or *Aspergillus niger.* Alternatively, a strain of a *Fusarium* species, e.g. *F. oxysporum,* can be used as a host cell. Fungal cells may be transformed by a process involving protoplast formation and transformation of the protoplasts followed by regeneration of the cell wall in a manner known *per se.* A suitable procedure for transformation of *Aspergillus* host cells is described in EP 238 023. A suitable method of transforming *Fusarium* species is described by Malardier et al., 1989.

The present invention thus provides a method of producing a recombinant laccase of the invention, which method comprises cultivating a host cell as described above under conditions conducive to the production of the enzyme and recovering the enzyme from the cells and/or culture medium. The medium used to cultivate the cells may be any conventional medium suitable for growing the host cell in question and obtaining expression of the laccase of the invention. Suitable media are available from commercial suppliers or may be prepared according to published formulae (e.g. in catalogues of the American Type Culture Collection).

In a preferred embodiment, the recombinant production of laccase in culture is achieved in the presence of an excess amount of copper. Although trace metals added to the culture medium typically contain a small amount of copper, experiments conducted in connection with the present invention show that addition of a copper supplement to the medium can increase the yield of active enzyme many-fold. Preferably, the copper is added to the medium in soluble form, preferably in the form of a soluble copper salt, such as copper chloride, copper sulfate, or copper acetate. The final concentration of copper in the medium should be in the range of from 0.2-2mM, and preferably in the range of from 0.05-0.5mM. This method can be used in enhancing the yield of any recombinantly produced fungal laccase, as well as other copper-containing enzymes, in particular oxidoreductases.

The resulting enzyme may be recovered from the medium by conventional procedures including separating the cells from the medium by centrifugation or filtration, precipitating the proteinaceous components of the supernatant or filtrate by means of a salt, e.g. ammonium sulphate, followed by purification by a variety of chromatographic procedures, e.g. ion exchange chromatography, gel filtration chromatography, affinity chromatography, or the like. Preferably, the isolated protein is about 90% pure as determined by SDS-PAGE, purity being most important in food, juice or detergent applications.

In a particularly preferred embodiment, the expression of laccase is achieved in a fungal host cell, such as *Aspergillus.* As described in detail in the following examples, the laccase gene is ligated into a plasmid containing the *Aspergillus oryzae* TAKA α-amylase promoter, and the *Aspergillus nidulans amdS* selectable marker. Alternatively, the *amdS* may be on a separate plasmid and used in co-transformation. The plasmid (or plasmids) is used to transform an *Aspergillus* species host cell, such as *A. oryzae* or *A. niger* in accordance with methods described in Yelton et al. (PNAS USA 81: 1470-1474,1984).

It is of particular note that the yields of *Polyporus* laccase in the present invention, using *Aspergillus* as host cell are unexpectedly and considerably higher than has previously been reported for expression of other laccases in other host cells. It is expected that the use of *Aspergillus* as a host cell in production of laccases from other basidiomycetes, such as *Coriolus* or *Trametes,* will also produce larger quantities of the enzyme than have been previously obtainable. The present invention therefore also encompasses the production of such *Polyporus-like* laccases in *Aspergillus* recombinant host cells.

Those skilled in the art will recognize that the invention is not limited to use of the nucleic acid fragments specifically disclosed herein, for example, in Figures 1-5. It will also be apparent that the invention encompasses those nucleotide sequences that encode the same amino acid sequences as depicted in Figure 1-5, but which differ from the specifically depicted nucleotide sequences by virtue of the degeneracy of the genetic code. Also, reference to Figures 1-5 in the specification and the claims will be understood to encompass both the genomic sequence depicted therein as well as the corresponding cDNA and RNA sequences, and the phrases "DNA construct" and "nucleic acid sequences" as used herein will be understood to encompass all such variations. "DNA construct" shall generally be understood to mean a DNA molecule, either single- or doublestranded, which may be isolated in partial form from a naturally occurring gene or which has been modified to contain segments of DNA which are combined and juxtaposed in a manner which would not otherwise exist in nature.

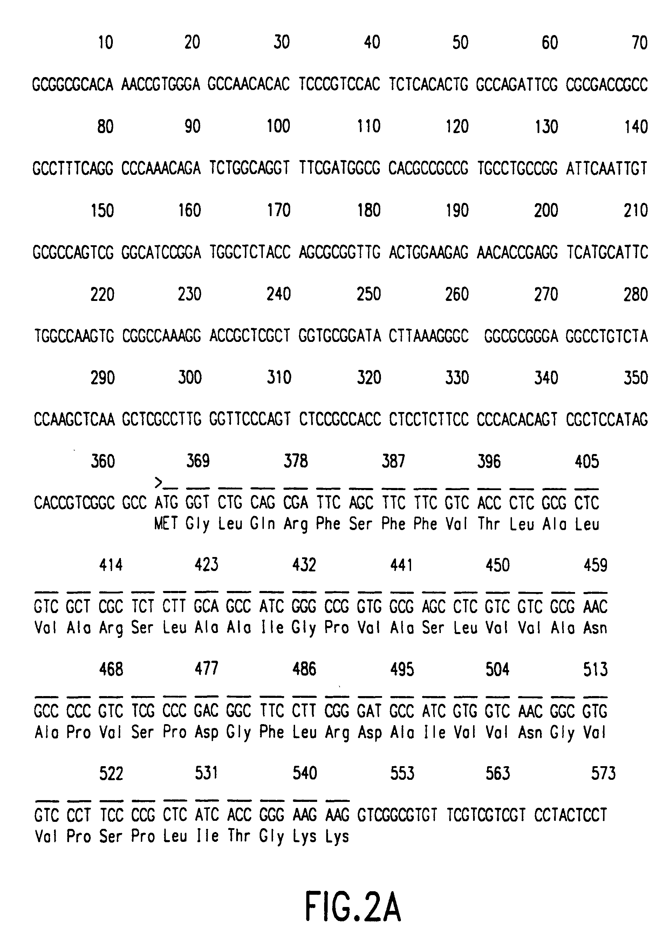

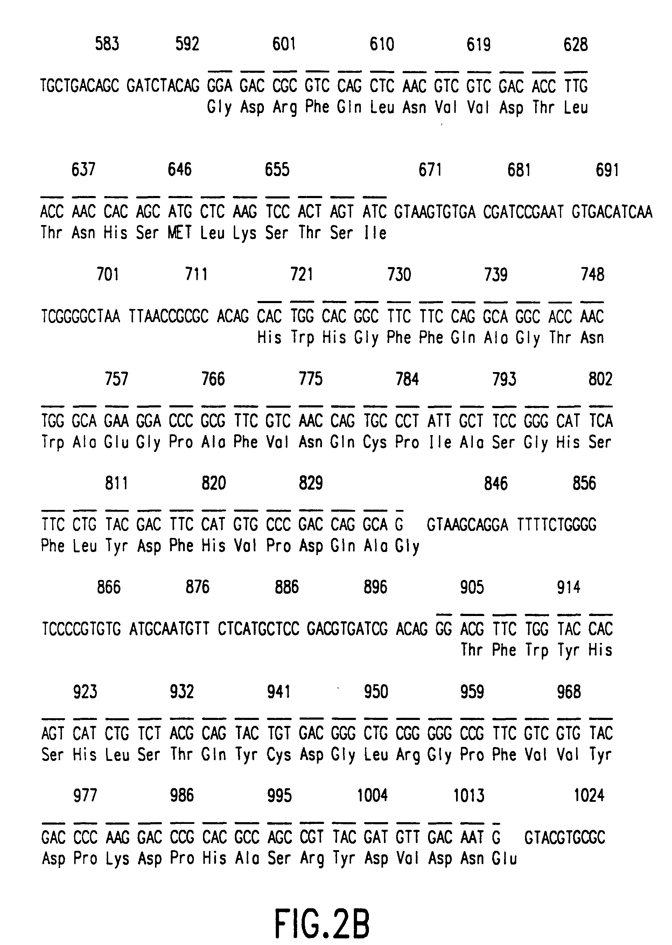

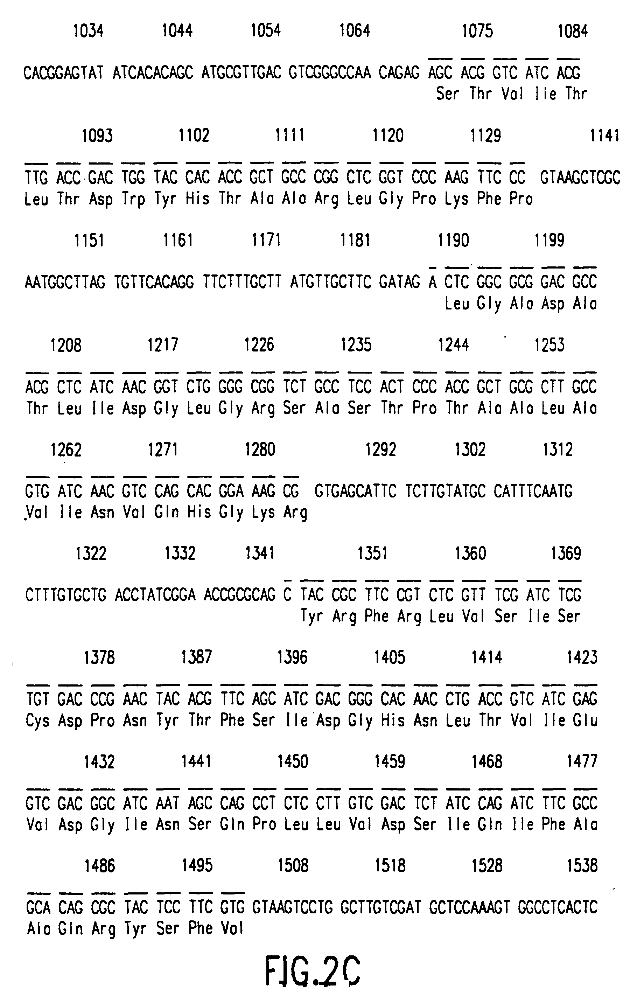

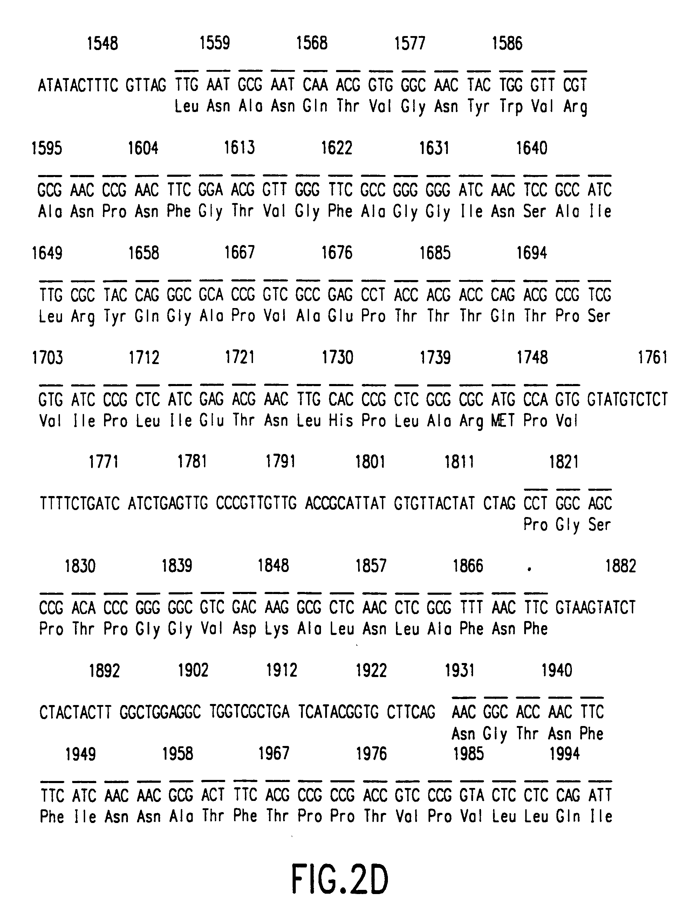

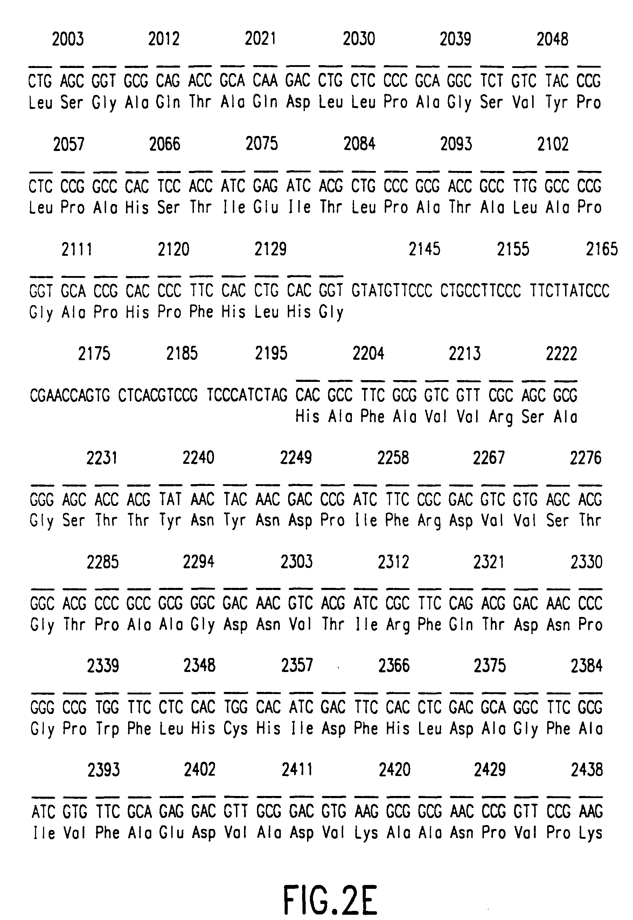

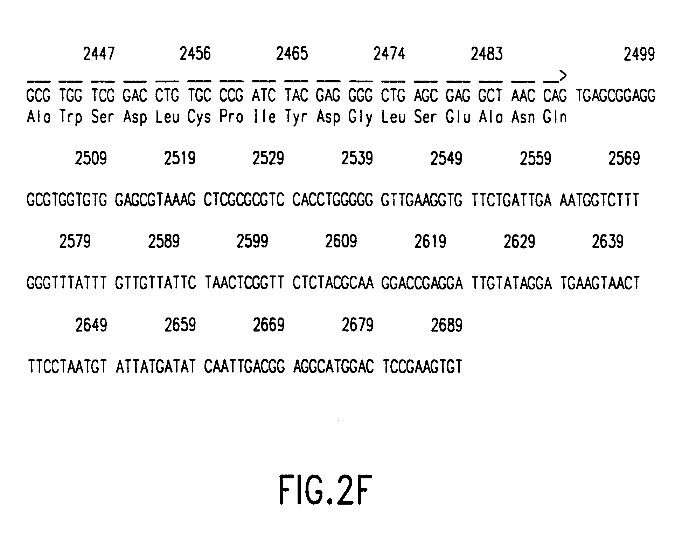

In addition, the invention also encompasses other *Polyporus* laccases, including alternate forms of laccase which may be found in *Polyporus pinsitus* and as well as laccases which may be found in other fungi falling within the definition of *Polyporus* as defined by Fries, or synonyms thereof as stated in Long et al., 1994, ATCC Names of Industrial Fungi, ATCC, Rockville, Maryland. Identification and isolation of laccase genes from sources other than those specifically exemplified herein can be achieved by utilization of the methodology described in the present examples, with publicly available *Polyporus* strains. Alternately, the sequence disclosed herein can be used to design primers and/or probes useful in isolating laccase genes by standard PCR or southern hybridization techniques. Other named *Polyporus* species include, but are not limited to, *P. zonatus, P. alveolaris, P. arcularius, P. australiensis, P. badius, P. biformis, P. brumalis, P. ciliatus, P. colensoi, P. eucalyptorum, P. meridionalis, P. varius, P. palustris, P. rhizophilus, P. rugulosus, P. squamosus, P. tuberaster,* and *P. tumulosus .* Also encompassed are laccases which are synonyms, e.g., anamorphs or perfect states of species or strains of the genus *Polyporus.* Strains of *Polyporus* are readily accessible to the public in a number of culture collections, such as the American Type Culture Collection (ATCC), e.g., ATCC 26721, 9385, 11088, 22084, Deutsche Sammlung von Mikroorganismen und Zellkulturen GmbH (DSM),e.g., DSM 1021, 1023, and 1182; and Centraalbureau Voor Schimmelcultures (CBS), e.g., CBS 678.70, 166.29, 101.15, 276.31, 307.39, 334.49, and 332.49. The invention also encompasses any variant nucleotide sequence, and the protein encoded thereby, which protein retains at least about 85%, and preferably at least about 90-95% homology with any one of the amino acid sequences depicted in Figures 2-5, and which qualitatively retains the laccase activity of the sequence described herein. Useful variants within the categories defined above include, for example, ones in which conservative amino acid substitutions have been made, which substitutions do not significantly affect the activity of the protein. By conservative substitution is meant that amino acids of the same class may be substituted by any other of that class. For example, the nonpolar aliphatic residues Ala, Val, Leu, and Ile may be interchanged, as may be the basic residues Lys and Arg, or the acidic residues Asp and Glu. Similarly, Ser and Thr are conservative substitutions for each other, as are Asn and Gln. It will be apparent to the skilled artisan that such substitutions can be made outside the regions critical to the function of the molecule and still result in an active enzyme. Retention of the desired activity can readily be determined by conducting a standard ABTS oxidation method, such as is described in the present examples.

The protein can be used in number of different industrial processes. These processes include polymerization of lignin, both Kraft and lignosulfates, in solution, in order to produce a lignin with a higher molecular weight. Such methods are described in, for example, Jin et al., Holzforschung 45(6): 467-468, 1991; US Patent No. 4,432,921; EP 0 275 544; PCT/DK93/00217, 1992.

The laccase of the present invention can also be used for in-situ depolymerization of lignin in Kraft pulp, thereby producing a pulp with lower lignin content. This use of laccase is an improvement over the current use of chlorine for depolymerization of lignin, which leads to the production of chlorinated aromatic compounds, which are an environmentally undesirable by-product of paper mills. Such uses are described in, for example, Current opinion in Biotechnology 3: 261-266, 1992; J. Biotechnol. 25: 333-339, 1992; Hiroi et al., Svensk papperstidning 5: 162-166, 1976.

Oxidation of dyes or dye precursors and other chromophoric compounds leads to decolorization of the compounds. Laccase can be used for this purpose, which can be particularly advantageous in a situation in which a dye transfer between fabrics is undesirable, e.g., in the textile industry and in the detergent industry. Methods for dye transfer inhibition and dye oxidation can be found in WO 92/01406, WO 92/18683, EP 0495836 and Calvo, Mededelingen van de Faculteit Landbouw-wetenschappen/Rijiksuniversitet Gent.56: 1565-1567, 1991; Tsujino et al., J. Soc. Chem.42: 273-282, 1991.

The laccase is particularly well-suited for use in hair dyeing. In such an application, the laccase is contacted with a dye precursor, preferably on the hair, whereby a controlled oxidation of the dye precursor is achieved to convert the precursor to a dye, or pigment producing compound, such as a quinoid compound. The dye precursor is preferably an aromatic compound belonging to one of three major chemical families: the diamines, aminophenols(or aminonaphthols) and the phenols. The dye precursors can be used alone or in combination. At least one of the intermediates in the copolymerization must be an ortho- or para-diamine or aminophenol(primary intermediate). Examples of such are found in Section V, below, and are also described in US Patent No. 3,251,742, the contents of which are incorporated herein by reference. In one embodiment, the starting materials include not only the enzyme and a primary intermediate, but also a modifier(coupler) (or combination of modifiers), which modifier is typically a meta-diamine, meta-aminophenol, or a polyphenol. The modifier then reacts with the primary intermediate in the presence of the laccase, converting it to a colored compound. In another embodiment, the laccase can be used with the primary intermediate directly, to oxidize it into a colored compound. In all cases, the dyeing process can be conducted with one or more primary intermediates, either alone or in combination with one or more modifiers. Amounts of components are in accordance with usual commericial amounts for similar components, and proportions of components may be varied accordingly.

The use of this laccase is an improvement over the more traditional use of H₂O₂, in that the latter can damage the hair, and its use usually requires a high pH, which is also damaging to the hair. In contrast, the reaction with laccase can be conducted at alkaline, neutral or even acidic pH, and the oxygen needed for oxidation comes from the air, rather than via harsh chemical oxidation. The result provided by the use of the *Polyporus* laccase is comparable to that achieved with use of H₂O₂, not only in color development, but also in wash stability and light fastness. An additional commercial advantage is that a single container package can be made containing both the laccase and the precursor, in an oxygen free atmosphere, which arrangement is not possible with the use of H₂O₂.

The present laccase can also be used for the polymerization of phenolic or aniline compounds present in liquids. An example of such utility is the treatment of juices, such as apple juice, so that the laccase will accelerate a precipitation of the phenolic compounds present in the juice, thereby producing a more stable juice. Such applications have been described in Stutz, Fruit processing 7/93, 248-252, 1993; Maier et al., Dt. Lebensmittelrindschau 86(5): 137-142, 1990; Dietrich et al., Fluss. Obst 57(2): 67-73, 1990.

Laccases such as the *Polyporus* laccase are also useful in soil detoxification (Nannipieri et al., J. Environ. Qual. 20: 510-517,1991; Dec and Bollag, Arch. Environ. Contam. Toxicol. 19: 543-550, 1990).

The invention is further illustrated by the following non-limiting examples.

### EXAMPLES

### I. ISOLATION OF A POLYPORUS PINISITUS LACCASE ENZYME MATERIALS AND METHODS

### 1. Enzymatic assays

Unless otherwise stated, throughout the examples, laccase activity is determined by syringaldazine and 2,2'-bisazino(3-ethylbenzthiazoline-6-sulfonic acid) (ABTS), as follows. The oxidation of syringaldazine is monitored at 530 nm with 19 µM substrate. In 25 mM sodium acetate, 40 µM cupric sulfate, pH 5.5, at 30°C, the activity is expressed as LACU(µmole/min). For pH profile studies, Britton & Robinson(B&R) buffers are used, and are prepared according to the protocol described in Quelle, Biochemisches Taschenbuch, H.M. Raven, II. Teil, S.93 u. 102, 1964. ABTS oxidation is carried out with 1mM ABTS in 0.1 M NaAc, pH 5.0 at room temperature by monitoring either ΔAbs₄₀₅ in a 96-well plate(Costar) or ΔAbs₄₁₈ in a quartz cuvette. The overlay ABTS oxidase activity assay is carried out by pouring cooled ABTS-agarose(0.03-0.1 g ABTS, 1 g agarose, 50 ml H₂O, heated to dissolve agarose) over a native IEF gel or PAGE and incubating at room temperature.

### 2. Initial isolation of laccase

In order to isolate the laccase, 800 ml of culture fluid is filtered by HFSC on a Supra filter(slow filtering). The clear filtrate is then concentrated and washed on an Amicon cell with a GR81 PP membrane to a volume of 72 ml.

One ml aliquots of laccase are bound to a Q-sepharose HP(Pharmacia, Sweden) column, equilibrated with 0.1 M phosphate, pH7 and the laccase is eluted with a NaCl gradient. In all, 10 x 1 ml samples are purified, pooled, concentrated and washed by ultrafiltration using a membrane with a molecular weight cut-off of 6kD.

### 3. Secondary purification

In a second purification, a fermentation broth is filtered and concentrated by ultrafiltration. The starting material contains 187 LACU/ml. The concentrate is quick-filtered on a Propex 23 filter(P & S Filtration), with 3% Hyflo Cuper-Cel(HSC; Celite Corporation), followed by two ultrafiltration on a Filtron filter with two membranes, each with a molecular weight cutof of 3 kD. The resulting sample (2.5 mS/cm, pH 7.0, at 4°C) is applied to a 130 ml Q-Sepharose column, equilibrated with sodium phosphate, 1.1 mS/cm, pH 7.0. Under these conditions the laccase does not bind to the column, but elutes slowly from the column during the application and wash with the equilibration buffer, resulting in a partial separation from other brownish material.

This partially purified preparation of 1.0mS, pH 7.0 at 20°C is applied to a Q-sepharose column. The column is equilibrated with 20mM sodium phosphate, 2.2 mS, pH 7.0. Under these conditions, the laccase binds to the column and is eluted by a gradient of 0-1 M NaCl over 20 column volumes.

### 3. Sequencing

For internal peptide sequencing, the purified protein is digested with trypsin, followed by peptide purification with HPLC. Purified peptides are sequenced in an Applied Biosystems 473A sequencer.

### B. RESULTS AND DISCUSSION

### 1. Initial characterization

Total yield of the initial purification is about 50 mg(estimated at A280nm). The purified enzyme has a rich blue color, and appears as only two very close bands on SDS-PAGE at about 65 kd. A native PAGE overlaid with substrate shows that both bands have laccase activity with ABTS. The absorption spectrum shows that besides an absorption at A280nm, the purified laccase also shows absorption at about 600nm.

### 2. Sequencing

A N-terminal determination of the protein initially purified shows a single sequence:

Since the N-terminal sequence is not the ideal sequence for constructing a probe, additional experiments with a trypsin digest are conducted, followed by further purification(described above) and sequencing of fragments

### 2. Secondary purification and characterization

In the second purification, the second Q-Sepharose chromatographic step yields the following pools:
Q-Sepharose-2-pool-1 40 ml 112 LACU 47 LACU/A₂₈₀
Q-Sepharose-2-pool-3 80 ml 385 LACU 65 LACU/A₂₈₀
The elution yields >80% of the applied amount. The highly purified preparation Q-Sepharose-2-pool-3 has an A₂₈₀ = 5.9, and A₂₈₀/A₂₆₀ = 1.4. The purity of the laccase in the starting material is extremely high on a protein basis but the starting material is a very dark brown color. In SDS-PAGE, a double band is seen, with a dominating 65 kD band and a smaller 62 kD band. By anionic chromatography, only the dominating band is seen in the first peak(Q-Sepharose-2-pool-1), whereas both bands are seen in the second peak(Q-Sepharose-2-pool-3).

### 3. Sequence

) A number of internal peptide sequences are determined, and compared with the *Coriolus hirsutus(Ch)* laccase sequence. The identified fragments are as follows:

### II. ISOLATION OF A POLYPORUS PINISITUS LACCASE CDNA CLONE

### A. MATERIALS AND METHODS

### 1. RNA preparation

RNA is isolated from 10 grams of *P. pinsitus* mycelium grown under xylidine induction for 6.5 hours, using the guanidium/CsCl cushion method. The RNA is poly-A selected on an oligo-dT column, using standard conditions. 120µg mRNA is obtained and stored as lyophilized pellet in 5µg aliquots at -80°C.

### 2. Single stranded cDNA

Single stranded cDNA is synthesized using the reverse transcriptase "Super Script" (BRL) according to manufacturer's directions.

### 3. Construction of cDNA library

A cDNA library is constructed using the librarian IV cDNA kit (Invitrogen). Fifty cDNA pools, each containing approximately 5000 individual transformants, are obtained.

### 4. PCR

PCR is conducted under the following standard conditions: 100pmol of each primer, 10µl 10X PCR buffer(Perkin-Elmer), 40µl dNTP 0.5 mM, 2µl single stranded cDNA(or approximately 100 ng chromosomal DNA or 100 ng PCR fragment), H₂O to 100 µl, 2.5U Taq polymerase. The cycles are 3x(40°C/two minutes, 72°C/two minutes, 94°C/one minute) followed by 30x(60°C/two minutes, 72°C/two minutes, 94°C/1 minute).

### B. RESULTS AND DISCUSSION

### 1. Cloning of Polyporus pinsitus laccase

PCR is carried out with the primer #3331: and primer #3332: A clear band of about 1500bp is obtained. The DNA is digested with NotI/HindIII, and fractionated on an agarose gel. The upper band(fragment #42) is purified and cloned into the *Aspergillus* vector pHD423. No transformants are obtained. Several attempts are carried out in order to clone the fragment, including redigestion with the restriction enzymes, phosphorylation of the ends, filling in with klenow and blunt-end cloning in SmaI cut puC18, without success. Hybridization with a laccase probe based on the laccase described in Coll et al., *supra,* indicates that the PCR product could be the *P. pinsitus* laccase. In a new attempt to clone the PCR fragment, a new PCR reaction is carried out, using the same conditions as for fragment #42. Again the result is a fragment of about 1500 bp(fragment #43). This time the fragment is cut with HindIII/BamHI, and ligated to HindIII/BamHI-cut pUC18. Three clones, #43-/A, -B, -G are found to contain a fragment of 1500 bp. Partial sequencing reveals that these fragments are laccase related.

### 2. Expression of Polyporus pinsitus laccase

To express the laccase, the fragment #43 is joined to a signal sequence from a 43kD cellulase. The primer pHD433
(TAGCGGATCCCACAATGCGTTCCTCCCCCCTCCTCCCGTCCGCCGTTGTGGCCGCCCTG CCGGTGTTGGCCCTTGCCGGCATTGGGCCCGTCGCGGACC) is used in a standard PCR reaction with a pUC forward primer(New England Biolabs). All three clones are used as templates in order to minimize the risk of working with DNA containing errors.

The PCR generated DNA from the reaction with a primer pHD433 and template 43-A and 43-G is cut with HindIII/BamHI and cloned into the *Aspergillus* expression vector pHD414(described in detail below). Several transformants are obtained.

Clones pHD433/43A-1,2, pHD433/43G-2,-3 are transformed into *A. oryzae.* The transformants from each transformation (between 3-10) are analyzed for laccase production. Activity is only obtained with pHD433/43G-3. The positive transformants (numbers 1, 4, 6) are reisolated on amdS plates, and retested. In an additional transformation round a further ten transformants are obtained with pHD433/43G-3. The clones #20, 23, 26, 28, and 29 are positive. The clones are reisolated and two single isolates are analyzed for laccase expression semiquantitatively by color development in an ABTS assay at pH 4.5. On a scale of +-+++, several clones show moderate to strong expression of laccase.

Further cloning is conducted to identify a full length clone. A xylidine-induced cDNA library consisting of approximately 350,000 transformants is screened using fragment #42-4 as a probe. More than 100 positive clones are detected. The clones are purified, rescreened, and analyzed on Southern blots. Two of the longest clones are further characterized by DNA sequence determination. The longest clones are found to be identical and found to contain a poly-A stretch in the 3'end and to start at the amino acid number 4 in the amino terminus. A partial DNA sequence is determined from different clones.

pHD433/43G-3 is then used in further cloning studies as described in the following Section IV.

### III. PURIFICATION AND CHARACTERIZATION OF ADDITIONAL POLYPORUS PINSITUS LACCASE WILD-TYPE ENZYMES

### A. MATERIALS AND METHODS

### 1. Culture conditions

Shake flasks(250 ml medium/2.8 l baffled flask)are inoculated wtih several agar plugs taken from a week-old PDA plate of *P. pinsitus.* The medium contains, per liter, 10 g glucose, 2.5 g L-asparagine, 0.2 g L-phenylalanine, 2.0 g yeast extract, 2.0 g KH₂PO₄, 0.5 g MgSO₄·7H₂O, 2.0 mlAMG trace metals, 0.002 g CuSO₄·7H₂O, 1.0 g citric acid, made with tape water, pH 5.0 before autoclaving. The cultures are grown at 18-22°C on a rotary shaker with low agitiation (~100 rpm). After 7 days, the pH of each shake flask is adjusted to ~6.0 by the addition of 0.25 ml 5 N NaOH and the cultures are induced by adding 0.5 ml of a 2,5-xylidine stock solution(xylidine diluted 1:10 into ethanol) to each flask. Flasks are incubated for an additional 24 hours, at which time the culture supernatant from each flask is recovered.

### 2. Materials.

Chemicals used as buffers are commercial products of at least reagent grade. Endo/N-glucosidase F is from Boehringer-Mannheim. Chromatography is performed on Pharmacia FPLC. Spectroscopic assays are conducted on either a spectrophotometer(Shimadzu PC160) or a microplate reader(Molecular Devices).

### 3. Purification

Culture broth is filtered first on cheesecloth and centrifuged at 1000 x g to remove gelatinous pinkish xylidine polymer. The supernatant is then filtered on Whatman #2 paper and concentrated from 1500 to 250 ml on S1Y100(Amicon, Spiral concentrator) at 4°C. The concentrated broth is diluted with water until it reaches 0.8 mS(from 2.5 mS) and then concentrated on S1Y100 to 250 ml. The washed broth, thawed from -20°C freezing overnight, is subjected to Whatman #2 paper filtration to remove residual pinkish material, and then pH adjusted by NaOH from pH 6.1 to pH 7.7. This yellowish broth, 275 ml with 0.8 mS, is applied on a Q-Sepharose XK-26 column(~64 ml gel) equilibrated with 10 mM Tris-HCl, pH 7,7, 0.7 mS. The first active laccase fraction runs through during loading and washing by the equilibrating buffer. The elution is carried out by a linear gradient of 0-0.5 M NaCl in the equilibrating buffer over 8.8 bed-volume. The second and third active fractions are eluted around 0.15 and 0.35M NaCl, respectively. No more active fractions are detected when the column is washed sequentially with 2 M NaCl and with 1 mM NaOH. The active fractions are pooled, adjusted to ~10mS, concentrated on Centricon-10(Amicon), and then applied onto Superdex 75(HR10/30, 24 ml, Pharmacia) equilibrated with 10mM Tris-HCl, 0.15 M NaCl, pH 8, 14 mS. During elution with the application buffer, laccase fractions are eluted off using the same elution volume for all three Q-Sepharose fractions, indicating very similar native molecular weight. The purity of the laccase is tested on SDS-PAGE.

### 4. Protein analysis

PAGE and native IEF are carried out on a Mini Protean II and a Model 111 Mini IEF cells(Bio-Rad). Western blots are carried out on a Mini trans-blot cell(Bio-Rad) with an alkaline phosphatase assay kit(Bio-Rad). The primary antibodies are diluted 1000-fold during blotting. N-terminus sequencing is performed on an Applied Biosystems (ABI) 476A protein sequencer using liquid phase TFA delivery for cleavage and on-line HPLC for identification of PTH-amino acids. Standard Fast Cycles and Pre-Mix Buffer System is used according to manufacturer's instructions. Deglycosylation with glycosidase is done as follows: 3µg of protein and 3.6 units of glycosidase in 0.25M NaAc, pH 5, 20 mM EDTA, 0.05% 2-mercaptoethanol is incubated at 37°C for 18 hours with ovalbumin and bovine serum albumin serving as positive and negative control, respectively, and the mobility is detected by SDS-PAGE.

Amino acid analysis for determining extinction coefficients is done using Amino Quant 1090 HPLC system from Hewlett Packard. Microwave facilitated vapor phase hydrolysis of lyophilized samples is done using the MDS-2000 hydrolysis-station(CEM, Matthews, NC). 6N HCl containing 1% phenol as a scavenger is used to create the acid vapors. Hydrolysis time is 20 minutes at 70 psi (~148°C). Hydrolyzed samples are lyophilized and redissolved in 20 µl of 500pmol/µl sarcosine and norvaline as internal standards. 1µl is injected and analyzed according to manufacturer's instructions.

### B. RESULTS AND DISCUSSION

### 1. Purification

The previously characterized *P. pinsitus* laccase has a pI of ~3.5. However, considerable laccase activity is detected in the run-through fraction of Q-Sepharose pre-equilibrated at pH 7.7. Upon a gradient elution, one more active fraction comes off the column before the active fraction initially anticipated. UV-visible spectra and SDS-PAGE show that all three fractions contain mainly laccase. After further purification by gel filtration, different pI's under native non-denaturing conditions are detected for the two new fractions and shown to be consistent with the elution order.

### 2. Characterization

The pure laccase preparations derived from Q-Sepharose eluates behave as a rather well-defined band on SDS-PAGE at ~63 kDa. Deglycosylation detects ~14% w/w carbohydrates based on mobility change on SDS-PAGE. On native-IEF, the laccase preparations have bands of pI 6-6.5, 5-6.5, and 3.5. ABTS-agarose overlay show that all bands are active. Each form in turn shows multiple isoforms under the IEF conditions.

The neutral and acidic forms have a typical UV-visible spectrum with maxima at 605 and 275 nm. The ratio of A₂₇₅/A₆₀₅ is 30-40. The spectrum for the acidic-neutral form has a peak at 276 nm and a shoulder around 600 nm.

The N-terminal sequencing shows that the neutral and neutral-acidic forms have the same first 29 residues(Table 1). The N-terminus of the acidic form matches 100% to that of the previously characterized form. All three forms exhibit comparable cross-reactivity toward antibodies raised against previously characterized form.

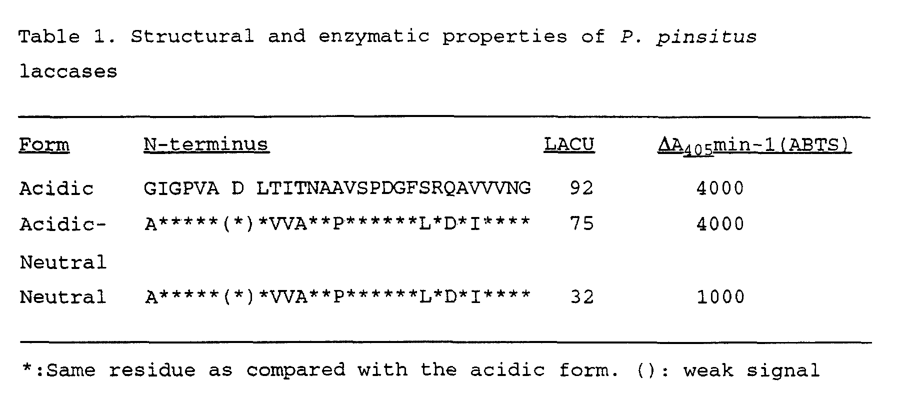

### 3. Laccase Activity

The specific activities(per A₂₇₅) of the three forms are tested by both ABTS and syringaldazine oxidations. The shapes and optima of the pH activity profiles for the three forms are very close: all have optima at ≤pH4 and pH 5-5.5 for ABTS and syringaldazine oxidations, respectively.

### IV. ISOLATION OF MULTIPLE COPIES OF POLYPORUS PINSITUS LACCASE ENZYMES AND GENES

### A. MATERIALS AND METHODS

### 1. Strains

The following strains are employed in the methods described below: *E. coli* K802(*e14*-(*mrca*), *mcrB, hsdR2, galK2, galT22, supE44, metB1*; Clonetech); *E. coli* XL-1 Blue(*recA1, endA1, gyrA96, thi-1, hsdR17, supE44, relA1, lac[F'proAB,* lacIqZDM15, Tn10(tet^{r})];Stratagene) and *Polyporus pinsitus* CBS 678.70.

### 2. Genomic DNA isolation

Cultures of *P.pinsitus* are grown in 500 ml YG (0.5% yeast extract, 2% dextrose) at room temperature for 3 to 4 days. Mycelia are harvested through miracloth, washed twice with TE and frozen quickly in liquid nitrogen. The frozen mycelia are stored at -80°C. To isolate DNA, the mycelia are ground to a fine powder in an electric coffee grinder. The powdered mycelia are resuspended in TE to a final volume of 22 ml. Four ml 20% SDS is added with mixing by inversion followed by incubation at room temperature for 10 minutes. The sample is gently extracted with phenol:chloroform and centrifuged to separate the phases. The aqueous phase is collected and 400µl proteinase A(10 mg/ml stock) is added. The sample is incubated at 37°C for 30 minutes followed by a phenol:chloroform extraction. The aqueous phase is precipitated by the addition of 0.1 volumes of 3 M Na acetate, pH 5.2 and 2.5 volumes 95% ethanol and freezing at 20°C for one hour. After centrifugation to precipitate the DNA, the pellet is resuspended in 6 ml TE, and 200 µl boiled RNase A(10 mg.ml stock) is added. After incubation at 37°C, 100 µl proteinase A(10 mg/ml stock) is added followed by incubation at 37°C for 30 minutes. The sample is phenol:chloroform extracted twice. To the aqueous phase, 0.1 volumes 3 M Na acetate and 2.5 volumes are added, and teh sample is frozen at -20°C for 1 hour. Following centrifugation, the pellet is gently resuspended in 400 µl TE, and 40 µl Na acetate and 1 ml 95% ethanol are added. The DNA is pelleted by centrifugation, and the pellet is washed in 70% ethanol. The final pellet is resuspended in 250 µl TE.

### 3. RNA preparation

RNA is isolated from mycelia which are harvested from *P. pinisitus* cultures which are either induced for laccase expression by the addition of 2,5-xylidine or are uninduced. The mycelia are washed and frozen quickly in liquid N₂. Frozen mycelia are ground to a fine powder in an electric coffee grinder. The powder is immediately suspended in 20 ml extraction buffer (0.2 M Tris-HCl, 0.25 M NaCl, 50 mM EGTA, 0.8% tri-isopropyl naphthalene sulfonic acids, 4.8% p-aminosalicylic acid, pH 8.5). All solutions for RNA extraction are made with diethylpyrocarbonate(DEP)-treated water. The sample is kept on ice and 0.5 volumes TE-saturated phenol:chloroform is added. The sample is mixed well by inversion for 2 minutes, and the phases are separated by centrifugation. The aqueous phase is saved, and the organic phase is extracted with 2 ml extraction buffer and incubated at 68°C for 5 minutes. After centrifugation to separate the phases, the aqueous phases are pooled and extracted several time with phenol:chloroform until there is no longer any protein at the interface. To the aqueous phase 0.1 volume 3 M Na-acetate, pH 5.2 and 2.5 volumes 95% ethanol are added to precipitate the RNA, and the sample is frozen at -20°C for 2 hours. The RNA is pelleted and resuspended in DEP water with RNase inhibitor.

### 4. DNA sequencing

Nucleotide sequences are determined using TAQ polymerase cycle sequencing with fluorescent-labeled nucleotides, and reactions are electrophoresed on an Applied Biosystems automatic DNA sequencer(Model 363A, version 1.2.0).

### 5. Preparation of genomic libraries

Two size-selected genomic libraries of *P. pinsitus* are constructed. A library of 5 to 6 kb BamHI fragments are constructed in pBluescript+. Genomic DNA is digested with BamHI, and the digest is electrophoresed on a preparative agarose(IBI) gel. The region containing the 5 to 6 BamHI fragments is sliced from the gel. The DNA is isolated from teh gel using a Geneclean kit(BIO 101). The DNA is ligated into pBluescript plasmid previously digested with BamHI and dephosphorylated with BAP(GIBCO BRL), *E. coli* XL-1 Blue competent cells (Stratagene) are transformed with the ligation, and 12,000 white colonies are obtained.

A library of 7 to 8 kb BamHI/EcoRI fragments is constructed in pUC118. Ten µg genomic DNA is digested with BamHI and EcoRI and treated with BAP(GIBCO BRL). Competent *E. coli* XL-1 Blue cells are transformed with the ligation, and the library contains ~8000 recombinants.

For the preparation of a total genomic library in lambda EMBL4, 25 µg of *P. pinsitus* genomic DNA is partially digested with Sau3A. After digestion, the DNA is electrophoresed on a preparative low-melt agarose gel, and a band containing the 9 to 23 kb sized DNA is sliced from the gel. The DNA is extracted from the gel using β-agarose(New England Biolabs). The isolated EMBL4 arms (Clonetech) according to the supplier's directions. The ligation is packaged *in vitro* using a Gigapack II kit(Stratagene). The library is titered using *E. coli* K802 cells. The unamplified library is estimated to contain 35,000 independent recombinants. The library is amplified using *E. coli* K802 cells.

### 6. Southern and Northern Blots

DNA samples are electrophoresed on agarose gels in TAE buffer using standard protocols. RNA samples are electrophoresed on agarose gels containing formaldehyde. Both DNA and RNA gels are transferred to Zeta-Probe membrane(BIO-RAD) using either capillary action under alkaline conditions or a vacuum blotter. After transfer, the DNA gels are UV crosslinked. Blots are prehybridized at 65°C in 1.5X SSPE, 1% SDS, 0.5% non-fat dried milk and 200 µg/ml salmon sperm DNA for 1 hour. Radioactive probes are added directly to the prehybridization solutions, and hybridizations are continued overnight at 65°C. Blots are washed with 2XSSC for 5 minutes at 65°C and with 0.2XSSC, 1%SDS,0.1% Na-pyrophosphate at 65°C for 30 minutes twice.

Radioactive labeled probes are prepared using a α-³²P-dCTP and a nick translation kit(GIBCO-BRL).

### 7. Library screening

For screening of the size-selected 5-6 kb BamHI and 7-8 kb BamHI/EcoRI libraries ~500 colonies on LB carb plates and lifted the colonies to Hybond N⁺ filters(Amersham) using standard procedures. The filters are UV crosslinked following neutralization. The filters are prehybridized at 65°C in 1,5X SSPE, 1% SDS, 0.5% non-fat dried milk, 200 µg/ml salmon sperm DNA for 1 hour. Nick-translated probes are added directly to the prehybridization solution, and hybridizations are done overnight at 65°C.

For screening of the genomic bank in EMBL, appropriate dilutions of the amplified library are plated with *E. coli* K802 cells on 100mM NZY top agarose. The plaques are lifted to Hybond N⁺ membranes(Amersham) using standard procedures. The DNA is crosslinked to the membranes using UV crosslinking. The filters are prehybridized and hybridized using the same conditions as those mentioned above.

### RESULTS AND DISCUSSION

### 1.Isolation of multiple copies of laccase gene

*P. pinsitus* genomic DNA is digested with several different restriction enzymes for southern analysis. The blot is probed with the cDNA insert(isolated as a BamHI/SphI fragment from the pYES vector) which is labeled with α-P³²-dCTP. The blot is hybridized and washed as described above. The cDNA hybridizes to several restriction fragments for most of the enzymes suggesting that there are multiple laccase genes in the genome. Because the cDNA hybridizes to a BamHI fragment of ~5.5 kb, a library of 5-6 kb BamHI fragments from *P. pinisitus* is constructed.

### 2. Screening of Genomic Libraries

The results from screening of the libraries are summarized in Table 2. The 5-6 kb BamHI size-selected library is screened with the original cDNA clone labeled with ³²p. Approximately 30,000 colonies are screened with hybridizations done at 65°C. Plasmid DNA is isolated from two positive colonies and digested with BamHI to check for insert size. Both clones contain an ~5.5 kb BamHI insert. The cloned insert(LCC3) is sequenced from either end; the sequence has homology to the cDNA, but is clearly not the cDNA encoded laccase. The partial DNA sequence of LCC3 also indicates that the LCC3 pUC118 clone does not contain the full gene.

From a southern blot of BamHI/EcoRI double digested DNA it is demonstrated that the cDNA hybridizes to an ~7.7 kb fragment. A size-selected library in pUC118 is constructed containing 7-8 BamHI/EcoRI fragments. A total of ~8000 independent colonies are obtained and screened by hybridization with a ³²P labeled insert. Plasmid DNA is isolated from the positive colonies and digested with BamHI and EcoRI. Restriction analysis of the plasmids demonstrate that they fall into two classes. One class (LCC4) contains four clones which are all identical and have an ~7.7 kb BamHI/EcoRI insert which hybridizes to the cDNA. A second class(LCC1) contains two clones which are identical and have inserts of ~7.2 kb which hybridize to the cDNA. Partial DNA sequencing of clones LCC1 and LCC4 demonstrate that clone 21 is the genomic clone of the original cDNA, while LCC4 codes for another laccase. The partial DNA sequence of LCC1 shows that the pUC118 clone does not contain the full gene and that a fragment upstream of the EcoRI site is needed.

At the same time the size selected 7-8 BamHI/EcoRI library is being constructed, a *P. pinisitus* genomic bank in EMBL4 is constructed containing ~35,000 independent recombinant phage. Ten positive plaques are picked and purified. DNA is isolated from the purified phage lysates. Restriction digests of EMBL DNAs demonstrates that there are three classes of clones. The first class(11GEN) is defined by two sibs whose inserts contain a BamHI/EcoRI fragment of the same size as LCC1 which hybridizes to the LCC1 insert. The second class(12GEN) contains one clone which has a different restriction pattern than the 11GEN class and whose insert contains a different restriction pattern than the 11GEN class and whose insert contains an ~5.7 kb BamHI/EcoRI fragment. The third class is defined by a single clone whose insert contains an ~3.2 kb BamHI/EcoRI fragment which hybridizes to the LCC1 insert. DNA sequence analysis demonstrates that clone 11GEN contains the LCC1 BamHI/EcoRI fragment and both 5' and 3" flanking regions. It is also demonstrated that clone 12GEN contains a portion of the LCC1 insert.

The *P. pinisitus* EMBL genomic bank is also screened with the LCC3 BamHI insert in order to clone the full gene. Approximately 30,000 plaques are plated and lifted from hybridization. Five plaques which hybridize to the LCC3(BamHI/EcoRI) insert are identified and purified. DNA is isolated from the purified phage stocks. Southern analysis of *P. pinisitus* genomic DNA demonstrates that the LCC3 BAmHI insert hybridizes to an ~7kb EcoRI fragment. Restriction digests and southerns demonstrate that 4 of the clones contain restriction fragments which hybridize to the EcoRI/BamHI(1.6 kb) fragment and that the clones fall into three classes. Class one is defined by a single clone(LCC5) whose insert contains a 3kb EcoRI fragment which hybridizes to the LCC3 BamHI/EcoRI fragment. Another class is defined by clone(LCC2) whose insert contains an ~11 kb EcoRI fragment which hybridizes to the LCC3 BamHI/EcoRI insert. The third class is defined by two clones which are not identical but contain many of the same restriction fragments; these clones both contain an ~7.5 kb EcoRI fragment which hybridizes to the LCC3 insert. Further analysis of this third class indicates that they are identical to clone LCC4. Partial DNA sequencing of LCC5 and LCC2 indicates that both of these clones code for laccases; however, neither is identical to any of the above mentioned laccase genes(LCC1, LCC3, or LCC4). At this point, five unique laccase genes are cloned; however, the fragments subcloned from LCC5 and LCC2 do not contain the full genes.

From the DNA sequencing of the 3 kb EcoRI fragment from clone LCC5 it is determined that ~200 base pairs of the N-terminus are upstream of the EcoRI site. A 380 bp EcoRI/MluI fragment from LCC5 is used to identify for subcloning a MluI fragment from the LCC5 EMBL clone. An ~4.5 MluI fragment from the LCC5 EMBL clone is subcloned for sequencing and shown to contain the N-terminal sequence.

To clone the N-terminal half of the LCC3 laccase gene, the *P. pinsitus* EMBL genomic bank is probed with an ~750 bp BamHI/StuI restriction fragment from the LCC3 pUC118 clone. Approximately 25,000 plaques are screened and five plaques appear to hybridize with the probe. Upon further purification only three of the clones are still positive. Two of the clones give very strong signals and the restrictions digests of DNA isolated from these phage demonstrate that both contain an ~750 bp BamHI/StuI fragment in their inserts and that the two clones are not identical but overlapped. Based on results of Southern analysis, an ~8.5 kb fragment from these clones are subcloned for sequencing. The EcoRI fragment is shown to contain the entire gene.

To clone the N-terminal half of the LCC2 laccase gene, the *P. pinsitus* genomic bank in EMBL4 is probed with an ~680 bp EcoRI/PvuI of the EMBL LCC2 clone. Thirty thousand plaques are screened by hybridization at 65°C, and 15 plaques appear to hybridize with the probe. All fifteen are purified, and DNA is isolated. The clones can be placed in four classes based on restriction patterns, Seven of the clones are all sibs, and are identical to the original EMBL clone of LCC2. The second class is defined by 3 clones which are sibs. An ~4 kb HindIII fragment is subcloned from this class for sequencing and is shown to contain the N-terminal half of LCC2. A third class is defined by a single clone and is not characterized further.

### 3. DNA sequencing

The complete DNA sequences of the five genomic clones is determined as described in Materials and Methods. Sequencing of clone LCC2 demonstrate that it probably codes for the second form of laccase(neutral pI) isolated from culture broth from an induced *P. pinsitus* culture as described above. The N-terminal protein sequence from the neutral pI laccase and the predicted N-terminus for the protein coded for by LCC2 are compared, and show identity. The predicted pI for the protein coded for by clone LCC2 is 5.95, which is in good agreement with the experimental pI determined for the second form of laccase being between 5.0 and 6.5. Figures 1-5 (SEQ ID NOS. 1-5) show the DNA sequences and predicted translation products for the genomic clones. For LCC1, the N-terminus of the mature protein as determined by protein sequencing and predicted by Von Heijne rules is Gly at position 22. The N-terminus is Gly-Ile-Gly-Pro-Val-Ala-. For LCC2 the N-terminal amino acid of the mature protein as determined by protein sequencing is Ala at position 21. The N-terminus is Ala-Ile-Gly-Pro-Val-Ala-. For LCC3 the predicted N-terminal amino acid of the mature protein is Ser at position 22, with the N terminus being Ser-Ile-Gly-Pro-Val-Thr-Glu-Leu-. For LCC4, the predicted N-terminal amino acid is Ala at position 23 with the N-terminus being Ala-Ile-Gly-Pro-Val-Thr-. For LCC5 the predicted N-terminal amino acid is Ala at position 24 with the N-terminus being Ala-Ile-Gly-Pro-Val-Thr-Asp. A comparison of the structural organization of the genes and the predicted proteins they code for is presented in Table 1. It will be seen that the five genes have different structural organizations and code for proteins of slightly different sizes. Comparisons between the predicted proteins of the genomic clones and other fungal laccase are also done. Table 2 shows a comparison of the predicted laccase to each other and to other fungal laccases. Clone LCC1(the induced laccase first characterized) has the most identity(90%) to the *Coriolus hirsutus* laccase and the PM1 basidiomycete laccase(Coll et al., *supra*). The other four laccases have between 64 and 80% identity to the *C. hirsutus* laccase. The laccase coded for by LCC3 has the least identity to the LCC1 laccase and the other fungal laccases shown in Table 2. LCC2 appears to be the second wild-type laccase isolated as described above; based on the N-terminal sequences of the isolated clones, it also appears that the "neutral" and acidic neutral" wild-type laccases are the same enzyme which is encoded by the LCC2 sequence.

**Table 1**

| Comparison of Structural Organization and Predicted Proteins of the *P. pinsitis* Genomic Clones. | | | | |
|---|---|---|---|---|
| Gene | # Introns | Size of Predicted Precursor Protein | Size of Predicted Mature Protein | Predicted Isolelectric Point |
| 21GEN | 8 | 520 | 499 | 4.49 |
| 23GEN | 10 | 519 | 498 | 5.95 |
| 24GEN | 12 | 516 | 495 | 5.23 |
| 31GEN | 11 | 510 | 488 | 4.06 |
| 41GEN | 11 | 527 | 504 | 4.07 |

**Table 2**

| Amino Acid Identity Between *P. pinsitis* Laccases and Other Fungal Laccases. | | | | | | | | | |
|---|---|---|---|---|---|---|---|---|---|
| | 21GEN | 23GEN | 24GEN | 31GEN | 41GEN | CRIPHA | CRIPHE | PBILAC | PM1 |
| 21GEN | ― | 79% | 64% | 70% | 72% | 90% | 91% | 64% | 80% |
| 23GEN | 79% | ― | 65% | 66% | 69% | 80% | 81% | 62% | 74% |
| 24GEN | 64% | 65% | ― | 61% | 65% | 64% | 65% | 61% | 63% |
| 31GEN | 70% | 66% | 61% | ― | 75% | 69% | 70% | 64% | 69% |
| 41GEN | 72% | 69% | 65% | 75% | ― | 71% | 72% | 64% | 71% |
| CRIPHA | 90% | 80% | 64% | 69% | 71% | ― | 99% | 64% | 80% |
| CRIPHE | 91% | 81% | 65% | 70% | 72% | 99% | ― | 65% | 81% |
| PBILAC | 64% | 62% | 61% | 64% | 64% | 64% | 65% | ― | 65% |
| PM1 | 80% | 74% | 63% | 69% | 71% | 80% | 81% | 65% | ― |
| 21GEN, 23GEN, 24GEN, 31GEN and 41GEN= *P. pinsitis* laccase clones CRIPHA= *Coriolus hirsutis* laccase A CRIPHE= *C. hirsutis* laccase B PBILAC= *Phlebia radiata* laccase PM1= Basidiomycete PM1 laccase (CECT2971) | | | | | | | | | |

### 5. Northern blots

RNA is isolated from mycelia from both a xylidine-induced culture and an uninduced culture. RNA is blotted to membrane after electrophoresis, and the blot is probed with the cDNA insert, or a small fragment containing ~100 bp of the 23GEN promoter and the first 100 bp of the coding region. A transcript of about 1.8 kb hybridizes to both the induced and uninduced RNA samples; however, transcription of this message is clearly induced by the addition of xylidine to the culture.

### III. EXPRESSION OF P. PINSITUS LACCASE IN ASPERGILLUS

### MATERIALS AND METHODS

### 1. Strains

*A. oryzae* A1560, *A. oryzae* HowB104(fungamyl delete, *pyrg),A. oryzae* HowB101*pyrg*, A. *niger* Bo-1, *A. niger* Bo-80, *A. niger* ATCC1040, *A. niger* NRRL337, *A. niger* NRRL326, *A. niger* NRRL326, *A. niger* NRRL2295, *A. niger* ATCC11358, *A. niger* NRRL322, *A. niger* AT10864, *A. japonicus* A1438, *A. phoenicis, A. foetidus* N953.

### 2. Media

For the shake flask cultivation of the *A. niger, A. foetidus,* and *A. phoenicis* MY50 (per liter:50 g maltodextrin, 2 g MgSO₄·H₂O, 10 gKH₂PO₄, 2 g K₂SO₄, 2 g citric acid, 10 g yeast extract, 0.5 ml trace metals, 2 g urea, pH 6.0) media is used. For the shake flask cultivation of the *A. oryzae* A1560 and HowB101 strains MY51(per liter: 30 g maltodextrin, 2 mg MgSO₄, 10 g KH₂PO₄, 2 g K₂SO₄, 2 g citric acid, 10 g yeast extract, 0.5 ml trace metals, 1 g urea, 2 g(NH₄)₂SO₄, pH 6.0) is used. For the shake flask analysis of the *A.oryzae* HowB104 strains, MY51 maltose(same as MY51 but with 50g of maltose instead of maltodextrin) media is used. For the shake flask analysis of the *A. japonicus* strains M400 media(per liter: 50 g maltodextrin, 2 g MgSO₄, 2 g KH₂PO₄, 4 g citric acid, 8 g yeast extract, 0.5 ml trace metals, 2 g urea, pH 6.0.

Cultures grown overnight for protoplast formation and subsequent transformation are grown in YEG(0.5% yeast extract, 2% dextrose). For strains that are *pyrg,* uridine is supplemented to 10 mM final concentration.

### 3. Screening for laccase production

Primary transformants are screened first on a minimal medium plates containing 1% glucose as the carbon source and 1mM ABTS to test for production of laccase. Transformants that give green zones on the plates are picked and spore purified before shake flask analysis is done.

Shake flask samples are centrifuged to clear the broth. Dilute or undiluted broth samples are assayed with ABTS

### RESULTS AND DISCUSSION

### 1. Expression in shake flasks

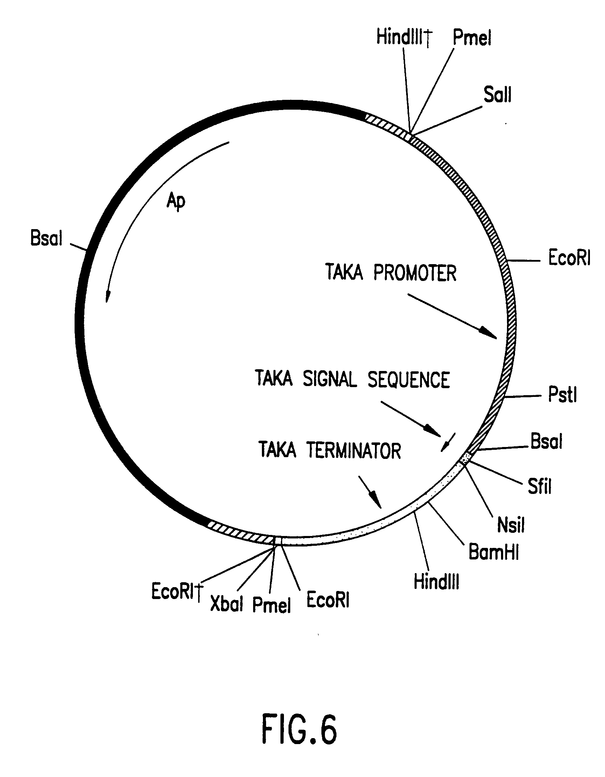

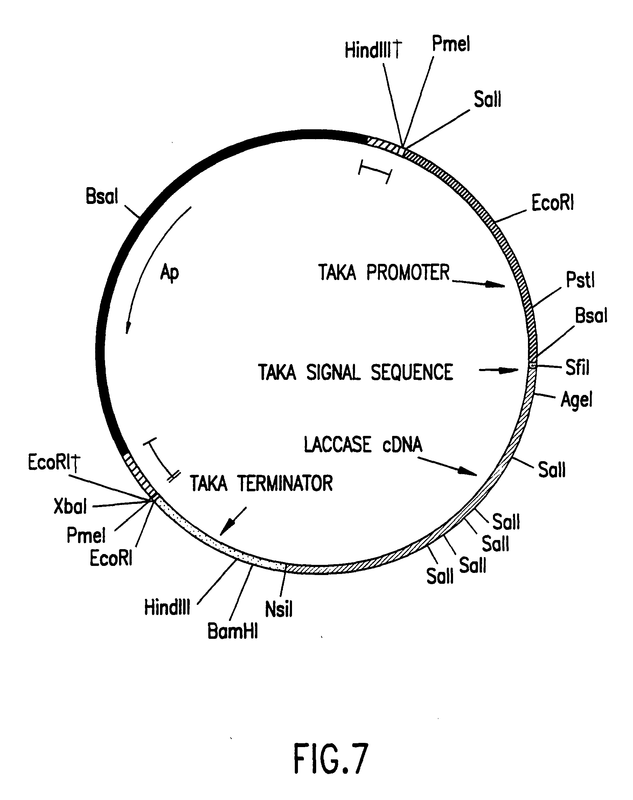

The first expression vector constructed is pDSY1, which contains the TAKA promoter, TAKA signal sequence, *P. pinisitus* laccase cDNA beginning at the mature N-terminus and the AMG terminator. The TAKA signal sequence: laccase insert is constructed in 2 steps. First by site directed mutagenesis, an AgeI site beginning at bp 107 of the laccase mature coding region is created by a single base change and a NsiI site is created ~120 bp downstream of the laccase stop codon(ACG GGT->ACC GGT and TTC GCT->ATG CAT, respectively). A small PCR fragment beginning with an SfiI site and ending with the AgeI site at 107 bp in laccase is PCR amplified. This fragment contains a piece of the TAKA signal sequence and the first ~107 bp of the mature laccase cDNA. Further DNA sequencing of this fragment shows it has a single base change that leads to a substitution of Asn for Thr at position 9 in mature laccase. This substitution creates a potential N-linked glycosylation site. The PCR fragment and AgeI/NsiI fragments are cloned into pMWR1(Figure 6) which has been digested with SfiI/NsiI. The vector pMWR1 contains the TAKA promoter, a portion of the TAKA signal sequence which ends with an SfiI site, and the TAKA terminator with a NsiI site inserted directly 5' to the terminator. The resulting expression vector (Figure 7) is used to cotransform several hosts. Methods for co-transformation of *Aspergillus* strains are as described in Christensen et al., *supra.*

In the second laccase expression vector, the base change in DSY1 which leads to the substitution of Asn for Thr at amino acid 9 is reverted back to wild type by a PCR reaction. The second expression vector pDSY2 is identical to pDSY1 except for this single base change. Three different *A. oryzae* strains and several *A. niger* strains are cotransformed with pDSY2 and either pTOC90(WO 91/17243) which carries the *A. nidulans amdS* gene or pSO2 which carries the *A. oryzae pyrG* gene.

Expression of laccase is observed in all hosts tested, with both DSY1 and DSY2. Yields range from 0.1-12.0 Δabs/min/ml, with highest yields being observed with *A. niger* strains.

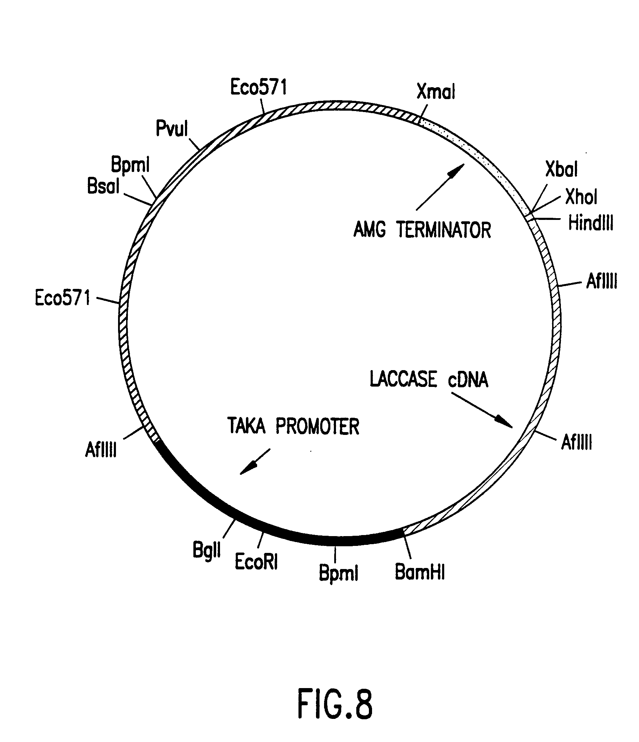

A construct pDSY10 is made which contains the TAKA promoter, laccase full-length cDNA including its own signal sequence and the AMG terminator. A 200 bp BamHI/AgeI fragment which has a BamHI site immediately 5' to the ATG of the initiation codon and an AgeI site at the same position as in pDSY1 is PCR amplified using *lac*1 as template. A MluI/HindIII fragment is PCR amplified using pDSY2 as template and begins with the MluI site present in the cDNA and ends with a HindII site directly 3' to the stop codon of laccase. The above two fragments and the AgeI/MluI fragment from pDSY2 are ligated into pHD414 to yield pDSY10 (Figure 8).

The vector pHD414 used in expression of laccase is a derivative of the plasmid p775(EP 238 023). In contrast to this plasmid, pHD414 has a string of unique restriction sites between the TAKA promoter and the AMG terminator. The plasmid is constructed by removal of an approximately 200 bp long fragment (containing undesirable RE sites) at the 3' end of the terminator, and subsequent removal of an approximately 250 bp long fragment at the 5' end of the promoter, also containing undesirable sites. The 200 bp region is removed by cleavage with NarI (positioned in the pUC vector) and XbaI (just 3' to the terminator), subsequent filling in the generated ends with Klenow DNA polymerase + dNTP, purification of the vector fragment on a gel and religation of the vector fragment. This plasmid is called pHD413. pHD413 is cut with StuI (positioned in the 5' end of the promoter) and PvuII (in the pUC vector), fractionated on gel and religated, resulting in pHD414. Cotransformation of *A. oryzae* HowB104 and *A. niger* Bo-1 are done using pToC90 for selection. Yields in shake flask are comparable to those seen with pDSY2.

### 2. Expression in fermentors

A 1 ml aliquot of a spore suspension of *Aspergillus niger* transformant Bo-1-pDSY10-4(approximately 10⁹ spores/ml) is added aseptically to a 500 ml shake flask containing 100 ml of sterile shake flask medium (glucose, 75g/l; soya meal, 20 g/l; MgSO₄·7H₂O, 2g/l; KH₂PO₄, 10g/l; K₂SO₄, 2g/l; CaCl₂·2H₂O 0.5 g/l; Citric acid, 2g/l; yeast extract, 10g/l; trace metals[ZnSO₄·7H₂O, 14.3 g/l; CuSO₄·5H₂O, 2.5 g/l; NiCl₂·6H₂O, 0.5 g/l; FeSO₄·7H₂O, 13.8 g/l, MnSO₄·H₂O, 8.5 g/l; citric acid, 3.0 g/l], 0.5 ml/l; urea, 2g/l, made with tap water and adjusted to pH 6.0 before autoclaving), and incubated at 37°C on a rotary shaker at 200 rpm for 18 hours. 50 ml of this culture is aseptically transferred to a 3 liter fermentor containing 1.8 liters of the fermentor media (maltodextrin MD01 300 g/l; MgSO₄·7H₂O, 2g/l; KH₂PO₄, 2g/l; citric acid 2g/l; K₂SO₄, 2.7 g/l;CaCl₂·2H₂O, 2g/l; trace metals, 0.5 ml/l; pluronic antifoam, 1ml/l; made with tap water and pH adjusted to 6.0 before autoclaving). The fermentor temperature is maintained at 34°C by the circulation of cooling water through the fermentor jacket. Sterile air is sparged through the fermentor at a rate of 1.8 liter/min (1v/v/m). The agitation rate is maintained at 800 rpm for the first 24 hours after inoculation and at 1300 rpm for the remainder of the fermentation. The pH of the fermentation is kept at 4.0 by the automatic addition of 5N NaOH or H₃PO₄. Sterile feed (urea, 50 g/l; pluronic antifoam, 1.5 ml/l, made up with distilled water and autoclaved) is added to the fermentor by use of a peristaltic pump. The feed rate profile during the fermentation is as follows: 40 g of feed is added initially before inoculation; after inoculation, feed is at a constant rate of 2.5 g/l h.

Copper is made as a 400X stock in water or a suitable buffer, filter sterilized and added aseptically to the tank to a final level of 0.5 mM. Samples for enzyme activity determination are withdrawn and filtered through Miracloth to remove mycelia. These samples are assayed for laccase activity by a LACU assay. Laccase activity is found to increase continuously during the course of the fermentation, with a value of approximately 55 LACU/ml is achieved after 190 hours. This corresponds to approximately 350mg/l of recombinant laccase expressed.

### IV. PURIFICATION OF RECOMBINANT LACCASE

### MATERIALS AND METHODS

### 1. Materials.

Chemicals used as buffers and substrates are commercial products of at least reagent grade. Endo/N-glycosidase G is from Boehringer-Mannheim. Chromatography is performed on either a Pharmacia's FPLC or a conventional open column low pressure system. Spectroscopic assays are conducted on a Shimadzu PC160 spectrophotometer.

### 2. Purification

### (a) DSY2

2.8 liters cheese-cloth filtered broth(pH 7, 19mS) obtained from an A. oryzae pDSY2 transformant as described above is filtered on 0.45 µ Corning filter and concentrated on Spiral Concentrator(Amicon) with S1Y30 membrane to 200ml. The concentrate pH is adjusted to 7.5, diluted with 4.8 l water to achieve 1.2 mS, and concentrated on S1Y30 to 200ml. 50ml of this broth solution is applied onto a Q-Sepharose column(XK16, 34ml gel), pre-equilibrated with 10mM Tris, pH 7.5, 0.7 mS(Buffer A). The blue laccase band that migrates slowly during loading is eluted by a linear gradient of Buffer B(Buffer A plus 0.5 M NaCl). 24 ml of pooled laccase fractions are concentrated on Centricon-100(Amicon) to 4.5 ml and applied onto a Superdex 200 column(HiLoad 16/60, 120 ml gel). During the development with Buffer C(Buffer A plus 0.15 M NaCl, 14.4 mS), the blue laccase fractions elute followed by brownish contaminant fractions. Only the first half of the elution band(detected by Abs₆₀₀) show a high laccase to contaminant ratio and are pooled. The pooled fractions are dialyzed in 10mM Bis-Tris, pH 6.8, 0.6mS(Buffer D), applied onto a Mono-Q column(Mono-Q 5/5, 1ml) equilibrated with Buffer D, and eluted with Buffer E(Bufer D plus 0.5 M NaCl) using a linear gradient. The laccase fractions, which ome out round 27% Buffer E, are pure as judged by SDS-PAGE. At each step, the laccase fractions are routinely checked by ABTS oxidation, SDS-PAGE, and Western Blot.

### (b) DSY10

2.8 liters cheese-cloth filtered broth(pH 7.3, 24mS) obtained from HowB104-pDSY10 is filtered on Whatman #2 paper and concentrated on Spiral Concentrator(Amicon) with S1Y100 membrane to 210ml. The concentrate pH is diluted with water to achieve 1.2 mS, and concentrated on S1Y100 to 328 ml. This broth solution is applied onto a Q-Sepharose column(XK26, 120 ml gel), pre-equilibrated with 10mM Tris, pH 7.5, 0.7 mS(Buffer A). The blue laccase band that migrates slowly during loading is eluted by a linear gradient of Buffer B(Buffer A plus 2 M NaCl). 120 ml of pooled laccase fractions are diluted with water to achieve 1.1mS and then concentrated on SIY100 to 294 ml and applied onto a Mono-Q column(HiLoad 16/10, 40 ml gel) pre-equilibrated with Buffer A. The laccase slowly passes through the column during loading and washing with Buffer A. The pooled fractions which have a pH reading of 5.6, are loaded on a Mono-Q column(HiLoad 16/10, 40 ml gel), pre-equilibrated with Buffer C(10mM MES, pH 5.5, 0.1 mS). The laccase fractions elute by a very shallow gradient of Buffer D(Buffer C + 1M NaCl). Enzymatic assays are conducted as described above.

### 3. Protein analysis

Total amino acid analysis, N-terminal sequencing, deglycosylation, SDS-PAGE, IEF, and Western blots are performed as decribed above.

### B. RESULTS AND DISCUSSION

### 1. Purification and Characterization

Overall a 256-fold purification and a yield of 37% are achieved for DSY10, and a 246-fold purification and a yield of 14% are achieved for DSY2 In terms of electorphoretic pattern, spectral properties and activity, purified DSY2 and DSY10 are indistinguishable. Purified recombinant laccases behave as a dimer on gel filtration, and exhibit subunit molecular weight which is somewhat larger than that of the wild type laccase, indicating a post-translational processing in *A. oryzae* that results in the extra glycosylation on the recombinants. Deglycosylation has confirmed the difference in mass arising from extra sugars(Table 3).

**Table 3.**

| Molecular and spectral properties of recombinant and wild-type laccase | | | | | |
|---|---|---|---|---|---|
| | MW,kDa | | Carbohydrate | pI | λₘₐₓ,nm(ε,l/g*cm) |
| | Native | subunit | w/w% | | |
| WT | ~130 | ~63 | ~7 | 3.5 | 275(1.8)615(0.12) |
| Rec. | ~130 | ~67 | ~13 | 3.5 | 275(1.7)615(0.11) |

The spectra of the purified laccases have maxima of 615 nm and 275, with the ratio of absorbance at 275 nm to that at 615 nm being 16, indicating one Type I Cu per subunit. The ratio of absorbance at 330nm to that at 615nm is 1.0, close to the 0.75 value of *Rhus vernicefera* laccase, suggesting the presence of one Type II and two Type III copper ions per subunit. The extinction coefficient determined by amino acid analysis is 1.71/(g*cm),

### 3. Activity

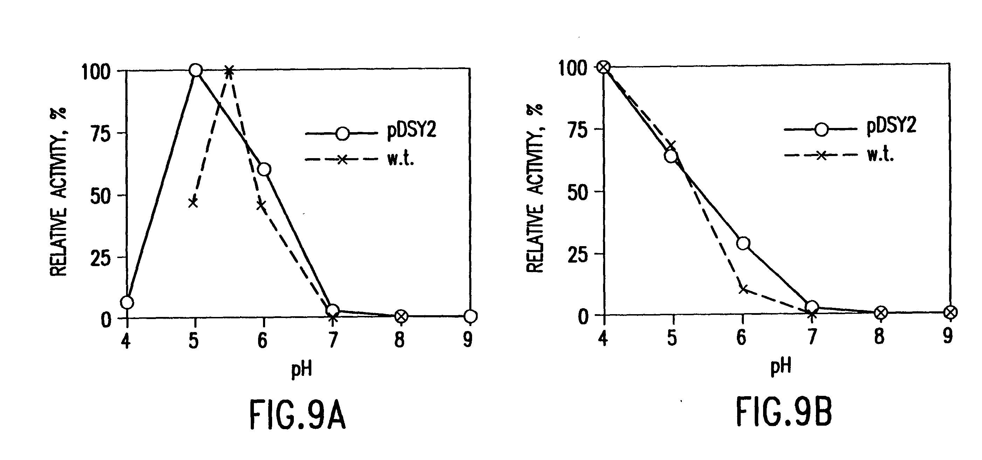

The laccase activity is measured by syringaldazine and ABTS oxidations. Expressed per A₂₇₅, the laccase has a value of 83 for LACU. Expressed per mg, it has a LACU of 141. The pH profile of the laccase is provided in Figure 9.

### V. USE OF POLYPORUS LACCASE TO DYE HAIR

The dyeing effect of *Polyporus pinsitus* laccase is tested and compared to the dyeing effect of 3% H₂O₂ on various dye precursors (listed below) and further on 0.1% p-phenylenediamine compared with a number of modifiers.

### Materials:

### Dye precursors:

0.1 % p-phenylene-diamine in 0.1 M K-phosphate buffer, pH 7.0.(pPD)
0.1 % p-toluylene-diamine in 0.1 M K-phosphate buffer, pH 7.0.
0.1 % chloro-p-phenylenediamine in 0.1 M K-phosphate buffer, pH 7.0.
0.1 % p-aminophenol in 0.1 M K-phosphate buffer, pH 7.0.
0.1 % o-aminophenol in 0.1 M K-phosphate buffer, pH 7.0.
0.1 % 3,4-diaminotoluene in 0.1 M K-phosphate, buffer pH 7.0.

### Modifiers:

0.1 % m-phenylene-diamine in 0.1 M K-phosphate buffer, pH 7.0.
0.1 % 2,4-diaminoanisole in 0,1 M K-phosphate buffer, pH 7.0.
0.1 % α-naphthol in 0.1 M K-phosphate buffer, pH 7.0.
0.1 % hydroquinone in 0.1 M K-phosphate buffer, pH 7.0.
0.1 % pyrocatechol in 0.1 M K-phosphate buffer, pH 7.0.
0.1% resorcinol in 0.1 M K-phosphate buffer, pH 7.0.
0.1 % 4-chlororesorcinol in 0.1 M K-phosphate buffer, pH 7.0.
When a modifier is used, the dye precursor p-phenylene-diamine is combined with one of the above indicated modifiers so that the final concentration in the dyeing solution is 0.1 % with respect to precursor and 0.1 % with respect to modifier. The enzyme used is a recombinant laccase from *Polyporus pinisitus*, at a concentration of 10 LACU/ml.

Other solutions used in the process are 3% H₂O₂ (in the final dye solution), and a commercial shampoo.

The quantitative color of the hair tresses is determined on a Datacolor Textflash 2000 (CIE-Lab) by the use of CIE-Lab parameters L* ("0"=black and "100"=white) combined with a* ("-"=green and "+"=red). DL* and Da* are the delta values of L* and a*, respectively, of a sample when compared to L* and a* of untreated hair. The Light fastness is determined under a day light bulb (D65) at 1000 LUX.

Hair tresses of blond European hair (1 gram) are used.
4 ml dye precursor solution (including modifier)is mixed with 1 ml laccase or 1 ml H₂O₂ on a Whirley mixer, applied to the hair tresses and kept at 30°C for 60 minutes. The hair tresses are then rinsed with running water, combed, and air dried.

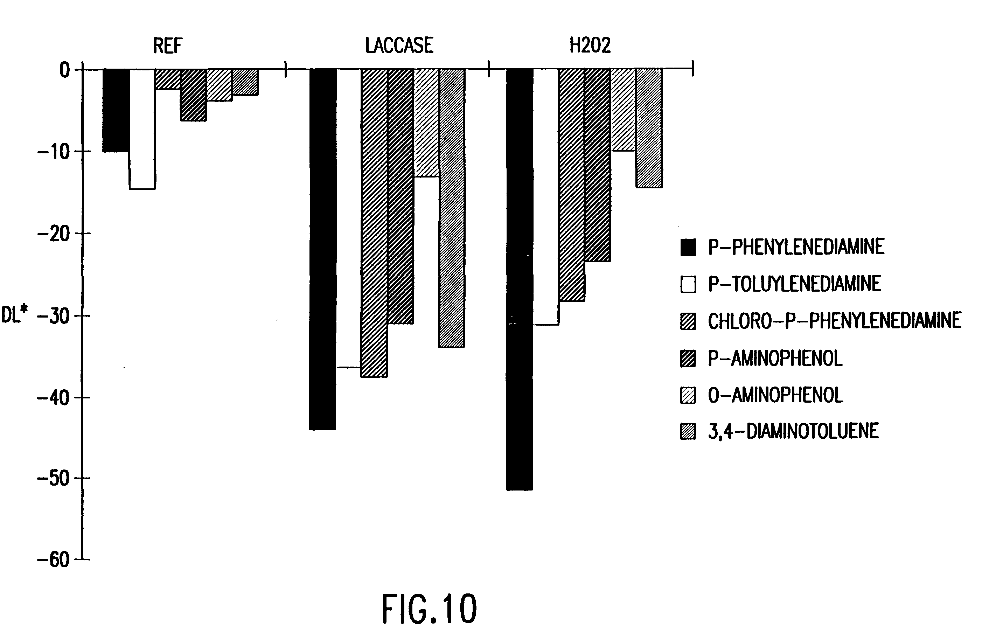

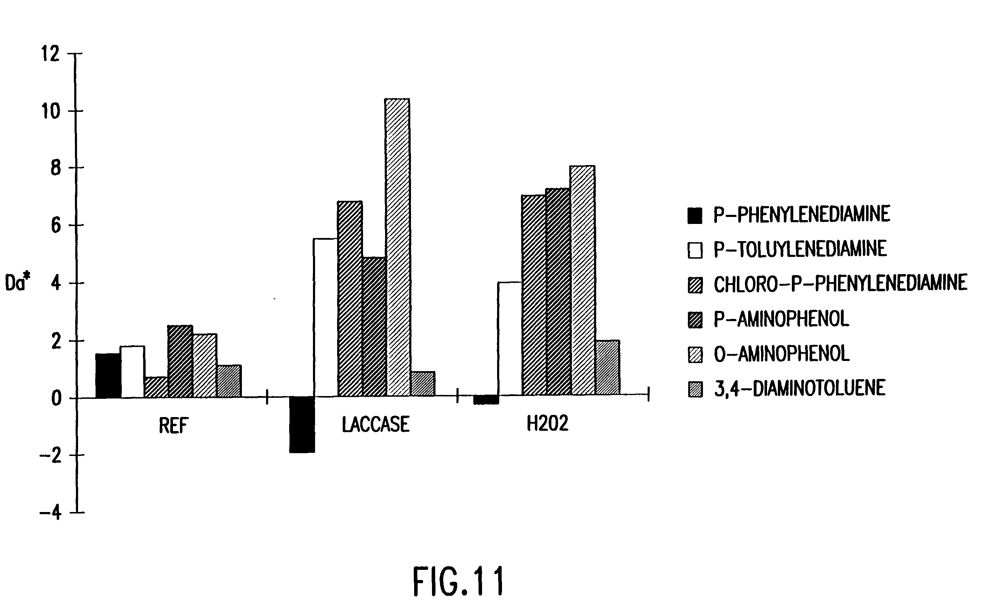

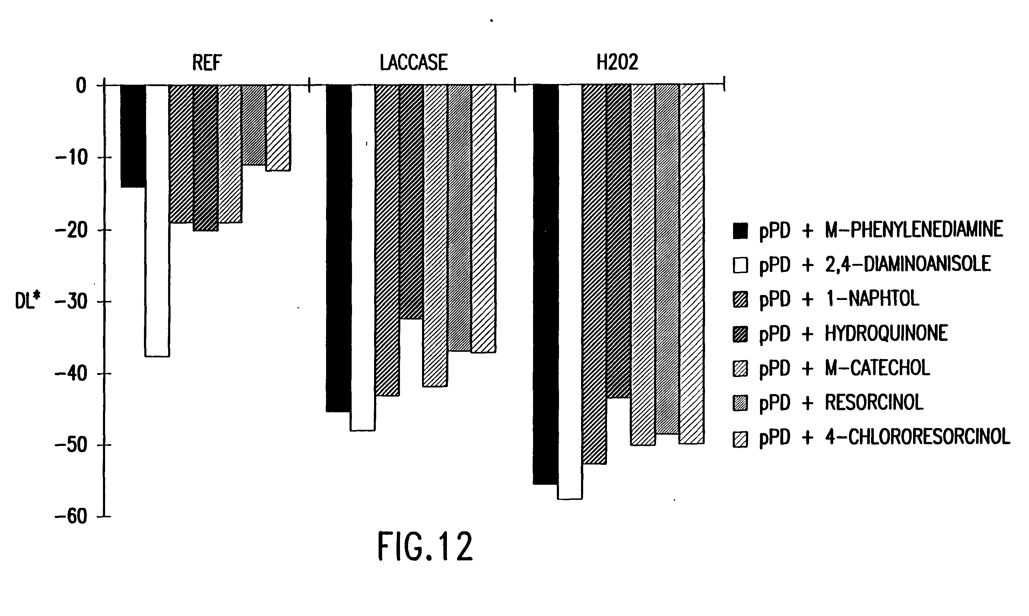

The results of the dyeing effect test are displayed below in Table 4-6 and further in the graphs in Figures 10 to 12.

**Table 4**

| Sample no. | Sample ID | L* | a* | DL* | Da* |
|---|---|---|---|---|---|
| | Untreated blond hair | 72.25 | 2.42 | | |
| 1 | p-phenylenediamine (Reference) | 62.85 | 4.03 | -9.41 | 1,61 |
| 2 | p-phenylenediamine + Laccase | 28.70 | 0.33 | -43.56 | -2,10 |
| 3 | p-phenylenediamine + 3% H₂O₂ | 21.88 | 2.04 | -50.37 | -0,39 |
| 4 | p-Toluylenediamine (Reference) | 58.14 | 4.34 | -14.11 | 1.92 |
| 5 | p-Toluylenediamine + Laccase | 36.70 | 8.09 | -35.56 | 5.67 |
| 6 | p-Toluylenediamine + 3% H₂O₂ | 42.30 | 6.24 | -29.95 | 3.81 |
| 7 | chloro-p-phenylenediamine (Reference) | 69.82 | 3.23 | -2.43 | 0.81 |
| 8 | chloro-p-phenylenediamine + Laccase | 35.58 | 9.36 | -36.68 | 6.93 |
| 9 | chloro-p-phenylenediamine + 3% H₂O₂ | 45.42 | 9.59 | -26.84 | 7.17 |
| 10 | p-aminophenol (Reference) | 66.62 | 5.03 | -5.63 | 2.61 |
| 11 | p-aminophenol + Laccase | 42,42 | 7.38 | -29,84 | 4.95 |
| 12 | p-aminophenol + 3% H₂O₂ | 50.54 | 9.42 | -21.72 | 7.26 |
| 13 | o-aminophenol (Reference) | 69.39 | 4.82 | -2.89 | 2.39 |
| 14 | o-aminophenol + Laccase | 60.20 | 12.92 | -12.05 | 10.50 |
| 15 | o-aminophenol + 3% H₂O₂ | 63.49 | 10.38 | -8.77 | 7.96 |
| 16 | 3,4-diaminotoluene (Reference) | 69.62 | 3.57 | -2.63 | 1.15 |
| 17 | 3,4-diaminotoluene + Laccase | 39.51 | 3.15 | -32.74 | 0.73 |
| 18 | 3,4-diaminotoluene + 3% H₂O₂ | 59.32 | 4.16 | -12.94 | 1.74 |

| | | | | | |
|---|---|---|---|---|---|
| L*: 0=black, 100=white | | | | | |
| a*: -=green, +=red | | | | | |

**Table 5**

| Sample no. | Sample ID | L* | a* | DL* | Da* |
|---|---|---|---|---|---|
| | Untreated blond hair | 72.25 | 2.42 | | |
| 19 | p-phenylenediamine+ m-phenylenediamin (Reference) | 58.82 | 0.43 | -13,44 | -1,99 |
| 20 | p-phenylenediamine + m-phenylenediamin + Laccase | 27.20 | 0.83 | -45,05 | -1,59 |
| 21 | p-phenylenediamine + m-phenylenediamine + 3% H2O2 | 16.96 | 0.13 | -55,29 | -2,59 |
| 22 | p-phenylenediamine + 2,4 - diaminoanisole (Reference) | 35.37 | -0.02 | -36,89 | -2,45 |
| 23 | p-phenylenediamine + 2,4 - diaminoanisole + Laccase | 24.56 | 2.99 | -47,70 | 0,57 |
| 24 | p-phenylenediamine + 2,4-diaminoanisole + 3% H2O2 | 15.06 | 2.21 | -57,20 | -0,21 |
| 25 | p-phenylenediamine + α-naphthol (Reference) | 54.33 | 2.54 | -17,93 | 0,12 |
| 26 | p-phenylenediamine + α-naphthol + Laccase | 29.53 | 4.03 | -42,72 | 1,60 |
| 27 | p-phenylenediamine + α-naphthol + 3% H2O2 | 19.58 | 3.90 | -52,68 | 1,47 |
| 28 | p-phenylenediamine + hydroquinone (Reference) | 53.25 | 4.08 | -19,01 | 1,65 |
| 29 | p-phenylenediamine + hydroquinone + Laccase | 40.48 | 5.00 | -31,77 | 2,58 |
| 30 | p-phenylenediamine + hydroquinone + 3% H2O2 | 29.06 | 4.96 | -43,20 | 2,53 |

| | | | | | |
|---|---|---|---|---|---|
| L*: 0=black, 100=white | | | | | |
| a*: -=green, +=red | | | | | |

**Table 6**

| Sample no. | Sample ID | L* | a* | DL* | Da* |
|---|---|---|---|---|---|
| | Untreated blond hair | 72.25 | 2.42 | | |
| 31 | p-phenylenediamine + pyrocatechol (Reference) | 53.78 | 1.68 | -18.47 | -0.74 |
| 32 | p-phenylenediamine + pyrocatechol + Laccase | 30.77 | 2.64 | -41.49 | 0.22 |
| 33 | p-phenylenediamine + pyrocatechol + 3% H₂O₂ | 22.15 | 3.30 | -50.11 | 0.88 |
| 34 | p-phenylenediamine + resorcinol (Reference) | 62.12 | 4.23 | -10.14 | 1.81 |
| 35 | p-phenylenediamine + resorcinol + Laccase | 36.14 | 2.91 | -36.11 | 0.49 |
| 36 | p-phenylenediamine + resorcinol + 3% H₂O₂ | 23.94 | 3.16 | -48.31 | 0.74 |
| 40 | p-phenylenediamine + 4-chlororesorcinol (Reference) | 61.18 | 4.70 | -11.07 | 2.28 |
| 41 | p-phenylenediamine + 4-chlororesorcinol + Laccase | 36.00 | 2.76 | -36.26 | 0.34 |
| 42 | p-phenylenediamine + 4-chlororesorcinol + 3% H₂O₂ | 22.63 | 2.60 | -49.63 | 0.18 |

| | | | | | |
|---|---|---|---|---|---|
| L*: 0=black, 100=white | | | | | |
| a*: -=green, +=red | | | | | |

The oxidative hair dyeing is carried out as described above, except that 50 LACU/ml *Polyporus pinsitus* laccase was used.

To test wash stability, the dyed hair tresses are wetted and washed for 15 seconds with 50 µl of commercial shampoo, and rinsed with water for 1 minute. The hair tresses are washed up to 20 times.

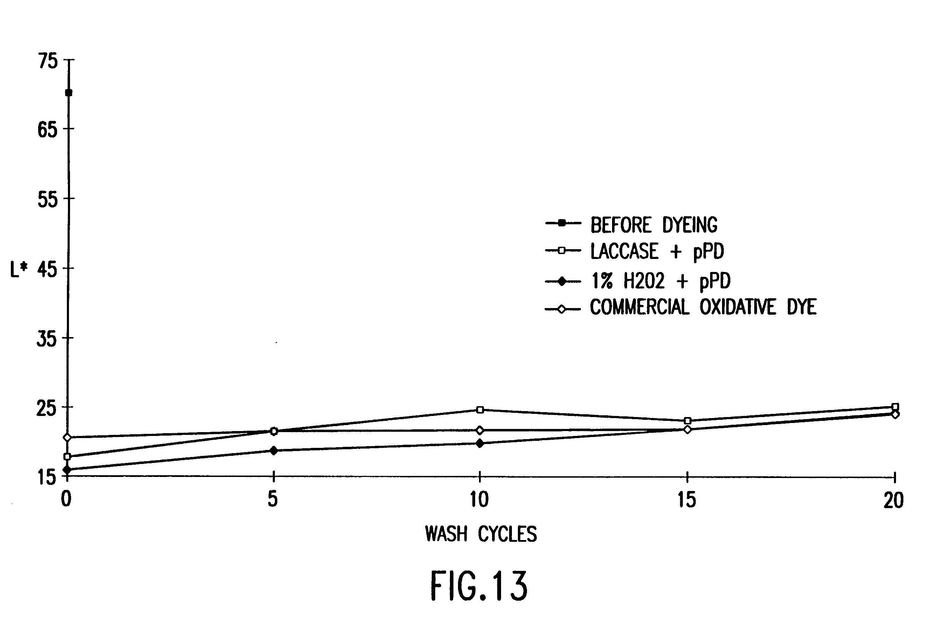

The results of the hair wash test are displayed in figure 13. It can be seen in figure 13 that the wash stability of hair washed up to 20 times is excellent, when using *Polyporus pinsitus* laccase for oxidative dyeing.

To test light fastness, tresses of blond european hair are used for testing the light fastness of hair dyed using *Polyporus pinsitus* laccase in comparison to hair dyed using H₂O₂. p-phenylene-diamine is the dye precursor. The dyeing of the hair is carried out as described above. One hair tress is kept dark, while an other is kept at day light (i.e. under a day light bulb (D65)), at approximately 1000 LUX) for up to 275 hours. The CIE-Lab-values are determined immediately after the dyeing of the hair, and further during exposure to day light.

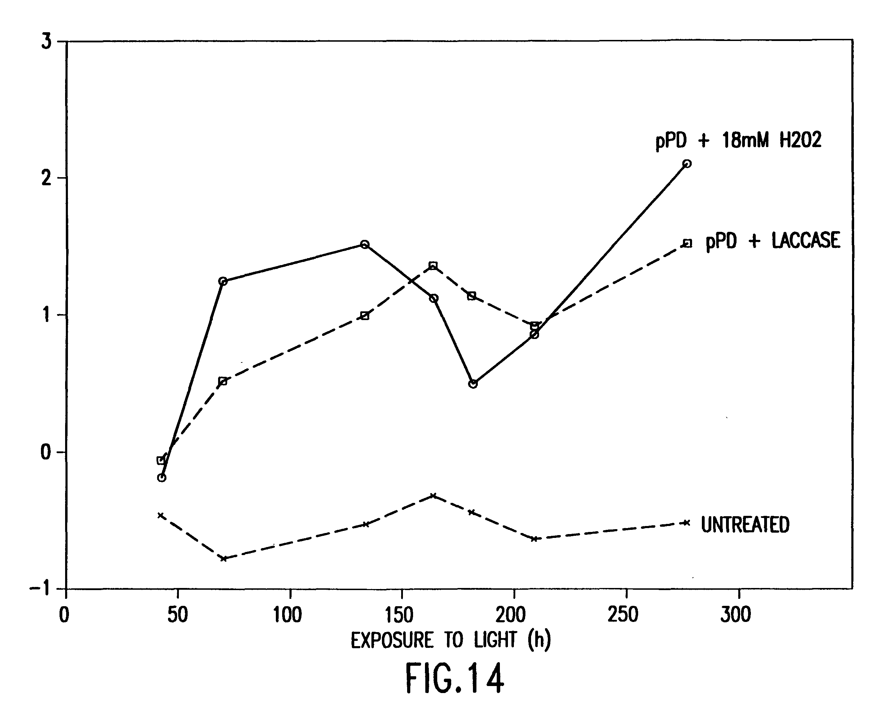

The results of the test are displayed in figure 14. Figure 14 shows that the hair dyed with p-phenylene-diamine using *Polyporus pinsitus* laccase has the same light fastness as hair dyed using H₂O₂.

### Deposit of Biological Materials

The following biological materials have been deposited under the terms of the Budapest Treaty with the Agricultural Research Service Patent Culture Collection, Northern Regional Research Center, 1815 University Street, Peoria, Illinois, 61604 on May 25, 1994 and given the following accession numbers.

| Deposit | Accession Number |
|---|---|
| *E. coli* DH5α containing pDSY22(41GEN; an ~3.0 kb EcoRI insert) | NRRL B-21263 |
| *E. coli* DH5α containing pDSY23(41GEN; an ~4.5 kb MluI insert; insert contains a small portion of the EcoRI fragment of pDSY22 and sequences 5' to the EcoRI fragment) | NRRL B-21268 |
| *E. coli* XL-1 Blue containing pDSY21(31GEN; an ~7.7 kb EcoRI/BamHI insert) | NRRL B-21264 |
| *E. coli* XL-1 Blue containing pDSY18(21GEN; an ~8.0 kb BamHI insert) | NRRL B-21265 |
| *E. coli* DH5α containing pDSY19(23GEN; an ~4 kb HindIII insert) | NRRL B-21266 |
| *E. coli* DH5α containing pDSY20(24GEN; an ~8.5 kb EcoRI insert) | NRRL B-21267 |

## Claims

1. A DNA construct encoding an enzyme comprising a nucleotide sequence encoding the amino acid sequence depicted in SEQ ID NO. 2.

2. The construct of Claim 1, wherein the nucleic acid sequence is depicted in SEQ ID NO. 1.

3. A DNA construct which comprises a nucleic acid sequence encoding the amino acid sequence depicted in SEQ ID NO. 4.

4. The construct of Claim 3 wherein the nucleic acid sequence is depicted in SEQ ID NO. 3.

5. A DNA construct which comprises a nucleic acid sequence encoding the amino acid sequence depicted in SEQ ID NO. 6.

6. The construct of Claim 5, wherein the nucleic acid sequence is depicted in SEQ ID NO. 5.

7. A DNA construct which comprises a nucleic acid sequence encoding the amino acid sequence depicted in SEQ ID NO. 8.

8. The construct of Claim 7, wherein the nucleic acid sequence is depicted in SEQ ID NO. 7.

9. A DNA construct which comprises a nucleic acid sequence encoding the amino acid sequence depicted in SEQ ID NO. 10.

10. The construct of Claim 9, wherein the nucleic acid sequence is depicted in SEQ ID NO. 9.

11. A DNA construct which comprises the nucleic acid sequence selected from those contained in NRRL B-21263, 21264, 21265, 21266, 21267 and 21268.

12. A substantially pure *Polyporus* laccase enzyme which comprises the amino acid sequence selected from the group consisting of the sequences depicted in SEQ ID NOS. 4, 6, 8, and 10 or a sequence with at least about 85% homology thereto.

13. A recombinant vector comprising the DNA construct of claim 1, 2, 3, 4, 5, 6, 7, 8, 9, 10 or 11.

14. The vector of Claim 13 in which the construct is operably linked to a promoter sequence.

15. The vector of Claim 14 in which the promoter is a fungal or yeast promoter.

16. The vector of Claim 14 in which the promoter is the TAKA amylase promoter of *Aspergillus oryzae.*

17. The vector of Claim 14 in which the promoter is the glucoamylase (glaA) promoter of *Aspergillus niger* or *Aspergillus awamori.*

18. The vector of claim 13 which also comprises a selectable marker.

19. The vector of Claim 18 in which the selectable marker is selected from the group consisting of *amdS, pyrG, argB, niaD, sC, trpC* and *hygB.*

20. The vector of Claim 18 in which the selectable marker is the amdS marker of *Aspergillus nidulans* or *Aspergillus oryzae,* or the pyrG marker of *Aspergillus nidulans*, *Aspergillus niger, Aspergillus awamori*, or *Aspergillus oryzae.*

21. The vector of Claim 13 which comprises both the TAKA amylase promoter of *Aspergillus oryzae* and the amdS or pyrG marker of *Aspergillus nidulans* or *Aspergillus oryzae.*

22. A recombinant host cell comprising the heterologous DNA construct of claims 1, 2, 3, 4, 5, 6, 7, 8, 9, 10 or 11 encoding a *Polyporus* laccase.

23. The cell of Claim 22 which is a fungal cell.

24. The cell of Claim 23 which is an *Aspergillus* cell.

25. The cell of Claim 22 in which the construct is integrated into the host cell genome.

26. The cell of Claim 22 in which the construct is contained on a vector.

27. The cell of Claim 22 which comprises a construct containing a sequence encoding an amino acid sequence selected from the group consisting of those depicted in SEQ ID NOS. 4, 6, 8, and 10.

28. A method for obtaining a laccase enzyme which comprises culturing a recombinant host cell comprising a DNA construct containing the nucleic acid sequence of claim 1 encoding a *Polyporus* laccase enzyme, under conditions conducive to expression of the enzyme, and recovering the enzyme from the culture.

29. A method for obtaining a laccase enzyme which comprises culturing a recombinant *Aspergillus* host cell comprising a DNA construct containing a nucleic acid sequence according to any of claims 1-11 encoding a *Polyporus* laccase enzyme, under conditions conducive to expression of the enzyme, and recovering the enzyme from the culture.

30. A method for polymerizing a lignin or lignosulfate substrate in solution which comprises contacting the substrate with a *Polyporus* laccase according to claim 12.

31. A method for in situ depolymerization in Kraft pulp which comprises contacting the pulp with a *Polyporus* laccase according to claim 12 or SEQ ID No. 2.

32. A method for oxidizing dyes or dye precursors which comprises contacting the dye or dye precursor with a *Polyporus* laccase according to claim 12.

33. A method for dyeing hair which comprises contacting a *Polyporus* laccase according to claim 12 or SEQ ID No. 2, in the presence or absence of at least one modifier, with at least one dye precursor, for a time and under conditions sufficient to permit oxidation of the dye precursor to a dye.

34. The method of claim 33 in which the dye precursor is selected from the group consisting of a diamine, aminophenol, and a phenol.

35. The method of claim 33, wherein the modifier, when used, is a meta-diamine, a meta-aminophenol or a polyphenol.

36. The method of claim 35 in which the dye precursor is a primary intermediate selected from the group consisting of an ortho- or para-diamine or aminophenol.

37. The method of claim 35 in which more than one dye precursor is used.

38. The method of claim 35 in which more than one modifier is used.

39. The method of claim 35 in which both a primary intermediate and a modifier are used.

40. A dye composition comprising a *Polyporus* laccase according to claim 12 or SEQ ID No. 2 combined with at least one dye precursor.

41. A dye composition comprising a *Polyporus* laccase according to claim 12 or SEQ ID No. 2 combined with at least one primary intermediate and at least one modifier.

42. A container containing a dye composition comprising a *Polyporus* laccase according to claim 12 or SEQ ID No. 2 and at least one dye precursor in an oxygen-free atmosphere.

43. The container of claim 42 which contains at least one primary intermediate dye precursor combined with at least one modifier.

44. A method of polymerizing or oxidizing a phenolic or aniline compound which comprises contacting the phenolic or aniline compound with a *Polyporus* laccase according to claim 12.

## Patentansprüche

1. DNA-Konstrukt kodierend ein Enzym umfassend eine Nukleotidsequenz kodierend die Aminosäuresequenz, die in SEQ ID NO.2 dargestellt ist.

2. Konstrukt nach Anspruch 1, wobei die Nukleinsäuresequenz in SEQ ID NO.1 dargestellt ist.

3. DNA-Konstrukt, das eine Nukleinsäuresequenz kodierend die Aminosäuresequenz, die in SEQ ID NO.4 dargestellt ist, umfasst.

4. Konstrukt nach Anspruch 3, wobei die Nukleinsäuresequenz in SEQ ID NO.3 dargestellt ist.

5. DNA-Konstrukt, das eine Nukleinsäuresequenz kodierend die Aminosäuresequenz, die in SEQ ID NO.6 dargestellt ist, umfasst.

6. Konstrukt nach Anspruch 5, wobei die Nukleinsäuresequenz in SEQ ID NO.5 dargestellt ist.

7. DNA-Konstrukt, das eine Nukleinsäuresequenz kodierend die Aminosäuresequenz, die in SEQ ID NO.8 dargestellt ist, umfasst.

8. Konstrukt nach Anspruch 7, wobei die Nukleinsäuresequenz in SEQ ID NO.7 dargestellt ist.

9. DNA-Konstrukt, das eine Nukleinsäuresequenz kodierend die Aminosäuresequenz, die in SEQ ID NO.10 dargestellt ist, umfasst.

10. Konstrukt nach Anspruch 9, wobei die Nukleinsäuresequenz in SEQ ID NO.9 dargestellt ist.

11. DNA-Konstrukt, das die Nukleinsäuresequenz umfasst, die ausgewählt ist von jenen, die in NRRL B-21263, 21264, 21265, 21266, 21267 und 21268 enthalten sind.

12. Ein im wesentlichen reines *Polyporus* Laccaseenzym, das die Aminosäuresequenz ausgewählt aus der Gruppe bestehend aus den Sequenzen, die dargestellt sind in SEQ ID NOs. 4, 6, 8 und 10 oder eine Sequenz mit wenigstens ca. 85 % Homologie dazu, umfasst.

13. Rekombinanter Vektor enthaltend ein DNA-Konstrukt nach Anspruch 1, 2, 3, 4, 5, 6, 7, 8, 9, 10 oder 11.

14. Vektor nach Anspruch 13, wobei das Konstrukt operativ mit einer Promotorsequenz verbunden ist.

15. Vektor nach Anspruch 14, wobei der Promotor ein Pilz- oder Hefepromotor ist.

16. Vektor nach Anspruch 14, wobei der Promotor der TAKA Amylasepromotor von *Aspergillus oryzae* ist.

17. Vektor nach Anspruch 14, wobei der Promotor der Glucoamylase (glaA) Promotor von *Aspergillus niger* oder *Aspergillus awamori* ist.

18. Vektor nach Anspruch 13, wobei außerdem ein selektiver Marker umfasst ist.

19. Vektor nach Anspruch 18, wobei der selektive Marker ausgewählt ist aus der Gruppe bestehend aus *amdS, pyrG, argB, niaD, sC, trpC* und *hygB.*

20. Vektor nach Anspruch 18, wobei der selektive Marker der amdS Marker von *Aspergillus nidulans* oder *Aspergillus oryzae*, oder der pyrG Marker von *Aspergillus nidulans, Aspergillus niger, Aspergillus awamori* oder *Aspergillus oryzae* ist.

21. Vektor nach Anspruch 13, wobei sowohl der TAKA Amylasepromotor von *Aspergillus oryzae* und der amdS oder pyrG Marker von *Aspergillus nidulans* oder *Aspergillus oryzae* umfasst ist.

22. Rekombinante Wirtszelle umfassend das heterologe DNA-Konstrukt nach den Ansprüchen 1, 2, 3, 4, 5, 6, 7, 8, 9, 10 oder 11 kodierend eine *Polyporus* Laccase.

23. Zelle nach Anspruch 22, die eine Pilzzelle ist.

24. Zelle nach Anspruch 23, die eine *Aspergillus* Zelle ist.

25. Zelle nach Anspruch 22, wobei das Konstrukt in das Wirtszellgenom integriert ist.

26. Zelle nach Anspruch 22, wobei das Konstrukt auf einem Vektor enthalten ist.

27. Zelle nach Anspruch 22, die ein Konstrukt umfasst, enthaltend eine Sequenz kodierend eine Aminosäuresequenz ausgewählt aus der Gruppe bestehend aus jenen, die in den SEQ ID NOs. 4, 6, 8 und 10 dargestellt sind.

28. Verfahren zum Erhalten eines Laccaseenzyms, das umfasst Kultivieren einer rekombinanten Wirtszelle umfassend ein DNA-Konstrukt enthaltend die Nukleinsäuresequenz aus Anspruch 1 kodierend ein *Polyporus* Laccaseenzym, unter Bedingungen, die geeignet sind für die Expression des Enzyms, und Erhalten des Enzyms aus der Kultur.

29. Verfahren zum Erhalten eines Laccaseenzyms, das umfasst Kultivieren einer rekombinanten *Aspergillus.* Wirtszelle umfassend ein DNA-Konstrukt enthaltend eine Nukleinsäuresequenz gemäß einem der Ansprüche 1 bis 11 kodierend ein *Polyporus* Laccaseenzym, unter Bedingungen, die geeignet sind für die Expression des Enzyms, und Erhalten des Enzyms aus der Kultur.

30. Verfahren zur Polymerisation eines Lignin- oder Lignosulfat-Substrats in Lösung, das umfasst Inkontaktbringen des Substrats mit einer *Polyporus* Laccase gemäß Anspruch 12.

31. Verfahren für in situ Depolymerisation in Kraft-Brei, das umfasst Inkontaktbringen des Breis mit einer *Polyporus* Laccase gemäß Anspruch 12 oder SEQ ID NO. 2.

32. Verfahren zur Oxidation von Farbstoffen oder Farbstoffvorläufern, das umfasst Inkontaktbringen des Farbstoffs oder des Farbstoffsvorläufers mit einer *Polyporus* Laccase gemäß Anspruch 12.

33. Verfahren zum Färben von Haar, das umfasst Inkontaktbringen einer *Polyporus* Laccase gemäß Anspruch 12 oder SEQ ID NO.2 in Anwesenheit oder Abwesenheit von wenigstens einem Modifizierer mit wenigstens einem Farbstoffvorläufer für einen Zeitraum und unter Bedingungen, die geeignet sind die Oxidation des Farbstoffvorläufers zu einem Farbstoff zu erlauben.

34. Verfahren nach Anspruch 33, wobei der Farbstoffvorläufer ausgewählt ist aus der Gruppe bestehend aus einem Diamin, Aminophenol und einem Phenol.

35. Verfahren nach Anspruch 33, wobei der Modifizierer, wenn er verwendet wird, ein meta-Diamin, ein meta-Aminophenol oder ein Polyphenol ist.

36. Verfahren nach Anspruch 35, wobei der Farbstoffvorläufer ein primäres Intermediat ist, das ausgewählt ist aus der Gruppe bestehend aus einem orthooder para-Diamin oder Aminophenol.

37. Verfahren nach Anspruch 35, wobei mehr als ein Farbstoffvorläufer verwendet wird.

38. Verfahren nach Anspruch 35, wobei mehr als ein Modifizierer verwendet wird.

39. Verfahren nach Anspruch 35, wobei sowohl ein primäres Intermediat als auch ein Modifizierer verwendet werden.

40. Eine Farbstoffzusammensetzung umfassend eine *Polyporus* Laccase gemäß Anspruch 12 oder SEQ ID NO.2 kombiniert mit wenigstens einem Farbstoffvorläufer.

41. Farbstoffzusammensetzung umfassend eine *Polyporus* Laccase gemäß Anspruch 12 oder SEQ ID NO.2 kombiniert mit wenigstens einem primären Intermediat und wenigstens einem Modifizierer.

42. Behälter enthaltend eine Farbstoffzusammensetzung umfassend eine *Polyporus* Laccase gemäß Anspruch 12 oder SEQ ID NO.2 und wenigstens einen Farbstoffvorläufer in einer sauerstofffreien Atmosphäre.

43. Behälter nach Anspruch 42, der wenigstens ein primäres Intermediat eines Farbstoffvorläufers kombiniert mit wenigstens einem Modifizierer enthält.

44. Verfahren zur Polymerisation oder Oxidation einer phenolischen oder Anilin-Verbindung, das umfasst Inverbindungbringen der phenolischen oder Anilin-Verbindung mit einer *Polyporus* Laccase gemäß Anspruch 12.

## Revendications

1. Produit de synthèse d'ADN codant pour une enzyme comportant une séquence nucléotidique codant pour la séquence d'acides aminés représentée dans la SEQ ID N° 2.

2. Produit de synthèse selon la revendication 1, dans lequel la séquence d'acide nucléique est représentée dans la SEQ ID N° 1.

3. Produit de synthèse d'ADN qui comporte une séquence d'acide nucléique codant pour la séquence d'acides aminés représentée dans la SEQ ID N° 4.

4. Produit de synthèse selon la revendication 3, dans lequel la séquence d'acide nucléique est représentée dans la SEQ ID N° 3.

5. Produit de synthèse d'ADN qui comporte une séquence d'acide nucléique codant pour la séquence d'acides aminés représentée dans la SEQ ID N° 6.

6. Produit de synthèse selon la revendication 5, dans lequel la séquence d'acide nucléique est représentée dans la SEQ ID N° 5.

7. Produit de synthèse d'ADN qui comporte une séquence d'acide nucléique codant pour la séquence d'acides aminés représentée dans la SEQ ID N° 8.

8. Produit de synthèse selon la revendication 7, dans lequel la séquence d'acide nucléique est représentée dans la SEQ ID N° 7.

9. Produit de synthèse d'ADN qui comporte une séquence d'acide nucléique codant pour la séquence d'acides aminés représentée dans la SEQ ID N° 10.

10. Produit de synthèse selon la revendication 9, dans lequel la séquence d'acide nucléique est représentée dans la SEQ ID N° 9.

11. Produit de synthèse d'ADN qui comporte la séquence d'acide nucléique sélectionnée parmi celles contenues dans NRRL B-21263, 21264, 21265, 21266, 21267 et 21268.

12. Enzyme laccase de *Polyporus* essentiellement pure qui comporte la séquence d'acides aminés sélectionnée parmi le groupe constitué des séquences représentées dans les SEQ ID N° 4, 6, 8 et 10 ou une séquence ayant une homologie d'au moins environ 85 % avec celle-ci.

13. Vecteur recombinant comportant le produit de synthèse d'ADN selon la revendication 1, 2, 3, 4, 5, 6, 7, 8, 9, 10 ou 11.

14. Vecteur selon la revendication 13, dans lequel le produit de synthèse est lié de manière opérationnelle à une séquence promoteur.

15. Vecteur selon la revendication 14, dans lequel le promoteur est un promoteur fongique ou de levure.

16. Vecteur selon la revendication 14, dans lequel le promoteur est le promoteur de l'amylase TAKA de *Aspergillus oryzae.*

17. Vecteur selon la revendication 14, dans lequel le promoteur est le promoteur de la glucoamylase (glaA) de *Aspergillus niger* ou *Aspergillus awamori.*

18. Vecteur selon la revendication 13, qui comporte également un marqueur pouvant être sélectionné.

19. Vecteur selon la revendication 18, dans lequel le marqueur pouvant être sélectionné est sélectionné parmi le groupe constitué de *amdS, pyrG, argB, niaD, sC, trpC* et *hygB.*

20. Vecteur selon la revendication 18, dans lequel le marqueur pouvant être sélectionné est le marqueur amdS de *Aspergillus nidulans* ou *Aspergillus oryzae*, ou le marqueur pyrG de *Aspergillus nidulans, Aspergillus niger, Aspergillus awamori,* ou *Aspergillus oryzae.*

21. Vecteur selon la revendication 13 qui comporte à la fois le promoteur de l'amylase TAKA de *Aspergillus oryzae* et le marqueur amdS ou pyrG de *Aspergillus nidulans* ou *Aspergillus oryzae.*

22. Cellule hôte recombinante comportant le produit de synthèse d'ADN hétérologue selon les revendications 1, 2, 3, 4, 5, 6, 7, 8, 9, 10 ou 11 codant pour une laccase de *Polyporus .*

23. Cellule selon la revendication 22, qui est une cellule fongique.

24. Cellule selon la revendication 23, qui est une cellule d'*Aspergillus.*

25. Cellule selon la revendication 22, dans laquelle le produit de synthèse est intégré dans le génome de la cellule hôte.

26. Cellule selon la revendication 22, dans laquelle le produit de synthèse est contenu sur un vecteur.

27. Cellule selon la revendication 22, qui comporte un produit de synthèse contenant une séquence codant pour une séquence d'acides aminés sélectionnée parmi le groupe constitué de celles représentées dans les SEQ ID N° 4, 6, 8 et 10.

28. Procédé pour obtenir une enzyme laccase qui comporte la culture d'une cellule hôte recombinante comportant un produit de synthèse d'ADN contenant la séquence d'acide nucléique selon la revendication 1, codant pour une enzyme laccase de *Polyporus*, sous des conditions amenant l'expression de l'enzyme, et la récupération de l'enzyme depuis la culture.

29. Procédé pour obtenir une enzyme laccase qui comporte la culture d'une cellule hôte de *Aspergillus* recombinante comportant un produit de synthèse d'ADN contenant une séquence d'acide nucléique selon l'une quelconque des revendications 1 à 11, codant pour une enzyme laccase de *Polyporus*, sous des conditions amenant l'expression de l'enzyme, et la récupération de l'enzyme depuis la culture.

30. Procédé pour polymériser un substrat de lignine ou lignosulfate en solution qui comporte la mise en contact du substrat avec une laccase de *Polyporus* selon la revendication 12.

31. Procédé pour la dépolymérisation in situ de pâte à papier Kraft qui comporte la mise en contact de la pâte avec une laccase de *Polyporus* selon la revendication 12 ou la SEQ ID N° 2.

32. Procédé pour oxyder des colorants ou précurseurs de colorant qui comporte la mise en contact du colorant ou précurseur de colorant avec une laccase de *Polyporus* selon la revendication 12.

33. Procédé pour colorer les cheveux qui comporte la mise en contact d'une laccase de *Polyporus* selon la revendication 12 ou la SEQ ID N° 2 en présence ou l'absence d'au moins un agent de modification, avec au moins un précurseur de colorant, pendant un temps et sous des conditions suffisants pour permettre l'oxydation du précurseur de colorant en un colorant.

34. Procédé selon la revendication 33, dans lequel le précurseur de colorant est sélectionné parmi le groupe constitué d'une diamine, d'un aminophénol, et d'un phénol.

35. Procédé selon la revendication 33, dans lequel l'agent de modification, lorsqu'il est utilisé, est une méta-diamine, un méta-aminophénol, ou un polyphénol.

36. Procédé selon la revendication 35, dans lequel le précurseur de colorant est un intermédiaire primaire sélectionné parmi le groupe constitué d'une ortho-diamine ou para-diamine ou d'un aminophénol.

37. Procédé selon la revendication 35, dans lequel plus d'un précurseur de colorant est utilisé.

38. Procédé selon la revendication 35, dans lequel plus d'un agent de modification est utilisé.

39. Procédé selon la revendication 35, dans lequel un intermédiaire primaire et un agent de modification sont tous deux utilisés.

40. Composition colorante comportant une laccase de *Polyporus* selon la revendication 12 ou la SEQ ID N° 2 combinée à au moins un précurseur de colorant.

41. Composition colorante comportant une laccase de *Polyporus* selon la revendication 12 ou la SEQ ID N° 2 combinée à au moins un intermédiaire primaire et au moins un agent de modification.

42. Conteneur contenant une composition colorante comportant une laccase de *Polyporus* selon la revendication 12 ou la SEQ ID N° 2 et au moins un précurseur de colorant dans une atmosphère exempte d'oxygène.

43. Conteneur selon la revendication 42, qui contient au moins un précurseur de colorant intermédiaire primaire combiné à au moins un agent de modification.

44. Procédé de polymérisation ou d'oxydation d'un composé phénolique ou d'aniline qui comporte la mise en contact du composé phénolique ou d'aniline avec une laccase de *Polyporus* selon la revendication 12.
